(19) Europäisches Patentamt / European Patent Office / Office européen des brevets

(11) EP 1 912 644 B1

(12) # FASCICULE DE BREVET EUROPEEN

(45) Date de publication et mention de la délivrance du brevet:
**16.12.2009 Bulletin 2009/51**

(21) Numéro de dépôt: **06778888.5**

(22) Date de dépôt: **19.07.2006**

(51) Int Cl.:
***A61K 31/437*** *(2006.01)* ***C07D 471/04*** *(2006.01)*
***A61P 25/00*** *(2006.01)*

(86) Numéro de dépôt international:
**PCT/FR2006/001767**

(87) Numéro de publication internationale:
**WO 2007/010138 (25.01.2007 Gazette 2007/04)**

(54) **DERIVES DE N-(ARYLALKYL)-1H-PYRROLOPYRIDINE-2-CARBOXAMIDES, LEUR PREPARATION ET LEUR APPLICATION EN THERAPEUTIQUE**

N-(ARYLALKYL)-1H-PYRROLOPYRIDIN-2-CARBOXAMID-DERIVATE, IHRE HERSTELLUNG UND VERWENDUNG

N-(ARYLALKYL)-1H-PYRROLOPYRIDINE-2-CARBOXAMIDE DERIVATIVES, PREPARATION AND USE THEREOF

(84) Etats contractants désignés:
**AT BE BG CH CY CZ DE DK EE ES FI FR GB GR HU IE IS IT LI LT LU LV MC NL PL PT RO SE SI SK TR**
Etats d'extension désignés:
**AL BA HR MK RS**

(30) Priorité: **22.07.2005 FR 0507804**

(43) Date de publication de la demande:
**23.04.2008 Bulletin 2008/17**

(73) Titulaire: **Sanofi-Aventis**
**75013 Paris (FR)**

(72) Inventeurs:
- **DUBOIS Laurent**
  **F-92350 Le Plessis-Robinson (FR)**
- **EVANNO, Yannick**
  **75013 Paris (FR)**
- **MALANDA, André**
  **F-91140 Villejust (FR)**

(74) Mandataire: **Gaslonde, Aude et al**
**Sanofi-Aventis**
**Département Brevets**
**174, Avenue de France**
**75013 Paris (FR)**

(56) Documents cités:
**EP-A- 1 535 922**
**WO-A-01/64639**
**WO-A-03/068749**
**WO-A-2004/072069**
**US-A1- 2005 165 049**

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 1 912 644 B1

## Description

L'invention a pour objet des composés dérivés de *N*-(arylalkyl)-1*H*-pyrrolopyridine-2-carboxamides, qui présentent une activité antagoniste *in vitro* et *in vivo* pour les récepteurs de type TRPV1 (ou VR1).

Un premier objet de l'invention concerne les composés répondant à la formule générale (I) ci-après.
Un autre objet de l'invention concerne des procédés de préparation des composés de formule générale (I).
Un autre objet de l'invention concerne l'utilisation des composés de formule générale (I) notamment dans des médicaments ou dans des compositions pharmaceutiques.

Les composés de l'invention répondent à la formule générale (I) :

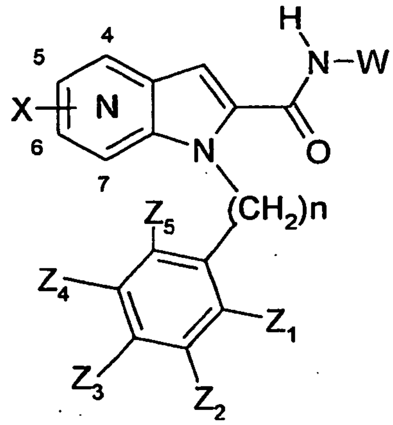

(I)

dans laquelle
n est égal à 0, 1, 2 ou 3 ;
le noyau pyrrolopyridine est un groupe pyrrolo[3,2-*b*]pyridine, un groupe pyrrolo[3,2-*c*]pyridine, un groupe pyrrolo[2,3-*c*]pyridine ou un groupe pyrrolo[2,3-*b*]pyridine ;
le noyau pyrrolopyridine étant éventuellement substitué en position carbonée 4, 5, 6 et /ou 7 par un ou plusieurs substituents X, identiques ou différents l'un de l'autre, choisis parmi un atome d'halogène ou un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, $C_1$-$C_6$-alcoxyle, $C_1$-$C_6$-fluoroalcoxyle, cyano, $C(O)NR_1R_2$, nitro, $NR_1R_2$, $C_1$-$C_6$-thioalkyle, -S(O)-$C_1$-$C_6$-alkyle, -$S(O)_2$-$C_1$-$C_6$-alkyle, $SO_2NR_1R_2$, $NR_3COR_4$, $NR_3SO_2R_5$ ou aryle, l'aryle étant éventuellement substitué par un ou plusieurs substitutants choisi parmi un halogène, un groupe $C_1$-$C_6$-alkyle,
$C_3$-$C_7$-cycloalkyle, $C_3$-$C_7$-cycloalkyle-$C_1$-$C_3$-alkylène, $C_1$-$C_6$-fluoroalkyle, $C_1$-$C_6$-alcoxyle, $C_1$-$C_6$-fluoroalcoxyle, nitro ou cyano ;
$Z_1$, $Z_2$, $Z_3$, $Z_4$ et $Z_5$ représentent, indépendamment l'un de l'autre, un atome d'hydrogène ou d'halogène ou un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, $C_1$-$C_6$-alcoxyle, $C_1$-$C_6$-fluoroalcoxyle, cyano, $C(O)NR_1R_2$, nitro, $NR_1R_2$, $C_1$-$C_6$-thioalkyle, -S(O)-$C_1$-$C_6$-alkyle, -$S(O)_2$-$C_1$-$C_6$-alkyle, $SO_2NR_1R_2$, $NR_3COR_4$, $NR_3SO_2R_5$, aryle-$C_1$-$C_6$-alkylène ou aryle, l'aryle et l'aryle-$C_1$-$C_6$-alkylène étant éventuellement substitué par un ou plusieurs substitutants choisis parmi un halogène, un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, $C_1$-$C_6$-alcoxyle, $C_1$-$C_6$-fluoroalcoxyle, nitro ou cyano ;
$R_1$ et $R_2$, représentent, indépendamment l'un de l'autre, un atome d'hydrogène ou un groupe $C_1$-$C_6$-alkyle, $C_3$-$C_7$-cycloalkyle, $C_3$-$C_7$-cycloalkyle-$C_1$-$C_3$-alkylène, aryle-$C_1$-$C_6$-alkylène ou aryle ; ou $R_1$ et $R_2$ formant ensemble, avec l'atome d'azote qui les porte, un groupe azétidine, pyrrolidine, pipéridine, azépine, morpholine, thiomorpholine, pipérazine, homopipérazine, ce groupe étant éventuellement substitué par un groupe $C_1$-$C_6$-alkyle, $C_3$-$C_7$-cycloalkyle, $C_3$-$C_7$-cycloalkyle-$C_1$-$C_3$-alkylène, aryle-$C_1$-$C_6$-alkylène ou aryle ;
$R_3$ et $R_4$ représentent, indépendamment l'un de l'autre, un atome d'hydrogène ou un groupe $C_1$-$C_6$-alkyle, aryle-$C_1$-$C_6$-alkylène ou aryle ;
$R_5$ représente un groupe $C_1$-$C_6$-alkyle ou aryle ;
W représente un groupe bicyclique fusionné de formule :

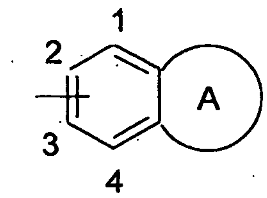

lié à l'atome d'azote par les positions 1, 2, 3 ou 4 ;
A représente un hétérocycle de 5 à 7 chaînons comprenant de un à trois hétéroatomes choisi parmi O, S ou N ;
le ou les atomes de carbones de A étant éventuellement substitués par un ou plusieurs groupes choisis parmi un atome d'hydrogène ou un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, aryle, aryle-$C_1$-$C_6$-alkylène, oxo ou thio;
le ou les atomes d'azote de A étant éventuellement substitués par $R_6$ lorsque l'azote est adjacent à un atome de carbone substitué par un groupe oxo, ou par $R_7$ dans les autres cas ;
$R_6$ représente un atome d'hydrogène ou un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, aryle-$C_1$-$C_6$-alkylène ou aryle ;
$R_7$ représente un atome d'hydrogène ou un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, aryle-$C_1$-$C_6$-alkylène, $C_1$-$C_6$-alkyle-C(O)-, $C_3$-$C_7$-cycloalkyle-$C_1$-$C_3$-alkylène-(CO)-, $C_1$-$C_6$-fluoroalkyle-C(O)-, $C_3$-$C_7$-cycloalkyle-C(O)-, aryle-C(O)-, aryle-$C_1$-$C_6$-alkylène-C(O)-, $C_1$-$C_6$-alkyle-$S(O)_2$-, $C_1$-$C_6$-fluoroalkyle-$S(O)_2$-, $C_3$-$C_7$-cycloalkyle-$S(O)_2$-, $C_3$-$C_7$-cycloalkyle-$C_1$-$C_3$-alkylène-$S(O)_2$-, aryle-$S(O)_2$- ou aryle-$C_1$-$C_6$-alkylène-$S(O)_2$- ou aryle.

Dans les composés de formule générale (I) :

- le ou les atomes de soufre de l'hétérocycle A peuvent être sous forme oxydée (S(O) ou $S(O)_2$);
- le ou les atomes d'azote de l'hétérocycle A peuvent être sous forme oxydée (N-oxyde) ;
- l'atome d'azote en position 4, 5, 6 ou 7 de la pyrrolopyridine peut être sous forme oxydée (N-oxyde).

Dans le cadre de l'invention, on peut citer à titre d'exemple de groupe W les groupes indolinyle, isoindolinyle, indolyle, isoindolyle, benzofuranyle, dihydrobenzofuranyle, benzothiophényle, dihydrobenzothiophényle, benzoxazolyle, dihydrobenzoxazolinyle, isobenzofuranyle, dihydroisobenzofuranyle, benzimidazolyle, dihydrobenzimidazolyle, indazolyle, benzothiazolyle, isobenzothiazolyle, dihydroisobenzothiazolyle, benzotriazolyle, quinoléinyle, dihydroquinoléinyle, tétrahydroquinoléinyle, isoquinoléinyle, dihydroisoquinoléinyle, tétrahydroisoquinoléinyle, benzoxazinyle, dihydrobenzoxazinyle, benzothiazinyle, dihydrobehzothiazinyle, cinnolinyle, quinazolinyle, dihydroquinazolinyle, tétrahydroquinazolinyle, quinoxalinyle, dihydroquinoxalinyle, tétrahydroquinoxalinyle, phtalazinyle, dihydrophtalazinyle, tétrahydrophtalazinyle, tétrahydrobenz[*b*]azépinyle, tétrahydrobenz[*c*]azépinyle, tétrahydrobenz[*d*]azépinyle, tétrahydrobenzo[*b*][1,4]diazépinyle, tétrahydrobenzo[*e*][1,4]diazépinyle, tétrahydrobenzo[*b*][1,4]oxazépinyle ou tétrahydrobenzo[*b*][1,4]thiazépinyle ; ces groupes pouvant être éventuellement substitués comme défini dans la formule générale (I).

Parmi les composés de formule générale (I) objets de l'invention, un premier sous-groupe de composés est constitué par les composés pour lesquels n est égal à 1 ou 2.

Parmi les composés de formule générale (I) objets de l'invention, un second sous-groupe de composés est constitué par les composés pour lesquels le noyau pyrrolopyridine est un groupe pyrrolo[3,2-*b*]pyridine, un groupe pyrrolo[3,2-*c*]pyridine, un groupe pyrrolo[2,3-*c*]pyridine ou un groupe pyrrolo[2,3-*b*]pyridine ;
le noyau pyrrolopyridine étant éventuellement substitué en position carbonée 4, 5, 6 et /ou 7 par un ou plusieurs substituents X, identiques ou différents l'un de l'autre, choisis parmi un atome d'hydrogène ou d'halogène, par exemple un fluor, un brome ou un chlore, ou un groupe $C_1$-$C_6$-alkyle, par exemple un méthyle, un propyle, un isopropyle, un secbutyle, un tertiobutyle, un pentyle, $C_3$-$C_7$-cycloalkyle, par exemple un cyclopentyle ou un cyclohexyle, $C_1$-$C_6$-fluoroalkyle, par exemple un groupe trifluorométhyle, $C_1$-$C_6$-alcoxyle, par exemple un méthoxyle ou un éthoxyle, $C_1$-$C_6$-fluoroalcoxyle, par exemple un groupe trifluorométhoxyle, nitro, $NR_1R_2$, $C_1$-$C_6$-thioalkyle, par exemple un thiométhyle, -S(O)-$C_1$-$C_6$-alkyle, -$S(O)_2$-$C_1$-$C_6$-alkyle, par exemple un -$S(O)_2$-$CH_3$, ou aryle, par exemple phényle ; $R_1$ et $R_2$ représentent, indépendamment l'un de l'autre, un atome d'hydrogène.

Parmi les composés de formule générale (I) objets de l'invention, un troisième sous-groupe de composés est constitué par les composés pour lesquels le noyau pyrrolopyridine est un groupe pyrrolo[3,2-*b*]pyridine, un groupe pyrrolo[3,2-*c*]pyridine, un groupe pyrrolo[2,3-*c*]pyridine ou un groupe pyrrolo[2,3-*b*]pyridine ;
le noyau pyrrolopyridine étant éventuellement substitué en position carbonée 4, 5, 6 et /ou 7, par exemple en position carbonée 5, par un ou plusieurs substituents X, identiques ou différents l'un de l'autre, par exemple par un substituant X, choisis parmi un atome d'halogène, par exemple un atome de chlore ou de fluor, ou un groupe $C_1$-$C_6$-fluoroalkyle,

par exemple un groupe trifluorométhyle, ou aryle, par exemple phényle.

Parmi les composés de formule générale (I) objets de l'invention, un quatrième sous-groupe de composés est constitué par les composés pour lesquels le noyau pyrrolopyridine est un groupe pyrrolo[3,2-*b*]pyridine, un groupe pyrrolo[3,2-*c*]pyridine ou un groupe pyrrolo[2,3-*b*]pyridine ;

le noyau pyrrolopyridine étant éventuellement substitué en position carbonée 4, 5, 6 et /ou 7 par un ou plusieurs substituents X, identiques ou différents l'un de l'autre, choisis parmi un atome d'halogène ou un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, $C_1$-$C_6$-alcoxyle, $C_1$-$C_6$-fluoroalcoxyle, cyano, $C(O)NR_1R_2$, nitro, $NR_1R_2$, $C_1$-$C_6$-thioalkyle, -S(O)-$C_1$-$C_6$-alkyle, -$S(O)_2$-$C_1$-$C_6$-alkyle, $SO_2NR_1R_2$, $NR_3COR_4$, $NR_3SO_2R_5$ ou aryle, l'aryle étant éventuellement substitué par un ou plusieurs substitutants choisi parmi un halogène, un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, $C_1$-$C_6$-alcoxyle, $C_1$-$C_6$-fluoroalcoxyle, nitro ou cyano ;

$R_1$ et $R_2$, représentent, indépendamment l'un de l'autre, un atome d'hydrogène ou un groupe $C_1$-$C_6$-alkyle, $C_3$-$C_7$-cycloalkyle, $C_3$-$C_7$-cycloalkyle-$C_1$-$C_3$-alkylène, aryle-$C_1$-$C_6$-alkylène ou aryle ; ou $R_1$ et $R_2$ formant ensemble, avec l'atome d'azote qui les porte, un groupe azétidine, pyrrolidine, pipéridine, azépine, morpholine, thiomorpholine, pipérazine, homopipérazine, ce groupe étant éventuellement substitué par un groupe $C_1$-$C_6$-alkyle, $C_3$-$C_7$-cycloalkyle, $C_3$-$C_7$-cycloalkyle-$C_1$-$C_3$-alkylène, aryle-$C_1$-$C_6$-alkylène ou aryle ;

$R_3$ et $R_4$ représentent, indépendamment l'un de l'autre, un atome d'hydrogène ou un groupe $C_1$-$C_6$-alkyle, aryle-$C_1$-$C_6$-alkylène ou aryle; $R_5$ représente un groupe $C_1$-$C_6$-alkyle ou aryle.

Parmi les composés de formule générale (I) objets de l'invention, un cinquième sous-groupe de composés est constitué par les composés pour lesquels $Z_1$, $Z_2$, $Z_3$, $Z_4$ et $Z_5$ représentent, indépendamment l'un de l'autre, un atome d'hydrogène ou d'halogène, par exemple un atome de fluor.

Parmi les composés de formule générale (I) objets de l'invention, un sixième sous-groupe de composés est constitué par les composés pour lesquels W est choisi parmi les groupes indolinyle, indolyle, isoindolyle, isoindolinyle, benzofuranyle, dihydrobenzofuranyle, benzothiophényle, dihydrobenzothiophényle, benzoxazolyle, dihydrobenzoxazolinyle, isobenzofuranyle, dihydroisobenzofuranyle, benzimidazolyle, dihydrobenzimidazolyle, indazolyle, benzothiazolyle, isobenzothiazolyle, dihydroisobenzothiazolyle, benzotriazolyle, quinoléinyle, dihydroquinoléinyle, tétrahydroquinoléinyle, isoquinoléinyle, dihydroisoquinoléinyle, tétrahydroisoquinoléinyle, benzoxazinyle, dihydrobenzoxazinyle, benzothiazinyle, dihydrobenzothiazinyle, cinnolinyle, quinazolinyle, dihydroquinazolinyle, tétrahydroquinazolinyle, quinoxalinyle, dihydroquinoxalinyle, tétrahydroquinoxalinyle, phtalazinyle, dihydrophtalazinyle, tétrahydrophtalazinyle, tétrahydrobenz[*b*]azépinyle, tétrahydrobenz[*c*]azépinyle, tétrahydrobenz[*d*]azépinyle, tétrahydrobenzo[*b*][1,4]diazépinyle, tétrahydrobenzo[*e*][1,4]diazépinyte, tétrahydrobenzo[*b*][1,4]oxazépinyle ou tétrahydrobenzo[*b*][1,4]thiazépinyle ;

le ou les atomes de carbone et/ou d'azote dudit groupe W étant éventuellement substitués comme défini dans la formule générale (I).

Parmi les composés de formule générale (I) objets de l'invention, un septième sous-groupe de composés est constitué par les composés pour lesquels W représente un groupe bicyclique fusionné de formule :

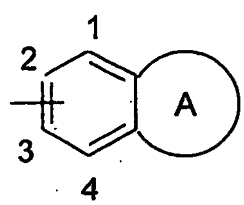

lié à l'atome d'azote par les positions 1, 2, 3 ou 4 ;

A représente un hétérocycle de 5 à 7 chaînons comprenant de un à trois hétéroatomes choisi parmi O, S ou N ;

et W est choisi parmi les groupes indolyle, benzimidazolyle, tétrahydroquinoléinyle, quinoléinyle, benzothiazolyle ; et/ou le ou les atomes de carbones de A étant éventuellement substitués par un ou plusieurs groupes choisis parmi un atome d'hydrogène ou un groupe $C_1$-$C_6$-alkyle, par exemple méthyle, ou un groupe oxo ; et/ou

le ou les atomes d'azote de A étant éventuellement substitués par $R_6$ lorsque l'azote est adjacent à un atome de carbone substitué par un groupe oxo, ou par $R_7$ dans les autres cas ;

$R_6$ représente un atome d'hydrogène ;

$R_7$ représente un atome d'hydrogène ou un groupe $C_1$-$C_6$-alkyle, par exemple méthyle.

Les composés pour lesquels à la fois n, X, $Z_1$, $Z_2$, $Z_3$, $Z_4$, $Z_5$ et W sont tels que définis dans les sous-groupes de composés de formule générale (I) ci-dessus, forment un huitième sous-groupe.

Parmi les composés de formule générale (I) objets de l'invention, un neuvième sous-groupe de composés est constitué par les composés pour lesquels :

n est égal à 0, 1, 2 ou 3 ;
le noyau pyrrolopyridine est un groupe pyrrolo[3,2-*b*]pyridine, un groupe pyrrolo[3,2-*c*]pyridine, un groupe pyrrolo [2,3-*c*]pyridine ou un groupe pyrrolo[2,3-*b*]pyridine ;
le noyau pyrrolopyridine étant éventuellement substitué en position carbonée 4, 5, 6 et /ou 7 par un ou plusieurs substituents X, identiques ou différents l'un de l'autre, choisis parmi un atome d'halogène ou un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, $C_1$-$C_6$-alcoxyle, $C_1$-$C_6$-fluoroalcoxyle, cyano, $C(O)NR_1R_2$, nitro, $NR_1R_2$, $C_1$-$C_6$-thioalkyle, -S(O)-$C_1$-$C_6$-alkyle, -$S(O)_2$-$C_1$-$C_6$-alkyle, $SO_2NR_1R_2$, $NR_3COR_4$, $NR_3SO_2R_5$ ou aryle, l'aryle étant éventuellement substitué par un ou plusieurs substitutants choisi parmi un halogène, un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, $C_1$-$C_6$-alcoxyle, $C_1$-$C_6$-fluoroalcoxyle, nitro ou cyano ;

$Z_1$, $Z_2$, $Z_3$, $Z_4$ et $Z_5$ représentent, indépendamment l'un de l'autre, un atome d'hydrogène ou d'halogène ou un groupe $C_1$-$C_6$-alkyle, $C_3$-$C_7$-cycloalkyle, $C_3$-$C_7$-cycloalkyle-$C_1$-$C_3$-alkylène, -$C_1$-$C_6$-fluoroalkyle, $C_1$-$C_6$-alcoxyle, $C_1$-$C_6$-fluoroalcoxyle, cyano, $C(O)NR_1R_2$, nitro, $NR_1R_2$, $C_1$-$C_6$-thioalkyle, -S(O)-$C_1$-$C_6$-alkyle, -$S(O)_2$-$C_1$-$C_6$-alkyle, $SO_2NR_1R_2$, $NR_3COR_4$, $NR_3SO_2R_5$, aryle-$C_1$-$C_6$-alkylène ou aryle, l'aryle et l'aryle-$C_1$-$C_6$-alkylène étant éventuellement substitué par un ou plusieurs substitutants choisis parmi un halogène, un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, $C_1$-$C_6$-alcoxyle, $C_1$-$C_6$-fluoroalcoxyle, nitro ou cyano ;
$R_1$ et $R_2$, représentent, indépendamment l'un de l'autre, un atome d'hydrogène ou un groupe $C_1$-$C_6$-alkyle, $C_3$-$C_7$-cycloalkyle, $C_3$-$C_7$-cycloalkyle-$C_1$-$C_3$-alkylène, aryle-$C_1$-$C_6$-alkylène ou aryle ; ou $R_1$ et $R_2$ formant ensemble, avec l'atome d'azote qui les porte, un groupe azétidine, pyrrolidine, pipéridine, azépine, morpholine, thiomorpholine, pipérazine, homopipérazine, ce groupe étant éventuellement substitué par un groupe $C_1$-$C_6$-alkyle, $C_3$-$C_7$-cycloalkyle, $C_3$-$C_7$-cycloalkyle-$C_1$-$C_3$-alkylène, aryle-$C_1$-$C_6$-alkylène ou aryle ;
$R_3$ et $R_4$ représentent, indépendamment l'un de l'autre, un atome d'hydrogène ou un groupe $C_1$-$C_6$-alkyle ou aryle ;
$R_5$ représente un groupe $C_1$-$C_6$-alkyle, aryle-$C_1$-$C_6$-alkylène ou aryle ;
W représente un groupe bicyclique fusionné de formule :

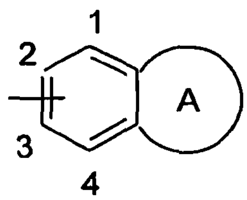

lié à l'atome d'azote par les positions 1, 2, 3 ou 4 ;
A représente un hétérocycle de 5 à 7 chaînons comprenant de un à trois hétéroatomes choisi parmi O, S ou N ;
le ou les atomes de carbones de A étant éventuellement substitués par un ou plusieurs groupes choisis parmi un atome d'hydrogène ou un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, aryle, aryle-$C_1$-$C_6$-alkylène, oxo ou thio;
le ou les atomes d'azote de A étant éventuellement substitués par $R_6$ lorsque l'azote est adjacent à un atome de carbone substitué par un groupe oxo, ou par $R_7$ dans les autres cas ;
$R_6$ représente un atome d'hydrogène ou un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, aryle-$C_1$-$C_6$-alkylène ou aryle ; $R_7$ représente un atome d'hydrogène ou un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, aryle-$C_1$-$C_6$-alkylène, $C_1$-$C_6$-alkyle-C(O)-, $C_3$-$C_7$-cycloalkyle-$C_1$-$C_3$-alkylène-(CO)-, $C_1$-$C_6$-fluoroalkyle-C(O)-, $C_3$-$C_7$-cycloalkyle-C(O)-, aryle-C(O)-, aryle-$C_1$-$C_6$-alkylène-C(O)-, $C_1$-$C_6$-alkyle-$S(O)_2$-, $C_1$-$C_6$-fluoroalkyle-$S(O)_2$-, $C_3$-$C_7$-cycloalkyle-$S(O)_2$-, $C_3$-$C_7$-cycloalkyle-$C_1$-$C_3$-alkylène-$S(O)_2$-, aryle-$S(O)_2$- ou aryle-$C_1$-$C_6$-alkylène-$S(O)_2$- ou aryle.
avec la condition que
quand $Z_1$, $Z_2$, $Z_3$, $Z_4$ et $Z_5$ représentent simultanément des atomes d'hydrogène et quand le noyau pyrrolopyridine est une pyrrolo[3,2-b]pyridine éventuellement substituée, alors n est égal à 2 ou 3.

Parmi les composés de formule générale (I) objets de l'invention, on peut citer les composés suivants:

- *N*-(1-méthyl-1*H*-indol-5-yl)-1-[(3-fluorophényl)méthyl]-1*H*-pyrrolo[2,3-*c*]pyridine-2-carboxamide ;
- *N*-(1-méthyl-1*H*-benzimidazol-5-yl)-5-fluoro-1-[(3-fluorophényl)méthyl]-1*H*-pyrrolo[2,3-*c*]pyridine-2-carboxamide ;
- *N*-(1,2-diméthyl-1*H*-benzimidazol-5-yl)-5-chloro-1-[(3-fluorophényl)méthyl]-1*H*-pyrrolo[2,3-*c*]pyridine-2-carboxamide ;
- *N*-(1,2-diméthyl-1*H*-benzimidazol-5-yl)-1-[(3-fluorophényl)méthyl]-1*H*-pyrrolo[2,3-*c*]pyridine-2-carboxamide ;

- *N*-(2-méthyl-1*H*-benzothiazol-5-yl)-5-fluoro-1-[(3-fluorophényl)méthyl]-1*H*-pyrrolo[2,3-*c*]pyridine-2-carboxamide ;
- *N*-(1,2-diméthyl-1*H*-benzimidazol-5-yl)-5-fluoro-1-[(3-fluorophényl)méthyl]-1*H*-pyrrolo[2,3-*c*]pyridine-2-carboxamide ;
- *N*-(1,2-diméthyl-1*H*-benzimidazol-5-yl)-5-phényl-1-[(3-fluorophényl)méthyl]-1*H*-pyrrolo[2,3-*c*]pyridine-2-carboxamide ;
- *N*-(1-méthyl-1*H*-indol-5-yl)-1-[(3-fluorophényl)méthyl]-1*H*-pyrrolo[2,3,*b*]pyridine-2-carboxamide ;
- *N*-(1-méthyl-1*H*-benzimidazol-5-yl)-1-[(3-fluorophényl)méthyl]-1*H*-pyrrolo[2,3-*b*]pyridine-2-carboxamide ;
- *N*-(2-oxo-1,2,3,4-tétrahydroquinoléin-7-yl)-1-[(3-fluorophényl)méthyl]-1*H*-pyrrolo[2,3-*b*]pyridine-2-carboxamide ;
- *N*-(quinoléin-7-yl)-1-[(3-fluorophènyl)méthyl]-1*H*-pyrrolo[2,3-*b*]pyridine-2-carboxamide;
- *N*-(1-méthyl-1*H*-indol-5-yl)-1-(phénylméthyl)-1*H*-pyrrolo[2,3-*b*]pyridine-2-carboxamide;
- *N*-(1-méthyl-1*H*-indol-5-yl)-1-(phényléthyl)-1*H*-pyrrolo[2,3-*b*]pyridine-2-carboxamide;
- *N*-(2-méthyl-benzothiazol-5-yl)-1-[(3-fluorophényl)méthyl]-1*H*-pyrrolo[2,3-*b*]pyridine-2-carboxamide ;
- *N*-(1-méthyl-1*H*-benzimidazol-5-yl)-5-fluoro-1-[(3-fluorophényl)méthyl]-1*H*-pyrrolo[2,3-*b*]pyridine-2-carboxamide ;
- *N*-(1,2-diméthyl-1*H*-benzimidazol-5-yl)-5-trifluorométhyl-1-[(3-fluorophényl)méthyl]-1*H*-pyrrolo[2,3-*b*]pyridine-2-carboxamide;
- *N*-(1-méthyl-1*H*-indol-5-yl)-5-trifluorométhyl-1-[(3-fluorophényl)méthyl]-1*H*-pyrrolo[2,3-*b*]pyridine-2-carboxamide ;
- *N*-(2-méthyl-benzothiazol-5-yl)-5-trifluorométhyl-1-[(3-fluorophényl)méthyl]-1*H*-pyrrolo[2,3-*b*]pyridine-2-carboxamide ;
- *N*-(1,2-diméthyl-1*H*-benzimidazol-5-yl)-1-[(3-fluorophényl)méthyl]-1*H*-pyrrolo[2,3-*b*]pyridine-2-carboxamide ;
- *N*-(1,2-diméthyl-1*H*-benzimidazol-5-yl)-5-fluoro-1-[(3-fluorophényl)méthyl]-1*H*-pyrrolo[2,3-*b*]pyridine-2-carboxamide ;
- *N*-(1,2-diméthyl-1*H*-benzimidazol-5-yl)-1-[(3-fluorophényl)méthyl]-1*H*-pyrrolo[3,2-*c*]pyridine-2-carboxamide ;
- *N*-(1-méthyl-1*H*-benzimidazol-5-yl)-1-[(3-fluorophényl)méthyl]-1*H*-pyrrolo[3,2-*b*]pyridine-2-carboxamide ;
- *N*-(1,2-diméthyl-1*H*-benzimidazol-5-yl)-5-trifluorométhyl-1-[(3-fluorophényl)méthyl]-1*H*-pyrrolo[3,2-*b*]pyridine-2-carboxamide
- *N*-(2-méthyl-benzothiazol-5-yl)-1-[(3-fluorophényl)méthyl]-1*H*-pyrrolo[3,2-*b*]pyridine-2-carboxamide ;
- *N*-(1,2-diméthyl-1*H*-benzimidazol-5-yl)-1-[(3-fluorophényl)méthyl]-1*H*-pyrrolo[3,2-*b*]pyridine-2-carboxamide ;
- *N*-(1,2-diméthyl-1*H*-benzimidazol-5-yl)-1-(phényl)-1*H*-pyrrolo[2,3-*b*]pyridine-2-carboxamide.

Parmi les composés de formule générale (I) objets de l'invention, un sous-groupe de composés est constitué par les composés de formule générale (I')

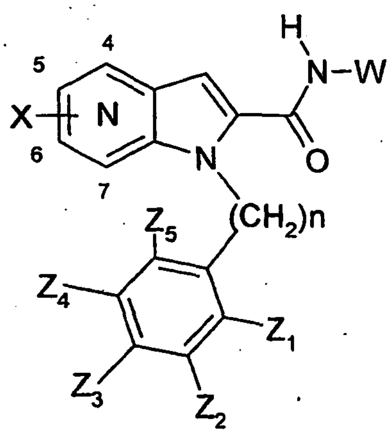

(I')

dans laquelle
n est égal à 1, 2 ou 3 ;
le noyau pyrrolopyridine est un groupe pyrrolo[3,2-*b*]pyridine, un groupe pyrrolo[3,2-*c*]pyridine, un groupe pyrrolo[2,3-*c*] pyridine ou un groupe pyrrolo[2,3-*b*]pyridine ;
le noyau pyrrolopyridine étant éventuellement substitué en position carbonée 4, 5, 6 et /ou 7 par un ou plusieurs substituents X, identiques ou différents l'un de l'autre, choisis parmi un atome d'halogène ou un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, $C_1$-$C_6$-alcoxyle, $C_1$-$C_6$-fluoroalcoxyle, cyano, $C(O)NR_1R_2$, nitro, $NR_1R_2$, $C_1$-$C_6$-thioalkyle, -S(O)-$C_1$-$C_6$-alkyle, -$S(O)_2$-$C_1$-$C_6$-alkyle, $SO_2NR_1R_2$, $NR_3COR_4$, $NR_3SO_2R_5$ ou aryle, l'aryle étant éventuellement substitué par un ou plusieurs substitutants choisi parmi un halogène,

un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, $C_1$-$C_6$-alcoxyle, $C_1$-$C_6$-fluoroalcoxyle, nitro ou cyano ;

$Z_1$, $Z_2$, $Z_3$, $Z_4$ et $Z_5$ représentent, indépendamment l'un de l'autre, un atome d'hydrogène ou d'halogène ou un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, $C_1$-$C_6$-alcoxyle, $C_1$-$C_6$-fluoroalcoxyle, cyano, $C(O)NR_1R_2$, nitro, $NR_1R_2$, $C_1$-$C_6$-thioalkyle, -S(O)-$C_1$-$C_6$-alkyle, -$S(O)_2$-$C_1$-$C_6$-alkyle, $SO_2NR_1R_2$, $NR_3COR_4$, $NR_3SO_2R_5$, aryle-$C_1$-$C_6$-alkylène ou aryle, l'aryle et l'aryle-$C_1$-$C_6$-alkylène étant éventuellement substitué par un ou plusieurs substitutants choisis parmi un halogène, un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, $C_1$-$C_6$-alcoxyle, $C_1$-$C_6$-fluoroalcoxyle, nitro ou cyano ;

$R_1$ et $R_2$, représentent, indépendamment l'un de l'autre, un atome d'hydrogène ou un groupe $C_1$-$C_6$-alkyle, $C_3$-$C_7$-cycloalkyle, $C_3$-$C_7$-cycloalkyle-$C_1$-$C_3$-alkylène, aryle-$C_1$-$C_6$-alkylène ou aryle ; ou $R_1$ et $R_2$ formant ensemble, avec l'atome d'azote qui les porte, un groupe azétidine, pyrrolidine, pipéridine, azépine, morpholine, thiomorpholine, pipérazine, homopipérazine, ce groupe étant éventuellement substitué par un groupe $C_1$-$C_6$-alkyle, $C_3$-$C_7$-cycloalkyle, $C_3$-$C_7$-cycloalkyle-$C_1$-$C_3$-alkylène, aryle-$C_1$-$C_6$-alkylène ou aryle ;

$R_3$ et $R_4$ représentent, indépendamment l'un de l'autre, un atome d'hydrogène ou un groupe $C_1$-$C_6$-alkyle, aryle-$C_1$-$C_6$-alkylène ou aryle ;

$R_5$ représente un groupe $C_1$-$C_6$-alkyle ou aryle ;

W représente un groupe bicyclique fusionné de formule :

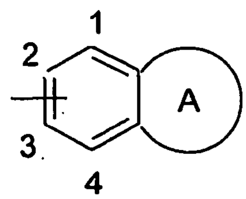

lié à l'atome d'azote par les positions 1, 2, 3 ou 4 ;

A représente un hétérocycle de 5 à 7 chaînons comprenant de un à trois hétéroatomes choisi parmi O, S ou N ;

le ou les atomes de carbones de A étant éventuellement substitués par un ou plusieurs groupes choisis parmi un atome d'hydrogène ou un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, aryle, aryle-$C_1$-$C_6$-alkylène, oxo ou thio;

le ou les atomes d'azote de A étant éventuellement substitués par $R_6$ lorsque l'azote est adjacent à un atome de carbone substitué par un groupe oxo, ou par $R_7$ dans les autres cas ;

$R_6$ représente un atome d'hydrogène ou un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, aryle-$C_1$-$C_6$-alkylène ou aryle ; $R_7$ représente un atome d'hydrogène ou un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, aryle-$C_1$-$C_6$-alkylène, $C_1$-$C_6$-alkyle-C(O)-, $C_3$-$C_7$-cycloalkyle-$C_1$-$C_3$-alkylène-(CO)-, $C_1$-$C_6$-fluoroalkyle-C(O)-, $C_3$-$C_7$-cycloalkyle-C(O)-, aryle-C(O)-, aryle-$C_1$-$C_6$-alkylène-C(O)-, $C_1$-$C_6$-alkyle-$S(O)_2$-, $C_1$-$C_6$-fluoroalkyle-$S(O)_2$-, $C_3$-$C_7$-cycloalkyle-$S(O)_2$-, $C_3$-$C_7$-cycloalkyle-$C_1$-$C_3$-alkylène-$S(O)_2$-, aryle-$S(O)_2$- ou aryle-$C_1$-$C_6$-alkylène-$S(O)_2$- ou aryle.

Parmi les composés de formule générale (I') objets de l'invention, un premier sous-groupe de composés est constitué par les composés pour lesquels n est égal à 1 ou 2.

Parmi les composés de formule générale (I') objets de l'invention, un second sous-groupe de composés est constitué par les composés pour lesquels le noyau pyrrolopyridine est un groupe pyrrolo[2,3-c]pyridine ou un groupe pyrrolo[2,3-b]pyridine éventuellement substitué en position carbonée 4, 5, 6 et / ou 7, par un ou plusieurs substituents X, identiques ou différents l'un de l'autre, choisis parmi un atome d'hydrogène ou d'halogène, par exemple un fluor, un brome ou un chlore, ou un groupe $C_1$-$C_6$-alkyle, par exemple un méthyle, un propyle, un isopropyle, un secbutyle, un tertiobutyle, un pentyle, $C_3$-$C_7$-cycloalkyle, par exemple un cyclopentyle ou un cyclohexyle, $C_1$-$C_6$-fluoroalkyle, par exemple un groupe trifluorométhyle, $C_1$-$C_6$-alcoxyle, par exemple un méthoxyle ou un éthoxyle, $C_1$-$C_6$-fluoroalcoxyle, par exemple un groupe trifluorométhoxyle, nitro, $NR_1R_2$, $C_1$-$C_6$-thioalkyle, par exemple un thiométhyle, -S(O)-$C_1$-$C_6$-alkyle, -$S(O)_2$-$C_1$-$C_6$-alkyle, par exemple un -$S(O)_2$-$CH_3$, ou aryle, par exemple phényle ; $R_1$ et $R_2$ représentent, indépendamment l'un de l'autre, un atome d'hydrogène.

Parmi les composés de formule générale (I') objets de l'invention, un troisième sous-groupe de composés est constitué par les composés pour lesquels le noyau pyrrolopyridine est un groupe pyrrolo[2,3-c]pyridine ou un groupe pyrrolo[2,3-b]pyridine éventuellement substitué en position carbonée 4, 5, 6 et / ou 7, par exemple en position carbonée 5, par un ou plusieurs substituents X, identiques ou différents l'un de l'autre, par exemple par un substituant X, choisis parmi un atome d'halogène, par exemple un atome de chlore ou de fluor, ou un groupe $C_1$-$C_6$-fluoroalkyle, par exemple un groupe trifluorométhyle.

Parmi les composés de formule générale (I') objets de l'invention, un quatrième sous-groupe de composés

est constitué par les composés pour lesquels $Z_1$, $Z_2$, $Z_3$, $Z_4$ et $Z_5$ représentent, indépendamment l'un de l'autre, un atome d'hydrogène ou d'halogène, par exemple un atome de fluor.

Parmi les composés de formule générale (I') objets de l'invention, un cinquième sous-groupe de composés est constitué par les composés pour lesquels W est choisi parmi les groupes indolinyle, indolyle, isoindolyle, isoindolinyle, benzofuranyle, dihydrobenzofuranyle, benzothiophényle, dihydrobenzothiophényle, benzoxazolyle, dihydrobenzoxazolinyle, isobenzofuranyle, dihydroisobenzofuranyle, benzimidazolyle, dihydrobenzimidazolyle, indazolyle, benzothiazolyle, isobenzothiazolyle, dihydroisobenzothiazolyle, benzotriazolyle, quinoléinyle, dihydroquinoléinyle, tétrahydroquinoléinyle, isoquinoléinyle, dihydroisoquinoléinyle, tétrahydroisoquinoléinyle, benzoxazinyle, dihydrobenzoxazinyle, benzothiazinyle, dihydrobenzothiazinyle, cinnolinyle, quinazolinyle, dihydroquinazolinyle, tétrahydroquinazolinyle, quinoxalinyle, dihydroquinoxalinyle, tétrahydroquinoxalinyle, phtalazinyle, dihydrophtalazinyle, tétrahydrophtalazinyle, tétrahydrobenz[*b*]azépinyle, tétrahydrobenz[*c*]azépinyle, tétrahydrobenz[*d*]azépinyle, tétrahydrobenzo[*b*][1,4]diazépinyle, tétrahydrobenzo[*e*][1,4]diazépinyle, tétrahydrobenzo[*b*][1,4]oxazépinyle ou tétrahydrobenzo[*b*][1,4]thiazépinyle ;
le ou les atomes de carbone et/ou d'azote dudit groupe W étant éventuellement substitués comme défini dans la formule générale (I).

Parmi les composés de formule générale (I') objets de l'invention, un sixième sous-groupe de composés est constitué par les composés pour lesquels W représente un groupe bicyclique fusionné de formule :

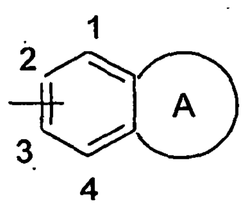

lié à l'atome d'azote par les positions 1, 2, 3 ou 4 ;
A représente un hétérocycle de 5 à 7 chaînons comprenant de un à trois hétéroatomes choisi parmi O, S ou N ;
et W est choisi parmi les groupes indolyle, benzimidazolyle, dihydroquinoléinyle, quinoléinyle, benzothiazolyle ; et/ou
le ou les atomes de carbones de A étant éventuellement substitués par un ou plusieurs groupes choisis parmi un atome d'hydrogène ou un groupe $C_1$-$C_6$-alkyle, par exemple méthyle ; et/ou
le ou les atomes d'azote de A étant éventuellement substitués par $R_7$ ;
$R_7$ représente un atome d'hydrogène ou un groupe $C_1$-$C_6$-alkyle, par exemple méthyle.

Les composés pour lesquels à la fois n, X, $Z_1$, $Z_2$, $Z_3$, $Z_4$, $Z_5$ et W sont tels que définis dans les sous-groupes de composés de formule générale (I') ci-dessus, forment un septième sous-groupe.

Dans le cadre de la présente invention on entend par :

- $C_t$-$C_z$ où t et z peuvent prendre les valeurs de 1 à 7, une chaîne carbonée pouvant avoir de t à z atomes de carbone, par exemple $C_1$-$C_3$ une chaîne carbonée qui peut avoir de 1 à 3 atomes de carbone ;
- un alkyle : un groupe aliphatique saturé linéaire ou ramifié. A titre d'exemples, on peut citer les groupes méthyle, éthyle, propyle, isopropyle, butyle, isobutyle, tertiobutyle, pentyle, etc ;
- un alkylène : un groupe alkyle divalent saturé, linéaire ou ramifié, par exemple un groupe $C_{1-3}$-alkylène représente une chaîne carbonée divalente de 1 à 3 atomes de carbone, linéaire ou ramifiée, par exemple un méthylène, éthylène, 1-méthyléthylène, propylène ;
- un cycloalkyle : un groupe carboné cyclique. A titre d'exemples, on peut citer les groupes cyclopropyle, cyclobutyle, cyclopentyle, cyclohexyle, etc ;
- un fluoroalkyle : un groupe alkyle dont un ou plusieurs atomes d'hydrogène ont été substitués par un atome de fluor ;
- un alcoxyle : un radical -O-alkyle où le groupe alkyle est tel que précédemment défini ;
- un fluoroalcoxyle : un groupe alcoxyle dont un ou plusieurs atomes d'hydrogène ont été substitués par un atome de fluor ;
- un thioalkyle : un radical -S-alkyle où le groupe alkyle est tel que précédemment défini ;
- un aryle : un groupe aromatique cyclique comprenant entre 6 et 10 atomes de carbones. A titre d'exemples de groupes aryles, on peut citer les groupes phényle ou naphtyle ;
- un hétérocycle : un groupe cyclique saturé, partiellement insaturé ou aromatique de 5 à 7 chaînons, comprenant de un à trois hétéroatomes choisi parmi O, S ou N ;
- un atome d'halogène : un fluor, un chlore, un brome ou un iode ;
- « oxo » signifie « =O » ;
- « thio » signifie « =S ».

Les composés de formule (I) peuvent comporter un ou plusieurs atomes de carbone asymétriques. Ils peuvent donc exister sous forme d'énantiomères ou de diastéréoisomères. Ces énantiomères, diastéréoisomères, ainsi que leurs mélanges, y compris les mélanges racémiques, font partie de l'invention.

Les composés de formule (I) peuvent exister à l'état de bases ou de sels d'addition à des acides. De tels sels d'addition font partie de l'invention.

Ces sels sont avantageusement préparés avec des acides pharmaceutiquement acceptables, mais les sels d'autres acides utiles, par exemple, pour la purification ou l'isolement des composés de formule (I) font également partie de l'invention.

Les composés de formule générale (I) peuvent se trouver sous forme d'hydrates ou de solvats, à savoir sous forme d'associations ou de combinaisons avec une ou plusieurs molécules d'eau ou avec un solvant. De tels hydrates et solvats font également partie de l'invention.

Dans ce qui suit, on entend par groupe partant, un groupe pouvant être facilement clivé d'une molécule par rupture d'une liaison hétérolytique, avec départ d'une paire électronique. Ce groupe peut ainsi être remplacé facilement par un autre groupe lors d'une réaction de substitution, par exemple. De tels groupes partants sont, par exemple, les halogènes ou un groupe hydroxyle activé tel qu'un méthanesulfonate, benzènesulfonate, p-toluènesulfonate, triflate, acétate, etc. Des exemples de groupes partants ainsi que des références pour leur préparation sont donnés dans « Advances in Organic Chemistry », J. March, 5th Edition, Wiley Interscience, 2001.

Conformément à l'invention on peut préparer les composés de formule générale (I) selon le procédé illustré par le schéma 1 qui suit.

Selon le schéma 1, les composés de formule générale (IV) peuvent être obtenus par réaction d'un composé de formule générale (II) dans laquelle X est tel que défini dans la formule générale (I) ci-dessus et B représente un groupe $C_1$-$C_6$-alcoxyle ou hydroxyle, avec un composé de formule générale (III), dans laquelle $Z_1$, $Z_2$, $Z_3$, $Z_4$, $Z_5$ et n sont tels que définis dans la formule générale (I) ci-dessus et R' représente un groupe partant ou un groupe hydroxyle lorsque n est égal à 1, 2 ou 3 ou R' représente un groupe partant lorsque n est égal à 0.

Lorsque le composé de formule générale (III), est défini tel que n est égal à 1, 2 ou 3 et R' représente un groupe partant tel qu'un atome de brome ou d'iode, la réaction peut être réalisée en présence d'une base telle que l'hydrure de sodium ou le carbonate de potassium, dans un solvant polaire tel que le diméthylformamide, le diméthylsulfoxyde ou l'acétone (n = 1 : Kolasa T., Bioorg.Med.Chem. 1997, 5 (3) 507, n = 2 : Abramovitch R., Synth. Commun., 1995, 25 (1), 1).

Schéma 1

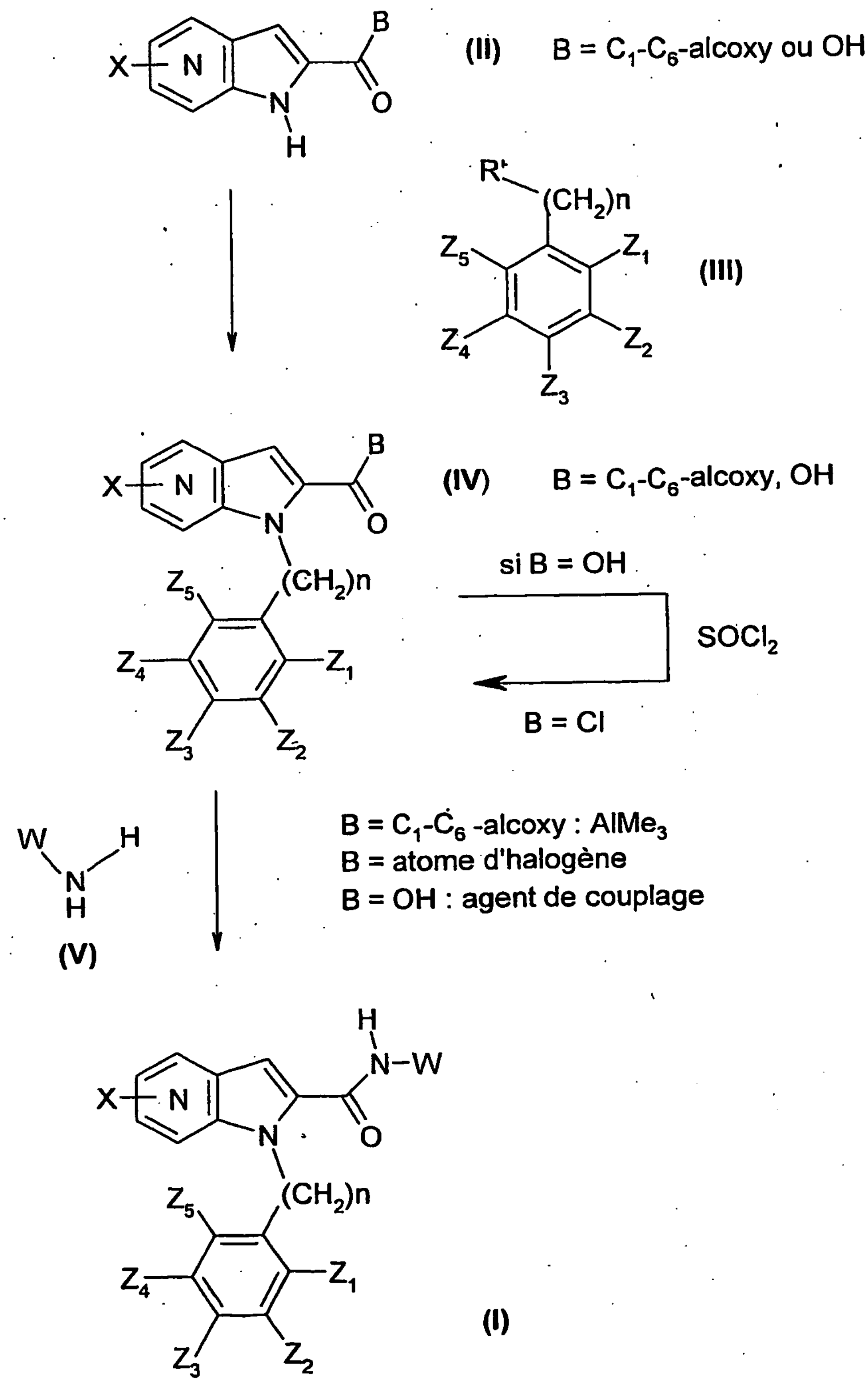


Lorsque le composé de formule générale (III) est défini tel que n est égal à 1, 2 ou 3 et R' représente un groupe hydroxyle, les composés de formule générale (IV), peuvent être obtenus par réaction du composé de formule générale

(II) avec un composé de formule générale (III) en présence d'une phosphine telle que la triphénylphosphine et d'un réactif tel que l'azodicarboxylate de diéthyle en solution dans un solvant tel que le dichlorométhane ou le tétrahydrofurane (O. Mitsonobu, Synthesis, 1981, 1-28).

Lorsque le composé de formule générale (III) est défini tel que n est égal à 0, R' représente un groupe partant tel qu'un atome de chlore, de brome ou d'iode et la réaction peut être réalisée à une température comprise entre 80°C et 250°C, en présence d'un catalyseur à base de cuivre tel que le bromure de cuivre ou l'oxyde de cuivre ainsi que d'une base telle que le carbonate de potassium (Murakami Y., Chem.Pharm.Bull., 1995, 43 (8), 1281). On peut également utiliser les conditions plus douces, décrites dans S.L. Buchwald, J.Am.Chem.Soc. 2002, 124, 11684.

Dans le cadre de l'invention, les composés de formule générale (IV) dans laquelle B représente un groupe $C_1$-$C_6$-alcoxyle peuvent être transformés en composés de formule générale (IV) dans laquelle B représente un groupe hydroxyle selon des méthodes connues de l'homme du métier, par exemple en présence d'une base telle que l'hydroxyde de sodium dans un solvant tel que le méthanol ou l'éthanol.

Dans le cadre de l'invention, les composés de formule générale (IV) dans laquelle B représente un groupe hydroxyle peuvent être transformés en composés de formule générale (IV) dans laquelle B représente un groupe $C_1$-$C_6$-alcoxyle selon des méthodes connues de l'homme du métier, par exemple en présence d'un acide tel que l'acide sulfurique dans un solvant tel que le méthanol ou l'éthanol.

Dans le cas des composés de formule générale (IV), dans laquelle B représente un groupe $C_1$-$C_6$-alcoxyle, le composé de formule générale (I) peut être obtenu par réaction d'un composé de formule générale (IV), tel qu'obtenu ci-dessus, avec un amidure du composé de formule générale (V), dans laquelle W est tel que défini dans la formule générale (I) ci-dessus, au reflux d'un solvant tel que le toluène. L'amidure d'aluminium du composé de formule générale (V) est préparé par action préalable du triméthylaluminium sur les amines de formule générale (V).

Dans le cas des composés de formule générale (IV), dans laquelle B représente un groupe hydroxyle, la fonction acide carboxylique peut préalablement être transformée en halogénure d'acide tel qu'un chlorure d'acide par action du chlorure de thionyle, au reflux d'un solvant tel que le dichlorométhane ou le dichloroéthane. Le composé de formule générale (I) est alors obtenu par réaction du composé de formule générale (IV), dans laquelle B représente un atome de chlore, avec le composé de formule générale (V), en présence d'une base telle que la triéthylamine ou le carbonate de sodium.

Alternativement, les composés de formule générale (IV), dans laquelle B représente un groupe hydroxyle, peuvent être couplés avec les composés de formule générale (V) en présence d'un agent de couplage tel qu'un dialkylcarbodiimide, l'hexafluorophosphate de [(benzotriazol-1-yl)oxy][tris(pyrrolidino)]phosphonium, le diéthylcyanophosphonate ou tout autre agent de couplage connu de l'homme de l'art, en présence d'une base comme la triéthylamine, dans un solvant tel que le diméthylformamide.

Dans le schéma 1, les composés de formule (II), (III) et (V) et les autres réactifs, quand leur mode de préparation n'est pas décrit, sont disponibles dans le commerce, décrits dans la littérature ou préparés par analogie à de nombreux procédés décrits dans la littérature (M. Nazare et al Angew Chem Int Ed 2004, 43(34), 4526-4528 ; P.M. Fresneda et al Tetrahedron Lett 2000, 41(24), 4777-4780 ; M.H. Fisher et al J Heterocyclic Chem 1969, 6, 775 ; B. Frydman et al J Am Chem Soc 1965, 87, 3530 ; L.N. Yakhontov Tetrahedron Lett 1969, 1909; G.P. Fagan et al J Med Chem 1988 31 (5), 944 ; OSI Pharmaceuticals WO2004104001 ; WO03049702 ; US0149367 ; WO03068749, US20050131012, par exemple).

Les composés de formules générales (II), (IV) ou (I), dans lesquelles X représente un groupe alkyle peuvent être obtenus par une réaction de couplage, catalysée par un métal tel que le palladium ou le fer, réalisée sur les composés de formules générales (II), (IV) ou (I) correspondants, dans lesquelles X représente un atome d'halogène, par exemple un chlore, en présence par exemple d'un halogénure d'alkylmagnésium ou d'un halogénure d'alkylzinc selon les méthodes décrites dans la littérature (A. Furstner et al J Am Chem Soc 2002, 124(46), 13856 ; G. Quéguiner et al J Org Chem 1998, 63(9), 2892 par exemple) ou connues de l'homme de métier.

Les composés de formules générales (II), (IV) et (I), dans lesquelles X, $Z_1$, $Z_2$, $Z_3$, $Z_4$ et/ou $Z_5$ représentent un groupe cyano ou un aryle, peuvent être obtenus par une réaction de couplage, catalysée par un métal tel que le palladium, réalisée sur les composés de formules générales (II), (IV) ou (I) correspondants, dans lesquelles X, $Z_1$, $Z_2$, $Z_3$, $Z_4$ et/ou $Z_5$ représente par exemple un atome de brome, en présence de cyanure de triméthylsilyle ou d'acide arylboronique, ou par toutes autres méthodes qui sont décrites dans la littérature ou qui sont connues de l'homme du métier.

Les composés de formules générales (I), (II) et (IV), dans lesquelles X, $Z_1$, $Z_2$, $Z_3$, $Z_4$ et/ou $Z_5$, représentent un groupe $NR_1R_2$, $NR_3COR_4$ ou $NR_3SO_2R_5$, peuvent être obtenus à partir des composés de formules générales (I), (II) et (IV) correspondants, dans lesquelles X, $Z_1$, $Z_2$, $Z_3$, $Z_4$ et/ou $Z_5$, représente, par exemple, un atome de brome, par réaction de couplage respectivement avec une amine, un amide ou une sulfonamide en présence d'une base, d'une phosphine et d'un catalyseur à base de palladium, selon des méthodes qui sont décrites dans la littérature ou qui sont connues de l'homme du métier. Les composés de formules générales (II), (IV) et (I), dans lesquelles X, $Z_1$, $Z_2$, $Z_3$, $Z_4$ et/ou $Z_5$ représentent un groupe $C(O)NR_1R_2$, peuvent être obtenus à partir des composés de formules générales (II), (IV) ou (I) correspondants, dans lesquelles X, $Z_1$, $Z_2$, $Z_3$, $Z_4$ et/ou $Z_5$ représente un groupe cyano, selon des méthodes qui sont

décrites dans la littérature ou qui sont connues de l'homme du métier.

Les composés de formules générales (II), (IV) et (I), dans lesquelles X, $Z_1$, $Z_2$, $Z_3$, $Z_4$ et/ou $Z_5$ représentent un groupe -S(O)-alkyle ou -$S(O)_2$-alkyle, peuvent être obtenus par oxydation des composés de formules générales (II), (IV) ou (I) correspondants, dans lesquelles X, $Z_1$, $Z_2$, $Z_3$, $Z_4$ et/ou $Z_5$ représente un groupe $C_1$-$C_6$-thioalkyle, selon des méthodes qui sont décrites dans la littérature ou qui sont connues de l'homme du métier. Les composés de formules générales (II), (IV) et (I), dans lesquelles X, $Z_1$, $Z_2$, $Z_3$, $Z_4$ et/ou $Z_5$ représentent un groupe $NR_1R_2$, $NR_3COR_4$ ou $NR_3SO_2R_5$, peuvent être obtenus à partir des composés de formules générales (II), (IV) ou (I) correspondants, dans lesquelles X, $Z_1$, $Z_2$, $Z_3$, $Z_4$ et/ou $Z_5$ représente un groupe nitro, par exemple par réduction, puis acylation ou sulfonylation, selon des méthodes qui sont décrites dans la littérature ou qui sont connues de l'homme du métier.

Les composés de formules générales (II), (IV) et (I), dans lesquelles X, $Z_1$, $Z_2$, $Z_3$, $Z_4$ et/ou $Z_5$ représentent un groupe $SO_2NR_1R_2$ peuvent être obtenus par une méthode analogue à celle décrite dans Pharmazie 1990, 45, 346, ou selon des méthodes qui sont décrites dans la littérature ou qui sont connues de l'homme du métier.

Les composés de formule générale (I), dans laquelle $R_7$ représente un atome d'hydrogène peuvent être obtenus à partir des composés de formule générale (I) dans laquelle, par exemple, $R_7$ représente un groupe phénylméthyle, par hydrogénation en présence d'un catalyseur à base de palladium ou par toutes méthodes qui sont décrites dans la littérature ou qui sont connues de l'homme du métier.

Les exemples suivants décrivent la préparation de certains composés conformes à l'invention. Ces exemples ne sont pas limitatifs et ne font qu'illustrer la présente invention. Les numéros des composés exemplifiés renvoient à ceux donnés dans le tableau 1. Les microanalyses élémentaires, les analyses LC-MS (chromatographie liquide couplée à la spectrométrie de masse) les spectres I.R. ou les spectres R.M.N. confirment les structures des composés obtenus.

**Exemple 1 (Composé N°1)**

*N*-(1-méthyl-1*H*-indol-5-yl)-1-[(3-fluorophényl)méthyl]-1*H*-pyrrolo[2,3-*c*]pyridine-2-carboxamide

1.1 3-(3-nitropyridin-4-yl)-2-oxopropionate d'éthyle

Dans un tricol de 100 mL, muni d'un agitateur magnétique et maintenu sous balayage d'azote, sont introduits 3,1 g (22,44 mmoles) de 4-méthyl-3-nitropyridine et 16,39 g (112,22 mmoles) d'oxalate d'éthyle. On ajoute ensuite au milieu réactionnel agité à température ambiante, 3,69 mL (24,69 mmoles) de 1,8-diazabicyclo[5.4.0]undec-7-ène. Le mélange réactionnel est alors agité à température ambiante pendant 1 heure puis dilué avec un mélange d'acétate d'éthyle (150 mL), d'eau (100 mL) et d'acide acétique (4 mL). Le mélange est extrait deux fois à l'acétate d'éthyle. Les phases organiques rassemblées sont lavées avec 100 mL d'eau, avec 100 mL d'une solution aqueuse saturée au chlorure de sodium, séchées sur sulfate de sodium puis concentrées sous pression réduite. Le brut réactionnel est ensuite trituré dans le pentane, filtré puis chauffé dans l'éther de pétrole, filtré et séché sous pression réduite. On isole ainsi 3,9 g (16,37 mmoles de produit qui est engagé tel quel dans l'étape suivante.

R.M.N. $^1H$ ($CDCl_3$), δ (ppm): 9,4 (s, 1H) ; 8,9 (d, 1H) ; 7,4 (d, 1H) 4,65 (s, 2H) ; 4,5 (q, 2H) ; 1,4 (t, 3H).

1.2 1*H*- pyrrolo[2,3-c]pyridine -2-carboxylate d'éthyle

Méthode A : A une solution de 3,9 g (16,37 mmoles) de produit obtenu à l'étape 1.1 dans 140 mL d'éthanol et 60 mL de tétrahydrofurane, sont ajoutés en une fois 60 mL d'une solution aqueuse saturée de chlorure d'ammonium et 5,48 g (98,2 mmoles) de poudre de fer. Le mélange réactionnel est ensuite agité à reflux pendant 2 heures. La solution refroidie est filtrée sur célite, que l'on rince plusieurs fois à l'acétate d'éthyle. Après concentration sous pression réduite du filtrat, on reprend le résidu par de l'eau et de l'acétate d'éthyle, la phase organique est lavée avec une solution aqueuse saturée au chlorure de sodium puis séchée sur sulfate de sodium. On isole un premier jet de 0,7 g (3,68 mmoles) de produit. On concentre la phase aqueuse sous pression réduite, on reprend le résidu par de l'acétate d'éthyle à chaud, on élimine le précipité par filtration et on concentre à nouveau le filtrat. On purifie le résidu obtenu par chromatographie sur colonne de gel de silice en éluant avec un mélange d'heptane et d'acétate d'éthyle. On obtient 0,7 g (3,68 mmoles) de produit supplémentaire.

Méthode B : A une solution de 0,25 g (1,05 mmole) de produit obtenu à l'étape 1.1 dans 10 mL d'éthanol, est ajouté 0,11 g (0,1 mmole) de palladium sur charbon à 10%. Le mélange réactionnel est hydrogéné sous une pression de 30 Psi pendant 2h30. à température ambiante. Après filtration sur fibre de verre, le filtrat est évaporé sous pression réduite et le brut réactionnel obtenu est recristallisé dans l'éthanol pour donner 0,08 g (0,42 mmol) de produit.

R.M.N. $^1H$ (DMSO $D_6$), δ (ppm): 8,9 (s, 1H) ; 8,3 (d, 1H) ; 7,7 (dd, 1H) ; 7,2 (d, 1H) ; 4,4 (q, 2H) ; 1,4 (t, 3H).

1.3 1-[(3-fluorophényl)méthyl]-1*H*- pyrrolo[2,3-*c*]pyridine -2-carboxylate d'éthyle

A une solution de 2 g (10,52 mmoles) de produit obtenu dans l'étape 1.2 dans 105 mL de tétrahydrofurane sec, maintenue sous atmosphère inerte, sont ajoutés successivement sous agitation, 2,03 g (15,77 mmoles) d'alcool 3-fluorobenzylique puis 4,17 g (15,77 mmoles) de triphénylphosphine. On ajoute ensuite, goutte à goutte à 0˚C, 2,83 g (15,77 mmoles) d'azodicarboxylate de diéthyle. Le mélange réactionnel est alors agité pendant 20h à température ambiante puis concentré sous pression réduite. On purifie l'huile résultante par chromatographies successives sur colonne de gel de silice en éluant avec un mélange d'heptane et d'acétate d'ethyle. On isole 1,9 g (6,37 mmoles) de produit. R.M.N. $^1$H ($CDCl_3$), δ (ppm): 8,8 (s, 1H) ; 8,3 (d, 1H) ; 7,6 (d, 1H) ; 7,2 (s, 1H) ; 7,1 (m, 1H) ; 6,85 (m, 2H) ; 6,65 (m, 1H) ; 5,8 (s, 2H) ; 4,3 (q, 2H) ; 1,3 (t, 3H).

1.4 *N*-(1-méthyl-1*H*-indol-5-yl)-1-[(3-fluorophényl)méthyl]-1*H*-pyrrolo[2,3-*c*]pyridine-2-carboxamide (composé n˚1)

On ajoute, à froid, sous argon et sous agitation magnétique, une solution de 0,29 g (2,01 mmoles) de 5-amino-1-méthylindole dans 10 mL de toluène sec à une solution de 1,68 mL (3,35 mmoles) de triméthylaluminium dans 5 mL de toluène sec. On porte le milieu réactionnel à 50˚C pendant 15 mn. Puis on additionne lentement 0,5 g (1,68 mmole) d'ester obtenu à l'étape 1.3 en solution dans 15 mL de toluène et on porte au reflux pendant 20h. A la solution refroidie, on ajoute de la glace, de l'acide chlorhydrique dilué puis de l'acétate d'éthyle. On recueille l'insoluble que l'on reprend par du dichlorométhane et une solution de soude. La phase organique est lavée à l'eau, séchée et concentrée sous pression réduite. On purifie le résidu par chromatographie sur colonne de silice en éluant avec un mélange de dichlorométhane et d'acétate d'éthyle. On triture le solide obtenu par de l'éther de pétrole, on le recueille par filtration et on le sèche sous pression réduite. On isole 0,385 g de produit attendu.
Point de fusion : 213 - 214,5 ˚C
R.M.N. $^1$H (DMSO $D_6$), δ (ppm) : 10,45 (s, 1H) ; 8,95 (s, 1H) ; 8,2 (d, 1H) ; 7,95 (s, 1H) ; 7,7 (d, 1H) ; 7,3 (m, 5H) ; 7,0 (m, 3H) ; 6,4 (d, 1H) ; 5,95 (s, 2H) ; 3,75 (s, 3H).

**<u>Exemple 2</u> (composé n˚2)**

*N*-(1-méthyl-1*H*-benzimidazol-5-yl)-5-fluoro-1-[(3-fluorophényl)méthyl]-1*H*-pyrrolo[2,3-*c*]pyridine-2-carboxamide

2.1 3-(2-fluoro-5-nitropyridin-4-yl)-2-oxopropionate d'éthyle

Dans un tricol de 100 mL, muni d'un agitateur magnétique et maintenu sous balayage d'azote, sont introduits, 2 g (12,81 mmoles) de 2-fluoro-4-méthyl-5-nitropyridine et 9,36 g (32,03 mmoles) d'oxalate d'éthyle. On ajoute ensuite au milieu réactionnel agité à température ambiante, 2,11 mL (14,09 mmoles) de 1,8-diazabicyclo[5.4.0]undec-7-ène. Le mélange réactionnel est alors agité à température ambiante pendant 4 heures. On ajoute ensuite un mélange d'acétate d'éthyle (100 mL), d'eau (40 mL) et d'acide acétique (2 mL). Le mélange est extrait deux fois à l'acétate d'éthyle. Les phases organiques rassemblées sont lavées avec 100 mL d'eau, avec 100 mL d'une solution aqueuse saturée au chlorure de sodium, séchées sur sulfate de sodium puis concentrées sous pression réduite. On purifie le résidu par chromatographie sur colonne de silice en éluant avec un mélange d'heptane et d'acétate d'éthyle. On isole ainsi 1,53 g de produit qui est engagé tel quel dans l'étape suivante.
R.M.N. $^1$H (DMSO $D_6$), δ(ppm): 8,9 (s, 1H) ; 7,9 (s, 1H) ; 6,7 (s, 1H) ; 4,7 (s, OH) ; 4,3 (q, 2H) ; 1,3 (t, 3H) ; forme céto-énolique majoritaire.

2.2 5-fluoro-1*H*- pyrrolo[2,3-*c*]pyridine -2-carboxylate d'éthyle

A une solution de 0,6 g (2,34 mmoles) de produit obtenu à l'étape 2.1 dans 30 mL d'éthanol et 15 mL de tétrahydrofurane, sont ajoutés en une fois 15 mL d'une solution aqueuse saturée de chlorure d'ammonium et 0,39 g (7,03 mmoles) de poudre de fer. Le mélange réactionnel est ensuite agité à reflux pendant 3 heures. La solution refroidie est filtrée sur célite et le filtrat est rincé plusieurs fois au méthanol. Après concentration sous pression réduite, on reprend le résidu par de l'acétate d'éthyle et de l'eau. La phase aqueuse est extraite par de l'acétate d'éthyle, les phases organiques rassemblées sont lavées avec 100 mL d'une solution aqueuse saturée au chlorure de sodium puis séchées sur sulfate de sodium, filtrées et évaporées sous pression réduite. On obtient 0,43 g (2,06 mmoles) de produit utilisé tel quel dans l'étape suivante.

R.M.N. $^1$H (DMSO De), δ (ppm): 12,5 (s, NH) ; 8,5 (s, 1H) ; 7,3 (s, 1 H) ; 7,1 (s, 1 H) ; 4,4 (q, 2H) ; 1,35 (t, 3H).

2.3 5-fluoro-1-[(3-fluorophényl)méthyl]-1*H*-pyrrolo[2,3-*c*]pyridine-2-carboxylate d'éthyle

A une solution de 0,4 g (1,92 mmole) de produit obtenu à l'étape 2.2 dans 20 mL de tétrahydrofurane sec, maintenue sous atmosphère inerte, sont ajoutés successivement sous agitation, 0,37 g (2,88 mmoles) d'alcool 3-fluorobenzylique puis 0,76 g (2,88 mmoles) de triphénylphosphine. Puis on ajoute, goutte à goutte à 0˚C, 0,52 g (2,88 mmoles) d'azodicarboxylate de diéthyle. Le mélange réactionnel est alors agité pendant 20h à température ambiante puis concentré sous pression réduite. On purifie l'huile résultante par chromatographie sur colonne de gel de silice en éluant avec un mélange de n-pentane et d'éther. On isole 0,49 g (1,55 mmole) de produit utilisé tel quel dans l'étape suivante.
R.M.N. $^1$H (DMSO $D_6$), δ (ppm): 8,7 (s, 1H) ; 8,5-7,1 (m, 6H) ; 5,9 (s, 2H) ; 4,3 (q, 2H) ; 1,25 (t, 3H).

2.4 *N*-(1-méthyl-1*H*-benzimidazol-5-yl)-5-fluoro-1-[(3-fluorophényl)méthyl]-1*H*-pyrrolo[2,3-*c*]pyridine-2-carboxamide (composé 2)

Dans un tricol de 100 mL refroidi à 0˚C et muni d'une agitation magnétique, est introduit sous balayage d'azote, 0,3 g (2,05 mmoles) de 5-amino-1-méthylbenzimidazole et 10 mL de toluène sec. A cette solution, on ajoute ensuite lentement 1,58 mL (3,16 mmoles) d'une solution 2M de triméthylaluminium dans le toluène. Le mélange réactionnel obtenu est maintenu sous atmosphère d'azote et agité en laissant la température progressivement atteindre 70˚C. On ajoute alors, goutte à goutte en 5 mn, à l'aide d'une ampoule d'addition, une solution de 0,5 g (1,58 mmole) de produit obtenu à l'étape 2.3, dans 10 mL de toluène sec. Le mélange réactionnel est alors porté à reflux pendant 2 heures. A la solution refroidie à 0˚C sont ensuite ajoutés 10 mL d'acide chlorhydrique 1 N et 20 mL d'eau glacée. Après 1 heure d'agitation à température ambiante, on recueille le précipité formé par filtration, on le lave à l'eau, on le sèche sous pression réduite et on le recristallise dans l'isopropanol. On isole le produit attendu sous la forme d'un solide jaune.
Point de fusion : 279 - 281 ˚C
R.M:N. $^1$H (DMSO $D_6$), δ (ppm): 10,6 (s, 1H) ; 8,65 (s, 1H) ; 8,15 (s, 1H) ; 8,05 (s, 1H) ; 7,65 (m, 2H) ; 7,35 (m, 3H) ; 6,99 (m, 3H) ; 5,9 (s, 2H) ; 3,9 (s, 3H).

**<u>Exemple 3</u> (composé n˚3)**

*N*-(1,2-diméthyl-1*H*-benzimidazol-5-yl)-5-chloro-1-[(3-fluorophényl)méthyl]-1*H*-pyrrolo[2,3-*c*]pyridine-2-carboxamide

3.1 3-(2-chloro-5-nitropyridin-4-yl)-2-oxopropionate d'éthyle

Dans un tricol de 100 mL, muni d'un agitateur magnétique et maintenu sous balayage d'azote, sont introduits, 1 g (5,79 mmoles) de 2-chloro-4-méthyl-5-nitropyridine et 4,23 g (28,94 mmoles) d'oxalate d'éthyle. On ajoute ensuite au milieu réactionnel agité à température ambiante, 0,96 mL (6,4 mmoles) de 1,8-diazabicyclo[5.4.0]undec-7-ène. Le mélange réactionnel est alors agité à température ambiante pendant 1 heure puis dilué avec un mélange d'acétate d'éthyle (40 mL), d'eau (30 mL) et d'acide acétique (1 mL). Le mélange est extrait deux fois à l'acétate d'éthyle. Les phases organiques rassemblées sont lavées avec 100 mL d'eau, avec 100 mL d'une solution aqueuse saturée en chlorure de sodium, séchées sur sulfate de sodium puis concentrées sous pression réduite. Le résidu est purifié par chromatographie sur colonne de gel de silice en éluant avec un mélange d'heptane et d'acétate d'éthyle. On isole ainsi 1,33 g (4,87 mmoles) du produit attendu sous forme d'une poudre rose.

3.2 5-chloro-1*H*-pyrrolo[2,3-*c*]pyridine-2-carboxylate d'éthyle

A une solution de 1,5 g (5,5 mmoles) de produit obtenu à l'étape 3.1 dans 50 mL d'éthanol et 25 mL de tétrahydrofurane, sont ajoutés en une fois 25 mL d'une solution aqueuse saturée de chlorure d'ammonium et 0,92 g (16,5 mmoles) de poudre de fer. Le mélange réactionnel est ensuite agité à reflux pendant 3 heures. La solution refroidie est filtrée sur célite, le filtrat est extrait à l'acétate d'éthyle, les phases organiques rassemblées sont lavées avec 100 mL d'une solution aqueuse saturée au chlorure de sodium puis séchées sur sulfate de sodium, filtrées et évaporées sous pression réduite. Le produit est purifié par chromatographie sur colonne de silice en éluant avec un mélange de n-heptane et d'acétate d'éthyle. On obtient 0,98 g (4,36 mmoles) du produit attendu sous la forme de poudre blanche.
R.M.N. $^1$H ($CDCl_3$), δ (ppm): 9,25 (s, NH) ; 8,75 (s, 1H) ; 7,70 (s, 1H) ; 7,2 (d, 1H) ; 4.5 (q, 2H) ; 1,4 (t, 3H):

3.3 5-chloro-1-[(3-fluorophényl)méthyl]-1*H*-pyrrolo[2,3-*c*]pyridine-2-carboxylate d'éthyle

A une solution de 0,25 g (1,11 mmole) de produit obtenu dans l'étape 3.2, dans 10 mL de tétrahydrofurane sec, maintenue sous atmosphère inerte, sont ajoutés successivement sous agitation, 0,21 g (1,67 mmole) d'alcool 3-fluorobenzylique puis 0,44 g (1,67 mmole) de triphénylphosphine. On ajoute, goutte à goutte à 0°C, 0,3 g (1,67 mmole) d'azodicarboxylate de diéthyle. Le mélange réactionnel est alors agité pendant 20h à température ambiante puis concentré sous pression réduite. On purifie l'huile résultante par chromatographie sur colonne de gel de silice en éluant avec un mélange de n-heptane et d'acétate d'éthyle (50/50). On isole 0,32 g (0,96 mmole) du produit attendu sous forme d'une poudre blanche.
R.M.N. $^1H$ (DMSO $D_6$), δ (ppm): 8,9 (s, 1H) ; 7,9 (s, 1 H) ; 7,3 (s, 1H) ; 7,25 (m, 1H) ; 7,1 (m, 1H) ; 6,9 (m, 2H) ; 5,9 (s, 2H) ; 4,3 (q, 2H) ; 1,3 (t, 3H).

3.4 acide 5-chloro-1-[(3-fluorophényl)méthyl]-1*H*-pyrrolo[2,3-*c*]pyridine-2-carboxylique

A une solution de 0,3 g (0,9 mmole) de produit obtenu dans l'étape 3.3, dans 10 mL d'éthanol est ajouté 0,6 mL (1,17 mmole) d'une solution 2N d'hydroxyde de sodium. Le mélange réactionnel est porté à reflux pendant 2 heures puis concentré à sec sous pression réduite. Le solide résultant est dissout dans 15 mL d'eau. Le pH de la solution est acidifié à 0°C jusqu'à pH 3 par ajouts d'acide acétique et le mélange est agité pendant 30 minutes. Le précipité formé est filtré, rincé plusieurs fois à l'eau puis séché sous pression réduite. On isole 0,25 g (0,82 mmole) du produit attendu sous forme d'une poudre blanche.
R.M.N. $^1H$ (DMSO $D_6$), δ (ppm): 8,9 (s, 1H) ; 7,9 (s, 1H) ; 7,3 (s, 1 H) ; 7,25 (m, 1H) ; 7,1 (m, 1 H) ; 6,9 (m, 2H) ; 5,9 (s, 2H)

3.5 *N*-(1,2-diméthyl-1*H*-benzimidazol-5-yl)-5-chloro-1-[(3-fluorophényl)méthyl]-1*H*-pyrrolo[2,3-*c*]pyridine-2-carboxamide

A une solution de 0,25 g (0,82 mmole) de produit obtenu à l'étape 3.4 dans 20 mL de dichlorométhane sec, sont ajoutés successivement 0,43 g (0,82 mmole) d'hexafluorophosphate de [(benzotriazol-1-yl)oxy][tris(pyrrolidino)] phosphonium puis 0,17 g (0,98 mmole) de 5-amino-1,2-diméthylbenzimidazole. A cette solution, on ajoute ensuite, goutte à goutte, 0,45 mL (2,46 mmoles) de *N*-*N*-(diisopropyl)éthylamine. Le mélange est agité pendant 2 heures à température ambiante. Le précipité rose qui se forme est filtré sur fritté, rincé plusieurs fois avec du dichlorométhane puis séché sous pression réduite. On isole ainsi 0,15 g du produit attendu sous la forme d'une poudre blanche.
Point de fusion : 240 - 242 °C
R.M.N. $^1H$ (DMSO $D_6$), δ (ppm): 10,75 (s, 1H) ; 8,85 (s, 1 H) ; 7,95 (s, 1 H) ; 7,85 (s, 1H) ; 7,5 (m, 2H) ; 7,3 (m, 2H) ; 6,95 (m, 3H) ; 5,9 (s, 2H) ; 3,75 (s, 3H) ; 2,5 (s, 3H).

**Exemple 4 (composé n°4)**

Chlorhydrate (2 : 1) de *N*-(1,2-diméthyl-1*H*-benzimidazol-5-yl)-1-[(3-fluorophényl)méthyl]-1*H*-pyrrolo[2,3-*c*]pyridine-2-carboxamide

On ajoute, sous azote et sous agitation, 1,26 mL (2,51 mmoles) de triméthylaluminium (2M dans le toluène) à une solution de 0,34 g (2 mmoles) de 1,2-diméthyl-1*H*-benzimidazole dans 20 mL de toluène sec. Après quelques minutes, on ajoute une solution de 0,5 g (1,68 mmole) de 1-[(3-fluorophényl)méthyl]-1*H*-pyrrolo[2,3-*c*]pyridine-2-carboxylate d'éthyle, préparé selon la méthode décrite à l'étape 1.3 de l'exemple 1, dans 40 mL de toluène sec. On chauffe le milieu réactionnel 3h au reflux. Après retour à température ambiante, on le verse sur un mélange de dichlorométhane et d'eau. Après élimination d'un insoluble et extraction de la phase aqueuse par du dichlorométhane, on lave, sèche et concentre sous pression réduite les phases organiques réunies. On purifie le résidu par chromatographie sur colonne de gel de silice en éluant avec un mélange de dichlorométhane et de méthanol. On isole 0,52 g (1,26 mmole) du produit attendu.
Point de fusion : 255 - 257 °C
Le sel de chlorhydrate correspondant est obtenu en mettant en réaction 0,5 g (1,21 mmole) de produit sous forme de base obtenu ci-dessus en solution dans 30 mL d'un mélange dichlorométhane/méthanol (9/1) avec 0,7 mL d'acide chlorhydrique 4N dans le dioxane. Le sel obtenu est recristallisé dans un mélange éthanol / eau (95 / 5). On obtient 0,27 g (0,55 mmole) du produit attendu.
Point de fusion: 309 - 310 °C
R.M.N. $^1H$ (DMSO $D_6$), δ (ppm): 11,5 (s, NH) ; 9,6 (s, 1H) ; 8,4-8,5 (s, 1H) ; 8,3 (s, 2H) ; 7,9 (m, 3H) ; 7,4 (m, 2H) ; 7,4 (d, 1H) ; 7,1 (m, 2H) ; 6,1 (s, 2H) ; 3,9 (s, 3H), 2,9 (s, 3H).

**Exemple 5 (composé n°8)**

*N*-(1-méthyl-1*H*-indol-5-yl)-1-[(3-fluorophényl)méthyl]-1*H*-pyrrolo[2,3-*b*]pyridine-2-carboxamide

5.1 2-(*t*-butyloxycarbonylamino)-3-méthylpyridine

Dans un tricol de 100 mL équipé d'un agitateur magnétique, on introduit 31 g (142,03 mmoles) de dicarbonate de ditertbutyle et 35 mL d'hexane que l'on porte au reflux. On ajoute alors, goutte à goutte sur une période de 2 heures, une solution de 10 g (88,77 mmoles) de 2-amino-3-méthylpyridine dans 10 mL d'acétate d'éthyle. Le reflux est maintenu 1 h après la fin de l'addition. Après retour à température ambiante, on ajoute 20 mL d'hexane et le précipité blanc formé après agitation du mélange réactionnel est recueilli par filtration, rincé à l'hexane et séché sous pression réduite. On obtient 15,5 g (74,43 mmoles) de cristaux blancs.
R.M.N. $^1H$ ($CDCl_3$), δ (ppm) : 8,3 (dd, 1H) ; 7,5 (dd, 1H) ; 7,4 (s, NH) ; 7,1 (ddd, 1H) ; 2,3 (s, 3H) ; 1,5 (s, 9H).

5.2 1 H-pyrrolo[2,3-b]pyridine -2-carboxylate d'éthyle

Dans un tricol de 250 mL équipé d'un agitateur magnétique et maintenu sous atmosphère d'azote, on introduit 5 g (24,01 mmoles) de produit obtenu dans l'étape 5.1 et 50 mL de tetrahydrofurane sec. On ajoute, goutte à goutte en maintenant la température inférieure à 5°C, 30 mL (48,02 mmoles) d'une solution de butyllithium 1,6M dans le THF. Après 1 h d'agitation à 0°C, le dérivé lithié ainsi obtenu est ajouté à une solution de 7,08 g (48,02 mmoles) d'oxalate de diéthyle dans 50 mL de tétrahydrofurane sec maintenue à une température de -3°C On laisse ensuite revenir le milieu réactionnel à température ambiante. Puis le milieu est versé dans une solution de 25 mL d'acide chlorhydrique 6N refroidie à 0°C en maintenant la température inférieure à 10°C. Le mélange obtenu est ensuite agité à 50°C pendant 2 heures puis à température ambiante pendant la nuit. Le milieu réactionnel est ajusté à pH 3 par de la soude et est extrait à l'éther diéthylique. On séche la phase organique sur du sulfate de sodium, on la filtre et on l'évapore sous pression réduite. On obtient 1,8 g (9,46 mmoles) de produit utilisé tel quel dans les étapes suivantes.
R.M.N. $^1H$ ($CDCl_3$), δ (ppm): 8,8 (dd, 1H) ; 8,15 (dd, 1H) ; 7,2 (m, 2H) ; 4,5 (q, 2H) ; 1,5 (t, 3H).

5.3 1-[(3-fluorophényl)méthyl]-1*H*-pyrrolo[2,3-*b*]pyridine-2-carboxylate d'éthyle

Méthode A : Dans un tricol de 500 mL équipé d'un agitateur magnétique et maintenu sous atmosphère d'argon, on introduit 1,64 g (41,01 mmoles) d'hydrure de sodium préalablement lavé au n-pentane puis 180 mL de diméthylformamide sec. On ajoute, par portions, 6 g (31,55 mmoles) de produit obtenu dans l'étape 5.2. Puis on porte le milieu réactionnel 1h à 50°C. On ajoute ensuite, goutte à goutte, une solution de 7,15 g (37,85 mmoles) de bromure de 3-fluorobenzyle dans 10 mL de diméthylformamide sec. Le mélange réactionnel est alors agité à reflux pendant 16h. La solution refroidie est diluée dans un mélange de 200 mL d'eau glacée et 200 mL d'acétate d'éthyle. Après décantation, la phase aqueuse est extraite à l'acétate d'éthyle et les phases organiques rassemblées sont lavées successivement avec 100 mL d'eau et 100 mL de solution aqueuse saturée de chlorure de sodium, séchées sur sulfate de sodium puis concentrées sous pression réduite. L'huile résultante est purifiée par chromatographie sur colonne de silice en éluant avec un mélange de dichlorométhane et de n-heptane. On obtient 5,73 g de produit que l'on utilise tel quel dans les étapes suivantes.
Methode B : A une solution de 5,2 g (27,34 mmoles) de produit obtenu à l'étape 5.2, dans 250 mL de tétrahydrofurane sec, maintenue sous atmosphère inerte, sont ajoutés successivement sous agitation, 5,28 g (41 mmoles) d'alcool 3-fluorobenzylique puis 10,87 g (41 mmoles) de triphénylphosphine. On ajoute ensuite, goutte à goutte à 0°C, 7,36 g (41 mmoles) d'azodicarboxylate de diéthyle. Le mélange réactionnel est alors agité pendant 20 h à température ambiante puis concentré sous pression réduite. On ajoute un mélange de pentane et d'éther diéthylique et on élimine par filtration le précipité. Après concentration sous pression réduite, on purifie l'huile résultante par chromatographies successives sur colonne de gel de silice. On isole 6,2 g de produit.
R.M.N. $^1H$ ($CDCl_3$), δ (ppm) : 8,6 (dd, 1H) ; 8,1 (dd, 1H) ; 7,4 (s, 1H) ; 7,2 (m, 2H) ; 6,95 (m, 3H) ; 6,0 (s, 2H) ; 4,4 (q, 2H) ; 1,4 (t, 3H).

5.4 *N*-(1-méthyl-1*H*-indol-5-yl)-1-[(3-fluorophényl)méthyl]-1*H*-pyrrolo[2,3-*b*]pyridine-2-carboxamide

On ajoute, à froid, sous argon et sous agitation magnétique, une solution de 0,31 g (1,75 mmole) de 5-amino-1-méthylindole dans 10 mL de toluène sec à une solution de 1,75 mL (3,50 mmoles) de triméthylaluminium dans 5 mL de toluène sec. On porte le milieu réactionnel à 50°C pendant 2h. Puis on additionne 0,52 g (1,75 mmole) d'ester obtenu à l'étape 5.3 en solution dans le toluène et on porte au reflux pendant 5h. A la solution refroidie, on ajoute de l'acétate d'éthyle, de l'eau glacée puis de l'acide chlorhydrique 1 N.

Après décantation, on extrait la phase aqueuse par de l'acétate d'éthyle. Les phases organiques rassemblées sont lavées à l'eau et par une solution saturée de chlorure de sodium, séchées et concentrées sous pression réduite. On purifie le résidu par chromatographie sur colonne de silice en éluant avec un mélange de dichlorométhane et d'acétate d'éthyle. On reprend le solide par une solution de soude et d'acétate d'éthyle, on sèche la phase organique sur sulfate de sodium et on la concentre sous pression réduite. On triture le solide obtenu par de l'éther de pétrole, on le recueille par filtration et on le sèche sous pression réduite. On isole 0,56 g du produit attendu.
Point de fusion : 191 -191,5 °C
R.M.N. $^1$H (DMSO $D_6$), δ (ppm) : 10,3 (s, 1 H) ; 8,45 (dd, 1 H) ; 8,2 (dd, 1H) ; 7,95 (s, 1H) ; 7,35 (m, 3H) ; 7,25 (m, 3H) ; 6,95 (m, 3H) ; 6,4 (d, 1H) ; 5,95 (s, 2H) ; 3,75 (s, 3H).

**Exemple 6 (composé n°9)**

Chlorhydrate (3 : 2) de *N*-(1-méthyl-1*H*-benzimidazol-5-yl)-1-[(3-fluorophényl)méthyl]-1*H*-pyrrolo[2,3-*b*]pyridine-2-carboxamide

On procède selon la méthode décrite à l'étape 5.4 de l'exemple 5 à partir de 0,5 g (1,68 mmole) de 1-[(3-fluorophényl)méthyl]-1*H*-pyrrolo[2,3-*b*]pyridine-2-carboxylate d'éthyle, préparé selon la méthode décrite à l'étape 5.3 de l'exemple 5, de 1,68 mL (3,35 mmoles) de triméthylaluminium 2M dans le toluène et de 0,30 g (2,01 mmoles) de 5-amino-1-méthylbenzimidazole. Après 3h de reflux et une nuit à température ambiante, on ajoute de l'eau glaçée et de l'acide chlorhydrique 1 N. On recueille le précipité par filtration, on le lave à l'eau et on le sèche sous pression réduite. On isole 0,42 g (1,05 mmole) de produit que l'on reprend par 15 mL d'éther diéthylique auquel on rajoute 1,1 mL d'acide chlorhydrique 2N dans l'éther diéthylique. On agite une nuit à température ambiante, on recueille le solide par filtration, on le lave à l'éther diéthylique et on le sèche sous pression réduite. On obtient 0,48 g du produit attendu sous forme de chlorhydrate.
Point de fusion : 171 -177 °C
R.M.N. $^1$H (DMSO $D_6$), δ (ppm) : 10,85 (s, NH) ; 9,5 (s, 1H) ; 8,45 (m, 2H) ; 8,25 (dd, 1H) ; 7,9 (m, 2H) ; 7,6 (s, 1H) ; 7,3 (m, 2H) ; 6,9 (m, 3H) ; 5,9 (s, 2H) ; 4,0 (s, 3H).

**Exemple 7 (composé n°10)**

*N*-(2-oxo-1,2,3,4-tétrahydroquinoléin-7-yl)-1-[(3-fluorophényl)méthyl]-1*H*-pyrrolo[2,3-*b*]pyridine-2-carboxamide

On ajoute, par portion, sous argon et sous agitation magnétique, 0,33 g (2,01 mmoles) de 7-amino-3,4-dihydroquinoléin-2(1*H*)-one à une solution de 2,51 mL (5,03 mmoles) de triméthylaluminium dans 20 mL de toluène sec. On porte le milieu réactionnel à 50°C pendant 30mn. Puis on additionne lentement 0,5 g (1,68 mmole) d'ester obtenu à l'étape 5.3 de l'exemple 5 en solution dans 5 mL de toluène et on porte au reflux pendant 2h. A la solution refroidie, on ajoute de l'eau et de l'acide chlorhydrique dilué. On recueille le précipité par filtration, on le lave à l'eau et on le sèche sous pression réduite. On le reprend par du dichlorométhane, on lave la phase organique à l'eau et avec une solution saturée de chlorure de sodium, on la sèche et on la concentre sous pression réduite. On purifie le résidu par chromatographie sur colonne de silice en éluant avec un mélange de dichlorométhane et de méthanol. On isole 0,48 g (1,16 mmole) du produit attendu. Point de fusion : 280 - 282 °C
R.M.N. $^1$H (DMSO $D_6$), δ (ppm) : 10,45 (s, NH) ; 10,1 (s, NH) ; 8,45 (d, 1H) ; 8,2 (d, 1H) ; 7,4 (m, 2H) ; 7,3-6,8 (m, 7H) ; 5,9 (s, 2H) ; 2,8 (t, 2H) ; 2,4 (t, 2H).

**Exemple 8 (composé n°11)**

Chlorhydrate (1 : 1) de *N*-(quinoléin-7-yl)-1-[(3-fluorophényl)méthyl]-1*H*-pyrrolo[2,3-*b*]pyridine-2-carboxamide

8.1 acide 1-[(3-fluorophényl)méthyl]-1*H*-pyrrolo[2,3-*b*]pyridine-2-carboxylique

On porte à reflux pendant 2h une solution de 0,6 g (2,01 mmoles) d'ester obtenu à l'étape 5.3. et de 0,23 g (4,02 mmoles) d'hydroxyde de potassium dans 60 mL de méthanol. On concentre sous pression réduite et on reprend le résidu par de l'eau que l'on acidifie par de l'acide chlorhydrique dilué. On recueille le précipité par filtration, on le lave à l'eau et on le sèche sous pression réduite. On obtient 0,37 g de produit que l'on utilise tel quel dans les étapes suivantes.

8.2 Chlorhydrate (1 : 1) de *N*-(quinoléin-7-yl)-1-[(3-fluorophényl)méthyl]-1*H*-pyrrolo[2,3-*b*]pyridine-2-carboxamide

Dans un ballon de 100 mL muni d'un agitateur magnétique, sont introduits 0,37 g (1,37 mmole) d'acide obtenu

à l'étape 8.1, 40 mL de dichlorométhane et 1 mL (13,69 mmoles) de chlorure de thionyle. La suspension ainsi obtenue est portée à reflux pendant 2h. Après évaporation du solvant à pression réduite, on ajoute 50 mL d'éther sec, 0,35 g (1,64 mmole) de dichlorhydrate de 7-aminoquinoléine (WO03068749) et une solution de 0,58 g (5,48 mmoles) de carbonate de sodium dans 5 mL d'eau. On agite le milieu réactionnel pendant une nuit et on évapore le solvant organique sous pression réduite. On ajoute de l'eau, on recueille le précipité par filtration. Le solide obtenu est repris par du dichlorométhane, on lave la phase organique à l'eau et avec une solution saturée de chlorure de sodium, on la sèche sur sulfate de sodium et on la concentre sous pression réduite. On purifie le résidu par chromatographie sur colonne de gel de silice en éluant avec un mélange de dichlorométhane et d'éthanol (95/5). On reprend le produit par 20 mL d'éther diéthylique et 1 mL d'acide chlorhydrique 2N dans l'éther diéthylique, puis on agite la solution pendant une nuit. On recueille le solide par filtration, on le lave à l'éther diéthylique. On le lave à nouveau par de l'éthanol à chaud. Après retour à température ambiante, on le recueille par filtration et on le sèche à l'étuve sous pression réduite. On obtient 0,19 g du produit attendu. Point de fusion : 260 - 262 °C
R.M.N. $^1H$ (DMSO $D_6$), $\delta$ (ppm) : 11,2 (s, 1H) ; 9,15 (d, 1H) ; 8,9 (m, 2H) ; 8,5 (m, 1H) ; 8,3 (m, 2H) ; 8,15 (m, 1 H) ; 7,85 (m, 1H) ; 7,65 (s, 1 H) ; 7,3 (m, 2H) ; 6,9 (m, 3H) ; 6,0 (s, 2H).

**Exemple 9 (composé n°12)**

*N*-(1-méthyl-1*H*-indol-5-yl)-1-(phénylméthyl)-1*H*-pyrrolo[2,3-*b*]pyridine-2-carboxamide

Le 1-(phénylméthyl)-1*H*-pyrrolo[2,3-*b*]pyridine-2-carboxylate d'éthyle est préalablement préparé selon la méthode décrite dans l'exemple 5.3B à partir du produit obtenu dans l'exemple 5.2 et d'alcool benzylique. On procède ensuite selon la méthode décrite à l'étape 5.4 de l'exemple 5 à partir de 1-(phénylméthyl)-1*H*-pyrrolo[2,3-*b*]pyridine-2-carboxylate d'éthyle (1 éq), de triméthylaluminium 2M dans le toluène (1,5 éq) et de 5-amino-1-méthylindole (1,2 éq). On purifie le brut réactionnel par chromatographie sur colonne de gel de silice.
Point de fusion : 181 - 182 °C
R.M.N. $^1H$ (DMSO $D_6$), $\delta$ (ppm) :10,3 (1H, NH) ; 8,45 (d, 1H) ; 8,2 (d, 1H) ; 7,95 (s, 1H) ; 7,4-7,05 (m, 10H) ; 6,4 (d, 1H) ; 5,95 (s, 2H) , 3,8 (s, 3H).

**Exemple 10 (composé n°13)**

*N*-(1-méthyl-1*H*-indol-5-yl)-1-(phényléthyl)-1*H*-pyrrolo[2,3-*b*]pyridine-2-carboxamide

Le 1-(phényléthyl)-1*H*-pyrrolo[2,3-*b*]pyridine-2-carboxylate d'éthyle est préalablement préparé selon la méthode décrite dans l'exemple 5.3B à partir du produit obtenu dans l'exemple 5.2 et de 2-phényléthanol. On procède ensuite selon la méthode décrite à l'étape 5.4 de l'exemple 5 à partir de 1-(phényléthyl)-1*H*-pyrrolo[2,3-*b*]pyridine-2-carboxylate d'éthyle (1 éq), de triméthylaluminium 2M dans le toluène (1,5 éq) et de 5-amino-1-méthylindole (1,2 éq). On purifie le brut réactionnel par chromatographie sur colonne de gel de silice.
Point de fusion : 196 - 199 °C
R.M.N. $^1H$ (DMSO $D_6$), $\delta$ (ppm) : 10,25 (s, NH) ; 8,45 (d, 1 H) ; 8,15 (dd, 1H) ; 8,0 (s, 1H) ; 7,5-7,1 (m, 10H) ; 6,45 (d, 1H) ; 4,9 (t, 2H) ; 3,8 (s, 3H) ; 3,05 (t, 2H).

**Exemple 11 (composé n°14)**

chlorhydrate (3 : 2) de *N*-(2-méthyl-benzothiazol-5-yl)-1-[(3-fluorophényl)méthyl]-1*H*-pyrrolo[2,3-*b*]pyridine-2-carboxamide

Dans un ballon de 100 mL refroidi à 0°C et muni d'une agitation magnétique, est introduit sous balayage d'azote, 1,68 mL (3,35 mmoles) d'une solution 2M de triméthylaluminium dans le toluène et 20 mL de toluène sec. On ajoute ensuite, par petites portions, 0,33 g (2,01 mmoles) de 5-amino-2-méthylbenzothiazole. Le mélange réactionnel est porté à 50°C pendant 30 mn puis on ajoute, goutte à goutte en 5 mn, une solution de 0,5 g (1,68 mmole) d'ester obtenu à l'étape 5.3 dans 20 mL de toluène sec. Le mélange réactionnel est alors porté à reflux pendant 4 heures. A la solution refroidie à 0°C sont ensuite ajoutés 50 mL d'eau glacée et 20 mL d'acétate d'éthyle. Après 30 minutes d'agitation, le solide formé est éliminé par filtration, lavé à l'eau et à l'acétate d'éthyle. Après décantation, la phase aqueuse est extraite à l'acétate d'éthyle et les phases organiques rassemblées sont lavées successivement par de l'eau et une solution aqueuse saturée de chlorure de sodium, séchées sur sulfate de sodium puis concentrées sous pression réduite. Le solide résultant est purifié par chromatographie sur colonne de silice en éluant avec un mélange de dichlorométhane et d'acétate d'éthyle. On prépare le chlorhydrate correspondant par traitement avec une solution d'acide chlorhydrique dans l'éther diéthylique. On isole 0,475 g du produit attendu.

Point de fusion : 211 - 212 °C
R.M.N. $^{1}H$ (DMSO $D_6$), δ (ppm) : 10,65 (s, 1H) ; 8,5 (dd, 1H) ; 8,4 (d, 1H) ; 8,2 (dd, 1H) ; 7,95 (d, 1H) ; 7,75 (dd, 1H) ; 7,5 (s, 1H) ; 7,3 (m, 2H) ; 6,9 (m, 3H) ; 5,9 (s, 2H) ; 2,8 (s, 3H).

**Exemple 12 (composé n°15)**

*N*-(1-méthyl-1*H*-benzimidazol-5-yl)-5-fluoro-1-[(3-fluorophényl)méthyl]-1*H*-pyrrolo[2,3-*b*]pyridine-2-carboxamide

12.1 2-amino-3-iodo-5-fluoropyridine

Dans un bicol de 500 mL, muni d'un agitateur magnétique sont introduits, 5 g (44,6 mmoles) de 2-amino-5-fluoropyridine, 13,9 g (44,6 mmoles) de sulfate d'argent et 400 mL d'éthanol. On ajoute ensuite par petite portion 11,31 g (44,6 mmoles) d'iode en poudre. L'agitation est poursuivie à température ambiante pendant 24 heures. La suspension jaune résultante est filtrée, le précipité est rincé à l'éthanol et le filtrat est concentré sous pression réduite. Le résidu ainsi obtenu est repris dans un mélange d'acétate d'éthyle (200 mL) et d'une solution de carbonate de sodium (200 mL). Après séparation, la phase organique est lavée successivement avec une solution aqueuse de thiosulfate de sodium à 25%, avec une solution aqueuse saturée au chlorure de sodium puis séchée sur sulfate de sodium et concentrée sous pression réduite. Le solide résultant est purifié par chromatographie sur colonne de silice en éluant avec un mélange de n-heptane et d'acétate d'éthyle. On obtient 2,67 g (11,22 mmoles) du produit attendu.
R.M.N. $^{1}H$ (DMSO $D_6$), δ (ppm) : 7,95 (s, 1H) ; 7,85 (s, 1H) ; 5,9 (s, NH2).

12.2 acide 5-fluoro-1*H*-pyrrolo[2,3-*b*]pyridine-2-carboxylique

Dans un tube scellé de 25 mL, muni d'un agitateur magnétique et maintenu sous bullage d'argon, sont introduits, 0,5 g (2,10 mmoles) de 2-amino-3-iodo-5-fluoropyridine obtenu à l'étape 12.1 , 0,55 g (6,3 mmoles) d'acide pyruvique, 0,71g (6,3 mmoles) de 1,4-diazabicyclo[2.2.2]octane (DABCO) et 15 mL de diméthylformamide anhydre. Après quelques minutes, on ajoute 0,05 g (0,22 mmole) d'acétate de palladium. Le mélange réactionnel est maintenu sous agitation et sous bullage d'argon pendant 20 minutes puis rapidement scellé et porté à 100 °C pendant 2h30. La solution refroidie est concentrée à sec sous pression réduite. Le résidu est ensuite repris par de l'acétate d'éthyle (100 mL) et de l'eau (75 mL). La phase organique est lavée à l'eau puis extraite par deux fois 50 mL d'une solution aqueuse de soude 2N. Les phases aqueuses basiques sont rassemblées, refroidies à 0°C puis acidifiées par ajout d'acide chlorhydrique (pH 3). On extrait le milieu par de l'acétate d'éthyle (4x50 mL), les phases organiques rassemblées sont séchées sur du sulfate de sodium puis concentrées sous pression réduite. On obtient 0,158 g (0,88 mmole) du produit attendu sous forme d'une poudre jaune.
R.M.N. $^{1}H$ (DMSO $D_6$), δ (ppm) : 13,2 (s, 1H) : 12,4 (s, 1H) ; 8,4 (d, 1H) ; 7,95 (dd, 1H) ; 7,1 (d, 1 H).

12.3 5-fluoro-1*H*-pyrrolo[2,3-*b*]pyridine-2-carboxylate d'éthyle

Dans un ballon de 100 mL, muni d'un agitateur magnétique, sont introduits 0,2 g ( 1,11 mmole) d'acide obtenu à l'étape 12.2 et 10 mL d'éthanol. On ajoute 1 mL d'acide sulfurique concentré au mélange réactionnel que l'on porte ensuite à reflux pendant 18 heures. La solution refroidie est concentrée à sec sous pression réduite. Le résidu est repris par de l'acétate d'éthyle (50 mL) que l'on lave successivement avec une solution aqueuse de soude normale (2 x 10 mL), avec de l'eau (10 mL) puis avec une solution aqueuse saturée en chlorure de sodium. On sèche la phase organique sur sulfate de sodium puis on la concentre sous pression réduite. On isole 0,21 g du produit attendu.
R.M.N. $^{1}H$ (DMSO $D_6$), δ (ppm): 12,6 (s, NH) ; 8,4 (d, 1H) ; 8,0 (dd, 1H) ; 7,1 (d, 1H) ; 4,35 (q, 2H) ; 1,35 (t, 3H).

12.4 5-fluoro-1-[(3-fluorophényl)méthyl]-1*H*-pyrrolo[2,3-*b*]pyridine-2-carboxylate d'éthyle

A une solution de 0,2 g (0,96 mmole) de produit obtenu à l'étape 12.3, dans 15 mL de tétrahydrofurane sec, maintenue sous atmosphère inerte, sont ajoutés successivement sous agitation, 0,18 g (1,44 mmole) d'alcool 3-fluorobenzylique puis 0,39 g (1,44 mmole) de triphénylphosphine. On ajoute ensuite, goutte à goutte à 0°C, 0,26 g (1,44 mmole) d'azodicarboxylate de diéthyle. Le mélange réactionnel est alors agité pendant 20h à température ambiante puis concentré sous pression réduite. On purifie l'huile résultante par chromatographie sur colonne de gel de silice en éluant avec un mélange de dichlorométhane et de méthanol. On isole 0,26 g (0,82 mmole) du produit attendu.

12.5 *N*-(1-méthyl-1*H*-benzimidazol-5-yl)-5-fluoro-1-[(3-fluorophényl)méthyl]-1*H*-pyrrolo[2,3-*b*]pyridine-2-carboxamide

Dans un tricol de 100 mL refroidi à 0°C et muni d'une agitation magnétique, sont introduits sous balayage

d'azote, 0,18 g (1,23 mmole) de 5-amino-1-méthylbenzimidazole et 10 mL de toluène sec. A cette solution, on ajoute ensuite lentement 0,95 mL (1,90 mmole) d'une solution 2M de triméthylaluminium dans le toluène. Le mélange réactionnel obtenu est maintenu sous atmosphère d'azote et agité en laissant la température progressivement atteindre 70 °C. On ajoute alors, goutte à goutte en 5 minutes à l'aidé d'une ampoule d'addition, une solution de 0,3 g (0,95 mmole) de produit obtenu à l'étape 12.4, dans 10 mL de toluène sec. Le mélange réactionnel est alors porté à reflux pendant 5 heures puis agité à température ambiante pendant la nuit. A la solution refroidie à 0°C sont ensuite ajoutés 20 mL d'eau froide suivie de l'addition de 10 mL d'acide chlorhydrique 1N. Après 1 h d'agitation, on recueille le précipité par filtration, on le rince à l'eau et on le sèche sous pression réduite. On obtient 0,22 g (0,53 mmole) du produit attendu sous la forme d'un solide blanc.
Point de fusion : 266 - 268 °C
R.M.N. $^{1}$H (DMSO $D_6$), δ (ppm) : 9,5 (s, 1H) ; 8,5 (s, 1H) ; 7,9 (s, 2H) ; 7,5 (m, 3H) ; 7,3-6,8 (m, 5H) ; 5,9 (s, 2H) ; 3,95 (s, 3H).

**Exempte 13 (composé n°16)**

*N*-(1,2-diméthyl-1*H*-benzimidazol-5-yl)-5-trifluorométhyl-1-[(3-fluorophényl)méthyl]-1*H*-pyrrolo[2,3-*b*]pyridine-2-carboxamide

13.1 2-amino-3-iodo-5-(trifluorométhyl)pyridine

Dans un bicol de 500 mL, muni d'un agitateur magnétique sont introduits, 2 g (12,34 mmoles) de 2-amino-5-trifluorométhylpyridine, 3,85 g (12,34 mmoles) de sulfate d'argent et 80 mL d'éthanol. On ajoute ensuite, par petites portions, au milieu réactionnel agité à température ambiante, 3,13 g (12,34 mmoles) d'iode en poudre. Le mélange réactionnel est alors agité à température ambiante pendant 48 heures. La suspension jaune résultante est filtrée, le précipité rincé à l'éthanol et le filtrat évaporé sous pression réduite. Le résidu ainsi obtenu est repris avec du dichlorométhane (200 mL). On lave la phase organique successivement avec une solution aqueuse de soude à 5%, à l'eau puis avec une solution aqueuse saturée au chlorure de sodium. On la sèche sur sulfate de sodium et on la concentre sous pression réduite. Le solide résultant est purifié par chromatographie sur colonne de silice en éluant avec un mélange de n-heptane et d'acétate d'éthyle. On obtient 1,71 g (5,94 mmoles) de produit sous la forme de poudre rose.
R.M.N. $^{1}$H (DMSO $D_6$), δ (ppm) : 8,3 (s, 1H) ; 8,1 (s, 1H) ; 6,8 (s, NH2).

13.2 acide 5-trifluorométhyl-1*H*-pyrrolo[2,3-*b*]pyridine-2-carboxylique

Dans un tube scellé de 25 mL, muni d'un agitateur magnétique et maintenu sous bullage d'argon, sont introduits, 2 g (6,94 mmoles) de produit obtenu à l'étape 13.1, 1,51 g (20,83 mmoles) d'acide pyruvique, 2,41 g (20,83 mmoles) de 1,4-diazabicyclo[2.2.2]octane (DABCO) et 20 mL de diméthylformamide anhydre. Après quelques minutes, on ajoute 2 g (8,91 mmoles) d'acétate de palladium. Le mélange réactionnel est maintenu sous agitation et sous bullage d'argon pendant 20 minutes puis rapidement scellé et porté à 110 °C pendant 6h. La solution refroidie est concentrée à sec sous pression réduite. Le résidu est repris par de l'acétate d'éthyle et de l'eau. Après décantation, on extrait la phase organique par deux fois 50 mL d'une solution aqueuse de soude 2N. Les phases aqueuses basiques sont rassemblées, refroidies à 0°C puis acidifiées par ajout d'acide chlorhydrique (pH 3). On extrait la phase aqueuse par de l'acétate d'éthyle (4x50 mL), les phases organiques rassemblées sont séchées sur sulfate de sodium puis concentrées sous pression réduite. On obtient 0,67 g (2,91 mmoles) du produit attendu sous forme de poudre jaune que l'on utilisé tel quel dans les étapes suivantes.
R.M.N. $^{1}$H (DMSO $D_6$), δ (ppm) : 12,8 (s, 1H) ; 8,7 (d, 1 H) ; 8,5 (d, 1H) ; 7,2 (s, 1 H).

13.3 5-trifluorométhyl-1*H*-pyrrolo[2,3-*b*]pyridine-2-carboxylate d'éthyle

Dans un ballon de 100 mL, muni d'un agitateur magnétique, est introduit 0,3 g (1,3 mmole) de l'acide obtenu à l'étape 13.2 et 50 mL d'éthanol. A cette solution, on ajoute 0,5 mL d'acide sulfurique concentré. Le mélange réactionnel est ensuite porté à reflux pendant 18 heures. La solution refroidie est concentrée à sec sous pression réduite. Le résidu est repris au dichlorométhane (100 mL), on lave la phase organique successivement avec une solution aqueuse de soude normale (30 mL), avec de l'eau (20 mL) puis avec une solution aqueuse saturée en chlorure de sodium. On la sèche sur sulfate de sodium puis on la concentre sous pression réduite. On isole 0,29 g (1,12 mmole) du produit attendu sous forme d'une poudre jaune.
R.M.N. $^{1}$H (DMSO $D_6$), δ (ppm) : 12,95 (s, NH) ; 8,8 (d, 1H) ; 8,6 (d, 1H) ; 7,3 (s, 1 H) ; 4,4 (q, 2H) ; 1.35 (t, 3H).

13.4 5-trifluorométhyl-1-[(3-fluorophényl)méthyl]-1*H*-pyrrolo[2,3-*b*]pyridine-2-carboxylate d'éthyle

A une solution de 0,3 g (1,16 mmole) de produit obtenu à l'étape 13.3, dans 20 mL de tétrahydrofurane sec, maintenue sous atmosphère inerte, sont ajoutés successivement sous agitation, 0,23 g (1,74 mmole) d'alcool 3-fluorobenzylique puis 0,46 g (1,74 mmole) de triphénylphosphine. On ajoute ensuite, goutte à goutte, 0,31 g (1,74 mmole) d'azodicarboxylate de diéthyle. Le mélange réactionnel est alors agité pendant 20 h à température ambiante puis concentré sous pression réduite. On purifie l'huile résultante par chromatographie sur colonne de gel de silice en éluant avec un mélange d'heptane et d'acétate d'éthyle. On isole 0,34 g (0,93 mmole) du produit attendu.
R.M.N. $^1$H (DMSO $D_6$), δ (ppm) : 8,9 (d, 1H) ; 8,7 (d, 1H) ; 7,5 (s, 1H) ; 7,4-6,95 (m, 2H) ; 6,85 (m, 2H) ; 5,9 (s, 2H) ; 4,3 (q, 2H) , 1,3 (t, 3H).

13.5 *N*-(1,2-diméthyl-1*H*-benzimidazol-5-yl)-5-trifluorométhyl-1-[(3-fluorophényl)méthyl]-1*H*-pyrrolo[2,3-*b*]pyridine-2-carboxamide

Dans un tricol de 100 mL refroidi à 0°C et muni d'une agitation magnétique, sont introduits sous balayage d'azote, 0,17 g (1 mmole) de 5-amino-1,2-diméthylbenzimidazole et 10 mL de toluène sec. A cette solution, on ajoute ensuite lentement 0,77 mL (1,54 mmole) d'une solution 2M de triméthylaluminium dans le toluène. Le mélange réactionnel obtenu est maintenu sous atmosphère d'azote et agité en laissant la température progressivement atteindre 70°C. A cette température, on ajoute, goutte à goutte en 5 minutes, une solution de 0,3 g (0,77 mmole) de produit obtenu à l'étape 13.4 dans 10 mL de toluène sec. Le mélange réactionnel est alors porté à reflux pendant 4 heures. A la solution refroidie à 0°C sont ensuite ajoutés 20 mL d'eau froide. Après 90 minutes d'agitation, le précipité qui se forme est extrait trois fois à l'acétate d'éthyle (3x50 mol) et les phases organiques rassemblées sont lavées successivement avec 20 mL d'une solution aqueuse saturée d'hydrogénocarbonate de sodium, 40 mL d'eau et 20 mL de solution aqueuse saturée de chlorure de sodium, séchées sur sulfate de sodium puis concentrées sous pression réduite.
Le solide résultant est purifié par chromatographie sur colonne de silice en éluant avec un mélange de dichlorométhane et de méthanol. Le solide obtenu est recristallisé dans un mélange isopropanol/éthanol (9/1) pour donner 0,23 g (0,48 mmole) du produit attendu sous la forme de cristaux blancs.
Point de fusion : 263 - 265 °C
R.M.N. $^1$H (DMSO $D_6$), δ (ppm) : 11 (s, 1H) ; 8,85 (s, 1H) ; 8,75 (s, 1H) ; 8,3 (s, 1 H) ; 7,9 (m, 2H) ; 7,7 (s, 1 H) ; 7,3 (m, 1 H) ; 6,95 (m, 3H) ; 5,95 (s, 2H) ; 3,9 (s, 3H) ; 2,8 (s, 3H).

**<u>Exemple 14</u> (composé n°18)**

*N*-(2-méthyl-1*H*-benzothiazol-5-yl)-5-trifluorométhyl-1-[(3-fluorophényl)méthyl]-1*H*-pyn-olo[2,3-*b*]pyridine-2-carboxamide
On procède selon la méthode décrite à l'étape 13.5 de l'exemple 13, à partir de 0,35 g (0,96 mmole) de 5-trifluorométhyl-1-[(3-fluorophényl)méthyl]-1*H*-pyrrolo[2,3-*b*]pyridine-2-carboxylate d'éthyle (exemple 13.4) et de 0,17 g (1,06 mmole) de 5-amino-2-méthylbenzothiazole. On isole 0,34 g de composé attendu.
Point de fusion : 204 - 206 °C
R.M.N. $^1$H (DMSO $D_6$), δ (ppm) : 10,78 (s, 1H) ; 8,8 (s, 1H) ; 8,7 (s, 1H) ; 8,31 (s, 1H) ; 7,97 (d, 1H) ; 7,71 (d, 1 H) ; 7,6 (s, 1H) ; 7,28 (m, 1H) ; 6,95 (m, 3H) ; 5,95 (d, 2H) ; 2,8 (s, 3H).

**<u>Exemple 15</u> (composé n°19)**

*N*-(1,2-diméthyl-1*H*-benzimidazol-5-yl)-1-[(3-fluorophényl)méthyl]-1*H*-pyrrolo[2,3-*b*]pyridine-2-carboxamide
On procède selon la méthode décrite à l'exemple 11, à partir de 0,4 g (1,34 mmole) de 1-[(3-fluorophényl)méthyl]-1*H*-pyrrolo[2,3-*b*]pyridine-2-carboxylate d'éthyle (exemple 5.3) et de 0,25 g (1,61 mmole) de 5-amino-1,2-diméthylbenzimidazole. On isole 0,477 g de composé attendu.
Point de fusion : 242 - 244 °C
R.M.N. $^1$H (DMSO $D_6$), δ (ppm) : 2,49 (s, 3H) ; 3,69 (s, 3H) ; 5,91 (s, 2H) ; 6,94 (m, 3H) ; 7,22 (m, 2H) ; 7,39 (m, 3H) ; 7,79 (s, 1 H) ; 8,19 (dxd, 1H) ; 8.41 (d, 1 H) ; 10,31 (s, 1 H).

**<u>Exemple 16</u> (composé n°6)**

*N*-(1,2-diméthyl-1*H*-benzimidazol-5-yl)-5-fluoro-1-[(3-fluorophényl)méthyl]-1*H*-pyrrolo[2,3-*c*]pyridine-2-carboxamide

On procède selon la méthode décrite à l'étape 2.4 de l'exemple 2, à partir de 0,3 g (0,95 mmole) de 5-fluoro-1-[(3-fluorophényl)méthyl]-1*H*-pyrrolo[2,3-*c*]pyridine-2-carboxylate d'éthyle (exemple 2.3) et de 0,183 g (1,14 mmole)

de 5-amino-1,2-diméthyl-benzimidazole. On isole 0,21 g de composé attendu.
Point de fusion : 245 - 247 °C
R.M.N. $^1$H (DMSO $D_6$), δ (ppm) : 10,59 (s, 1H) ; 8,62 (s, 1H) ; 7,91 (s, 1H) ; 7,4 (m, 5H) ; 7,01 (m, 3H) ; 5,91 (s, 2H) ; 3,71 (s, 3H) ; 2;49 (s, 3H).

**Exemple 17 (composé n°5)**

*N*-(2-méthyl-1*H*-benzothiazol-5-yl)-5-fluoro-1-[(3-fluorophényl)méthyl]-1*H*-pyrrolo[2,3-*c*]pyridine-2-carboxamide

On procède selon la méthode décrite à l'étape 2.4 de l'exemple 2, à partir de 0,3 g (0,95 mmole) de 5-fluoro-1-[(3-fluorophényl)méthyl]-1*H*-pyrrolo[2,3-*c*]pyridine-2-carboxylate d'éthyle (exemple 2.3) et de 0,189 mg (1,14 mmole) de 5-amino-2-méthyl-benzothiazole. On isole 0,36 g de composé attendu.
Point de fusion : 193 - 195 °C
R.M.N. $^1$H (DMSO $D_6$), δ (ppm) : 10,87 (s, 1H) ; 8,68 (s, 1H) ; 8,32 (s, 1H) ; 7,98 (d, 1H) ; 7,71 (d, 1 H) ; 7,35 (m, 3H) ; 6,99 (m, 3H) ; 5,9 (s, 2H) ; 2,79 (s, 3H).

**Exemple 18 (composé n°7)**

*N*-(1,2-diméthyl-1*H*-benzimidazol-5-yl)-5-phényl-1-[(3-fluorophényl)méthyl]-1*H* pyrrolo[2,3-*c*]pyridine-2-carboxamide

18.1 4-méthyl-5-nitro-2-phényl-pyridine

On chauffe à reflux pendant 12 heures un mélange de 2 g (11,59 mmoles) de 2-chloro-4-méthyl-5-nitro-pyridine, de 1,41 g (11,59 mmoles) d'acide phénylboronique, de 1,33 g (1,16 mmole) de tetrakis(triphénylphosphine)palladium et de 4 g (28,97 mmoles) de carbonate de potassium en suspension dans 50 mL de dioxane dégazé. Le mélange est ensuite refroidi, dilué avec 50 mL d'acétate d'éthyle, lavé successivement deux fois avec 20 mL d'eau puis avec 20 mL d'une solution saturée en chlorure de sodium, séché sur sulfate de sodium, filtré puis concentré sous pression réduite. Le résidu obtenu est purifié par chromatographie sur colonne de silice (éluants : heptane - acétate d'éthyle) puis recristallisé dans un mélange isopropanol - éther isopropylique. On isole ainsi 2,19 g d'un solide jaune qui sera engagé tel quel dans la suite de la synthèse.

18.2 3-(5-nitro-2-phényl-pyridin-4-yl)-2-oxo-propionate d'éthyle

On agite à température ambiante, pendant 4 heures, un mélange de 1,3 g (6,07 mmoles) de 4-méthyl-3-nitro-2-phényl-pyridine, obtenue à l'étape 18.1, et de 1,01 g (6,68 mmoles) de 1,8-diazabicyclo[5.4.0]undec-7-ène dans 4,14 mL d'oxalate de diéthyle. Après ce temps, le mélange est dilué avec 30 mL d'acétate d'éthyle, 20 mL d'eau et 2 mL d'acide acétique. La solution obtenue est extraite deux fois avec 50 mL d'acétate d'éthyle. Les phases organiques rassemblées sont lavées successivement deux fois avec 20 mL d'eau puis avec 20 mL d'une solution saturée en chlorure de sodium, séchées sur sulfate de sodium, filtrées puis concentrées sous pression réduite. Le résidu obtenu est purifié par chromatographie sur colonne de silice (éluants : heptane - acétate d'éthyle). On isole ainsi 1,46 g du produit attendu, sous la forme d'un solide blanc.

18.3 5-phényl-1*H*-pyrrolo[2,3-*c*]pyridine-2-carboxylate d'éthyle.

On porte à reflux pendant trois heures un mélange de 1,3 g (4,3 mmoles) de 3-(5-nitro-2-phényl-pyridin-4-yl)-2-oxo-propionate d'éthyle, obtenu à l'étape 18.2, et 0,71 g (12,89 mmoles) de poudre de fer dans un mélange de 20 mL d'une solution saturée en chlorure d'ammonium, 20 mL de tétrahydrofurane et 40 mL d'éthanol. Le mélange réactionnel est ensuite refroidi puis filtré sur un tampon de célite. Le filtrat est concentré au tiers de son volume sous pression réduite, puis extrait trois fois avec 50 mL d'acétate d'éthyle. Les phases organiques rassemblées sont lavées successivement deux fois avec 20 mL d'eau puis 20 mL d'une solution saturée en chlorure de sodium, séchées sur sulfate de sodium, filtrées puis concentrées sous pression réduite. Le résidu obtenu est purifié par chromatographie sur colonne de silice (éluants : heptane - acétate d'éthyle). On isole ainsi 0,93 g du produit attendu, sous la forme d'une poudre beige.

18.4 5-phényl-1-[(3-fluorophényl)méthyl]-1*H*-pyrrolo[2,3-*c*]pyridine-2-carboxylate d'éthyle

A une solution, agitée à 0°C sous argon, de 1 g (3,76 mmoles) de 5-phényl-1*H*-pyrrolo[2,3-*c*]pyridine-2-carboxylate d'éthyle, obtenu à l'étape 18.3, dans 30 mL de tétrahydrofurane sec, sont ajoutés successivement 0,72 g (5,63 mmoles) d'alcool 3-fluorobenzylique, 1,47 g (5,63 mmoles) de triphénylphosphine puis 1,01 g (5,63 mmoles) d'azodicarboxylate de diéthyle. Le mélange est ensuite agité durant 20 heures à 20°C, concentré sous pression réduite puis purifié par chromatographie sur colonne de silice (éluants : heptane - acétate d'éthyle). On isole ainsi 1,1 g du produit attendu, sous la forme d'une poudre blanche.

18.5 *N*-(1,2-diméthyl-1*H*-benzimidazol-5-yl)-5-phényl-1-[(3-fluorophényl)méthyl]-1*H*-pyrrolo[2,3-*c*]pyridine-2-carboxamide (composé n° 7)

Dans un tricol de 100 mL refroidi à 0°C et muni d'une agitation magnétique, sont introduits sous balayage d'azote, 0,217 g (1,35 mmole) de 5-amino-1,2-diméthylbenzimidazole et 5 mL de toluène sec. A cette solution, on ajoute ensuite lentement 0,84 mL (1,68 mmole) d'une solution 2M de triméthylaluminium dans le toluène. Le mélange réactionnel obtenu est maintenu sous atmosphère d'azote et agité en laissant la température progressivement atteindre 70°C. A cette température, on ajoute, goutte à goutte en 5 minutes, une solution de 0,42 g (1,12 mmole) de 5-phényl-1-[(3-fluorophényl)méthyl]-1*H*-pyrrolo[2,3-*c*]pyridine-2-carboxylate d'éthyle, obtenu à l'étape 18.4, dans 10 mL de toluène sec. Le mélange réactionnel est alors porté à reflux pendant 4 heures. A la solution refroidie à 0°C sont ensuite ajoutés 20 mL d'eau froide. Après 90 minutes d'agitation, la solution est extraite trois fois à l'acétate d'éthyle (3x50 mL) et les phases organiques rassemblées sont lavées successivement avec 20 mL d'une solution aqueuse saturée d'hydrogénocarbonate de sodium, 40 mL d'eau et 20 mL de solution aqueuse saturée de chlorure de sodium, séchées sur sulfate de sodium puis concentrées sous pression réduite.
Le solide résultant est trituré dans l'éther isopropylique bouillant puis recristallisé dans un mélange isopropanol/méthanol (9/1) pour donner 0,187 g du produit attendu sous la forme d'une poudre beige.
Point de fusion : 288 - 290 °C
R.M.N. $^1H$ (DMSO $D_6$), $\delta$ (ppm) : 10,6 (s, 1H) ; 9,08 (s, 1H) ; 8,27 (s, 1H) ; 8,1(d, 2H) ; 7,98 (s, 1H) ; 7,41 (m, 7H) ; 7,01 (m, 3H) ; 5,98 (s, 2H) ; 3,71 (s, 3H) ; 2,48 (s, 3H).

**Exemple 19 (composé n°24)**

*N*-(1,2-diméthyl-1*H*-benzimidazol-5-yl)-5-trifluoromethyl-1-[(3-fluorophényl)méthyl]-1*H*-pyrrolo[3,2-*b*]pyridine-2-carboxamide

19.1 3-amino-2-iodo-6-trifluorométhyl-pyridine

On ajoute, en plusieurs portions, 1,56 g (6,17 mmoles) d'iode à un mélange, agité sous argon à 20°C, de 1 g (6,17 mmoles) de 3-amino-6-trifluorométhyl-pyridine et de 1,25 g (6,17 mmoles) de sulfate d'argent dans 40 mL d'éthanol. L'agitation est maintenue pendant 18 heures. La suspension jaune résultante est filtrée et rincée à l'éthanol. Le filtrat est concentré sous pression réduite, le résidu est repris dans 100 mL de dichlorométhane. La phase organique est lavée successivement avec 20 mL d'une solution aqueuse de soude à 5%, 40 mL d'eau et 20 mL de solution aqueuse saturée de chlorure de sodium, séchée sur sulfate de sodium, concentrée sous pression rréduite puis purifiée par chromatographie sur colonne de silice (éluants : heptane - acétate d'éthyle). On isole ainsi 1,17 g du produit attendu que l'on engage tel quel dans la suite de la synthèse.

19.2 acide 5-trifluorométhyl-pyrrolo[3,2-*b*]pyridine-2-carboxylique

On introduit sous argon, dans un tube scellé, 0,5 g (1,74 mmole) de 3-amino-2-iodo-6-trifluorométhyl-pyridine, obtenu à l'étape 19.1, 0,45 g (5,21 mmoles) d'acide pyruvique, 0,51 mL (5,21 mmoles) de 1,4-diazabicyclo[2.2.2]octane et 10 mL de diméthylformamide sec. La solution est dégazée quelques minutes puis on ajoute 0,19 g (0,87 mmole) d'acétate de palladium, on ferme le tube et on porte au reflux à 130°C durant 4 heures. La solution refroidie est ensuite concentrée sous pression réduite et le résidu résultant est repris par 100 mL d'acétate d 'éthyle. La phase organique est lavée successivement avec deux fois 50 mL d'une solution aqueuse de soude 2N. Les phases aqueuses basiques sont réunies, refroidies à 0°C, acidifiées par ajouts d'acide chlorhydrique puis extraites par 4 fois 50 mL d'acétate d'éthyle. Ces phases organiques sont rassemblées, lavées avec 20 mL d'une solution aqueuse saturée en chlorure de sodium, séchées sur sulfate de sodium puis concentrées sous pression réduite. On obtient 0,22 g de produit que l'on utilise tel quel dans l'étape suivante.

19.3 5-trifluorométhyl-pyrrolo[3,2-*b*]pyridine-2-carboxylate d'éthyle

On ajoute 1 mL (18,71 mmoles) d'acide sulfurique concentré à une solution de 0,2 g (0,87 mmole) d'acide 5-trifluorométhyl-pyrrolo[3,2-*b*]pyridine-2-carboxylique, obtenu à l'étape 19.2, dans 10 mL d'éthanol. On agite à reflux durant 20 heures puis on refroidit la solution quel l'on concentre sous pression réduite. Le résidu résultant est ensuite

repris avec 50 mL de dichlorométhane puis lavé successivement avec 20 mL d'une solution aqueuse saturée d'hydrogénocarbonate de sodium, 40 mL d'eau et 20 mL de solution aqueuse saturée de chlorure de sodium, séchée sur sulfate de sodium puis concentrée sous pression réduite. On obtient 0,19 g de produit que l'on utilise tel quel dans l'étape suivante.

19.4 1-(3-fluorobenzyl)-5-trifluorométhyl-pyrrolo[3,2-*b*]pyridine-2-carboxylate d'éthyle

A une solution de 0,2 g (0,77 mL) de 5-trifluorométhyl-pyrrolo[3,2-*b*]pyridine-2-carboxylate d'éthyle, obtenu à l'étape 19.3, dans 120 mL de tétrahydrofurane sec, maintenue à 0˚C sous argon, sont ajoutés successivement 0,13 mL (1,16 mmole) d'alcool 3-fluorobenzylique, 0,3 g (1,16 mmole) de triphénylphosphine puis 0,2 g (1,16 mmole) d'azodicarboxylate de diéthyle. Le mélange réactionnel est agité durant 20 heures à 20˚C puis concentré sous pression réduite. Le résidu résultant est purifié par chromatographie sur colonne de silice (éluants : heptane - acétate d'éthyle). On isole ainsi 0,21 g du produit attendu sous la forme d'une huile jaune.

19.5 *N*-(1,2-diméthyl-1*H*-benzimidazol-5-yl)-5-trifluoromethyl-1-[(3-fluorophényl)méthyl]-1*H*-pyrrolo[3,2-*b*]pyridine-2-carboxamide (composé n˚ 24)

Dans un tricol de 100 mL refroidi à 0˚C et muni d'une agitation magnétique, sont introduits sous balayage d'azote, 0,097 g (0.6 mmole) de 5-amino-1,2-diméthylbenzimidazole et 5 mL de toluène sec. A cette solution, on ajoute ensuite lentement 0,41 mL (0,59 mmole) d'une solution 2M de triméthylaluminium dans le toluène. Le mélange réactionnel obtenu est maintenu sous atmosphère d'azote et agité en laissant la température progressivement atteindre 70˚C. A cette température, on ajoute, goutte à goutte en 5 minutes, une solution de 0,2 g (0,55 mmole) de 1-(3-fluorobenzyl)-5-trifluorométhyl-pyrrolo[3,2-*b*]pyridine-2-carboxylate d'éthyle, obtenu à l'étape 19.4, dans 10 mL de toluène sec. Le mélange réactionnel est alors porté à reflux pendant 18 heures. A la solution refroidie à 0˚C sont ensuite ajoutés 20 mL d'eau froide. Après 90 minutes d'agitation, la solution est extraite trois fois à l'acétate d'éthyle (3x50 mL) et les phases organiques rassemblées sont lavées successivement avec 20 mL d'une solution aqueuse saturée d'hydrogénocarbonate de sodium, 40 mL d'eau et 20 mL de solution aqueuse saturée de chlorure de sodium, séchées sur sulfate de sodium puis concentrées sous pression réduite.
Le solide résultant est trituré dans l'éther isopropylique bouillant pour donner, après séchage, 97 mg du produit attendu sous la forme d'une poudre jaune claire.
Point de fusion : 249 - 251 ˚C
R.M.N. $^1H$ (DMSO $D_6$), δ (ppm) : 10,6 (s, 1H) ; 8,3 (d, 1H) ; 7,91 (s, 1H) ; 7,75 (d, 1H) ; 7,61 (s, 1H) ; 7,49 (m, 2H) ; 7,31 (m, 1H) ; 7,01 (m, 3H) ; 5,95 (s, 2H) ; 3,72 (s, 3H) ; 2,48 (s, 3H).

Les tableaux 1 et 2 qui suivent illustrent les structures chimiques et les propriétés physiques de quelques composés de formule générale (I) selon l'invention. Le tableau 1 illustre des composés de formule générale (I) dans laquelle le noyau pyrrolopyridine est une pyrrolo[2,3-c]pyridine éventuellement substituée. Le tableau 2 illustre des composés de formule générale (I) dans laquelle le noyau pyrrolopyridine est une pyrrolo[2,3-b]pyridine éventuellement substituée. Le tableau 3 illustre des composés de formule générale (I) dans laquelle le noyau pyrrolopyridine est une pyrrolo[3,2-c]pyridine éventuellement substituée. Le tableau 4 illustre des composés de formule générale (I) dans laquelle le noyau pyrrolopyridine est une pyrrolo[3,2-b]pyridine éventuellement substituée.

Dans ces tableaux :

- la colonne « PF » renseigne les points de fusion des produits en degrés Celsius (˚C) ;
- dans la colonne « Sel/base », « - » représente un composé sous forme de base libre, alors que « HCl » représente un composé sous forme de chlorhydrate et le rapport entre parenthèses est le rapport (acide:base) ;
- Ph représente un groupe phényle.

**Tableau 1**

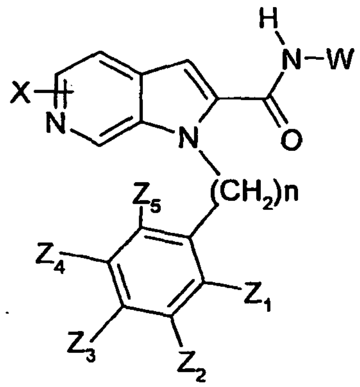

(I)

| N° | X | n | $Z_1, Z_2, Z_3, Z_4, Z_5$ | W | Sel | PF (°C) |
|---|---|---|---|---|---|---|
| 1 | H | 1 | H, F, H, H, H | 1-méthyl-indol-5-yle | - | 213 - 214,5 |
| 2 | 5-F | 1 | H, F, H, H, H | 1-méthyl-benzimidazol-5-yle | - | 279- 281 |
| 3 | 5-Cl | 1 | H, F, H, H, H | 1,2-diméthyl-benzimidazol-5-yle | - | 240- 242 |
| 4 | H | 1 | H, F, H, H, H | 1,2-diméthyl-benzimidazol-5-yle | HCl (2:1) | 309- 310 |
| 5 | 5-F | 1 | H, F, H, H, H | 2-méthyl-benzothiazol-5-yle | - | 193 - 195 |
| 6 | 5-F | 1 | H, F, H, H, H | 1,2-diméthyl-benzimidazol-5-yle | - | 245- 247 |
| 7 | 5-Ph | 1 | H, F, H, H, H | 1,2-diméthyl-benzimidazol-5-yle | - | 288 - 290 |

**Tableau 2**

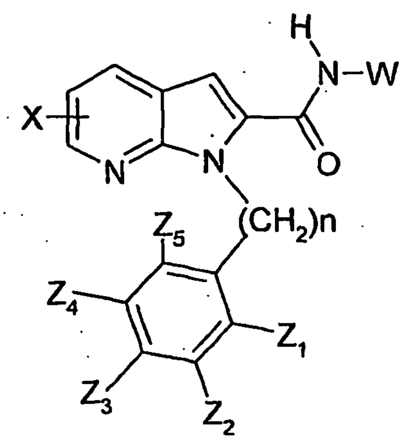

(I)

| N° | X | n | $Z_1, Z_2, Z_3, Z_4, Z_6$ | W | Sel | PF (°C) |
|---|---|---|---|---|---|---|
| 8 | H | 1 | H, F, H, H, H | 1-méthyl-indol-5-yle | - | 191 - 191,5 |
| 9 | H | 1 | H, F, H, H, H | 1-méthyl-benzimidazol-5-yle | HCl (3:2) | 171 - 177 |
| 10 | H | 1 | H, F, H, H, H | 2-oxo-1,2,3,4-tétrahydroquinoléin-7-yle | - | 280 - 282 |
| 11 | H | 1 | H, F, H, H, H | quinoléin-7-yle | HCl (1:1) | 260 - 262 |
| 12 | H | 1 | H, F, H, H, H | 1-méthyl-indol-5-yle | - | 181 - 182 |
| 13 | H | 2 | H, H, H, H, H | 1-méthyl-indol-5-yle | - | 196 - 199 |

(suite)

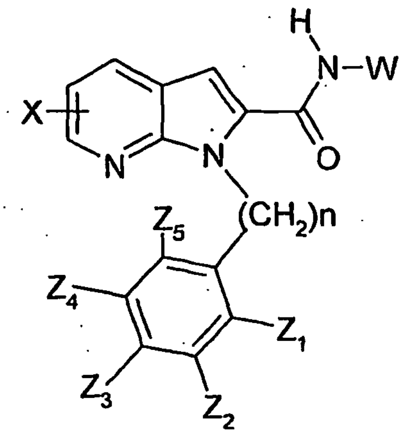

(I)

| N° | X | n | $Z_1, Z_2, Z_3, Z_4, Z_6$ | W | Sel | PF (°C) |
|---|---|---|---|---|---|---|
| 14 | H | 1 | H, F, H, H, H | 2-méthyl-benzothiazol-5-yle | HCl (3:2) | 211 - 212 |
| 15 | 5-F | 1 | H, F, H, H, H | 1-méthyl-benzimidazol-5-yle | - | 266 - 268 |
| 16 | 5-$CF_3$ | 1 | H, F, H, H, H | 1,2-diméthyl-benzimidazol-5-yle | - | 263- 265 |
| 17 | 5-$CF_3$ | 1 | H, F, H, H, H | 1-méthyl-indol-5-yle | - | 229- 231 |
| 18 | 5-$CF_3$ | 1 | H, F, H, H, H | 2-méthyl-benzothiazol-5-yle | - | 204 - 206 |
| 19 | H | 1 | H, F, H, H, H | 1,2-diméthyl-benzimidazol-5-yle | - | 242 - 244 |
| 20 | 5-F | 1 | H, F, H, H, H | 1,2-diméthyl-benzimidazol-5-yle | - | 226 - 228 |
| 21 | H | 0 | H, H, H, H, H | 1,2-diméthyl-benzimidazol-5-yle | - | 286 - 288 |

**Tableau 3**

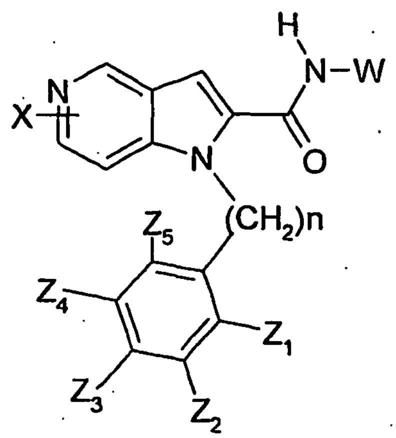

(I)

| N° | X | n | $Z_1, Z_2, Z_3, Z_4, Z_5$ | W | Sel | PF (°C) |
|---|---|---|---|---|---|---|
| 22 | H | 1 | H, F, H, H, H | 1,2-diméthyl-benzimidazol-5-yle | - | 263 - 264 |

**Tableau 4**

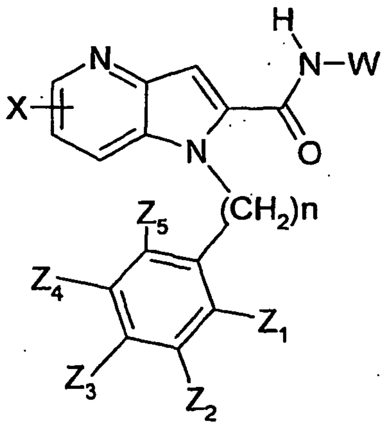

(I)

| N° | X | n | $Z_1, Z_2, Z_3, Z_4, Z_5$ | W | Sel | PF (°C) |
|---|---|---|---|---|---|---|
| 23 | H | 1 | H, F, H, H, H | 1-méthyl-benzimidazol-5-yle | - | 277 - 281 |
| 24 | 5-$CF_3$ | 1 | H, F, H, H, H | 1,2-diméthyl-benzimidazol-5-yle | - | 249 - 251 |
| 25 | H | 1 | H, F, H, H, H | 2-méthyl-benzothiazol-5-yle | - | 263 - 265 |
| 26 | H | 1 | H, F, H, H, H | 1,2-diméthyl-benzimidazol-5-yle | - | 245- 247 |

Les composés de l'invention ont été soumis à des essais pharmacologiques *in vitro* et *in vivo* qui ont mis en évidence leur intérêt comme substances à activités thérapeutiques.

Test d'inhibition du courant induit par la capsaïcine sur les DRG de rat

**[0093]** - Culture primaire de cellules de ganglions de racine dorsale (DRG) de rat :
Les neurones du DRG expriment naturellement le récepteur TRPV1.
Les cultures primaires de DRG de rats nouveaux nés sont préparées à partir de ratons de 1 jour. Brièvement, après dissection, les ganglions sont trypsinés et les cellules dissociées mécaniquement par trituration ménagée. Les cellules sont re-suspendues dans un milieu de culture basal Eagle contenant 10 % de sérum de veau foetal, 25 mM KCl, 2 mM glutamine, 100 μg/mL gentamicine et 50 ng/mL de NGF, puis déposées sur des lamelles de verre recouvertes de laminine (0.25 x 106 cellules par lamelle) qui sont ensuite placées dans des boîtes 12 puits Corning. Les cellules sont incubées à 37°C en atmosphère humidifié contenant 5% de $CO_2$ et 95% d'air. De la cytosine β-D-arabinoside (1 μM) est ajoutée 48 h après la mise en culture, pour prévenir le développement des cellules non neuronales. Les lamelles sont transférées dans les chambres expérimentales pour les études de patch-clamp après 7-10 jours de culture.

**[0094]** - Electrophysiologie :
Les chambres de mesure (volume 800 μl) contenant la préparation cellulaire sont placées sur la platine d'un microscope inversé (Olympus IMT2) équipé d'optiques Hoffman (Modulation Contrast, New York) et observées au grossissement de 400X. Les chambres sont continuellement perfusées par gravité (2,5 mL/min) à l'aide d'un distributeur de solutions acceptant 8 entrées et dont la sortie unique, constituée par un tube de polyéthylène (ouverture 500μm) est placée à moins de 3 mm de la cellule étudiée. La configuration "cellule entière" de la technique de patch-clamp à été utilisée. Les pipettes en verre borosilicaté (résistance 5-10 MOhms) sont approchées de la cellule grâce à un micromanipulateur piézoélectrique 3D ( Burleigh, PC1000) . Les courants globaux (potentiel de membrane fixé à -60 mV) sont enregistrés avec un amplificateur Axopatch 1 D (Axon Instruments, Foster city, Californie), connecté à un PC piloté par les logiciels de Pclamp8 (Axon Instrument). Les traces de courant sont enregistrées sur papier et simultanément digitalisées (fréquence d'échantillonnage 15 à 25 Hz) et acquises sur le disque dur du PC.
L'application d'une solution de capsaïcine 300 nM, provoque sur les cellules de DRG (voltage fixé à -70 mV) un courant cationique entrant. Afin de minimiser la désensibilisation des récepteurs, l'intervalle d'une minute minimum entre deux applications de capsaïcine est respecté. Après une période contrôle (stabilisation de la réponse capsaïcine seule), les

composés à tester sont appliqués seuls à une concentration donnée (concentration de 10 nM ou de 1 nM) pendant une durée de 4 à 5 minutes, au cours desquelles plusieurs tests capsaïcine + composé sont réalisés (obtention de l'inhibition maximale). Les résultats sont exprimés en % d'inhibition de la réponse capsaïcine contrôle.
Les pourcentages d'inhibition de la réponse capsaïcine (300 nM) sont compris entre 20% et 100% pour les composés les plus actifs de l'invention testés à des concentrations de 0,1 à 10 nM.Les composés de l'invention sont donc des antagonistes efficaces in vitro des récepteurs de type TRPV1.

Test d'irritation cornéenne souris

Le caractère irritant de la capsaïcine est aisément apprécié au niveau de la cornée puisque cet organe est un des plus innervés par les fibres C. Dans ce contexte, d'après des expériences préliminaires, l'application d'une très faible quantité de capsaïcine (2 μl à une concentration de 160 μM) à la surface de la cornée d'un animal entraîne un certain nombre de comportements stéréotypés liés à l'irritation et qu'il est facile de répertorier. Parmi ceux-ci, on note : clignement de l'oeil, frottement de l'oeil instillé par la patte avant ipsilatérale, frottement de la face avec les deux pattes avant, grattement de la face ipsilatérale par la patte arrière. La durée de ces comportements ne dépasse pas les 2 minutes d'observation, et l'animal reprend alors son activité normale. Son aspect est par ailleurs également normal. La souris n'est pas recluse dans un coin avec les poils hérissés et ne développe aucun signe observable de souffrance. On peut en conclure que la durée d'action de la capsaïcine à ces doses est inférieure à 2 minutes.

Résumé de la méthodologie :
Le principe de la série d'expériences est de déterminer si les composés de l'invention peuvent influencer la réponse comportementale induite par une quantité donnée de capsaïcine. La capsaïcine est initialement diluée à 25 mM dans le DMSO et diluée, pour son utilisation finale, dans du Tween 80 à 10% dans le sérum physiologique. Il apparaît, à partir d'études contrôles que dans ces conditions, le solvant n'a aucun effet.
En pratique, le produit à tester est administré par voie orale, et, avec un délai (temps de prétraitement : t) qui dépend des données de pharmacocinétique, l'animal reçoit l'instillation oculaire de 2 μl d'une solution de capsaïcine à 160 μM préparée comme indiqué ci-dessus. Au cours d'une observation de 2 minutes suivant l'instillation, le nombre de frottements de l'oeil instillé par la patte antérieure ipsilatéral est répertorié.
Pour un animal donné, le pourcentage de protection est calculé comme suit :

**P= 100 – ((nombre de grattages observés / nombre moyen de grattages du groupe traité par le solvant) x 100)**

Ce pourcentage de protection est moyenné pour chaque groupe d'animaux (n = nombre d'animaux testés avec le composé de l'invention).

Les pourcentages de protection évalués, dans ce modèle, pour les composés de l'invention les plus actifs, utilisés à des doses de 1 à 10 mg/kg (po), sont compris entre 20% et 100% (voir exemple dans le tableau 5).

**Tableau 5**

| n°composé | % P - (t) à 1 mg/kg (*po*) - (n = 10) |
|---|---|
| 15 | 50 % - (1h) |

Les résultats de ces essais montrent que les composés les plus actifs de l'invention bloquent les effets induits par la stimulation des récepteurs TRPV1.

Les composés de l'invention peuvent donc être utilisés pour la préparation de médicaments, notamment pour la préparation d'un médicament destiné à prévenir ou à traiter les pathologies dans lesquelles les récepteurs de type TRPV1 sont impliqués.

Ainsi, selon un autre de ses aspects, l'invention a pour objet des médicaments qui comprennent un composé de formule (I), ou un sel pharmaceutiquement acceptable, ou encore un hydrate ou un solvat dudit composé.

Ces médicaments trouvent leur emploi en thérapeutique, notamment dans la prévention et/ou le traitement de la douleur et de l'inflammation, de la douleur chronique, neuropathique (traumatique, diabétique, métabolique, infectieuse, toxique, induite par un traitement anticancéreux ou hiatrogène), (ostéo-) arthritique, rhumatismale, des fibromyalgies, de la douleur du dos, de la douleur liée au cancer, de la névralgie faciale, des céphalées, de la migraine, de la douleur dentaire, de la brûlure, du coup de soleil, de la morsure ou de la piqûre, de la nevralgie post-herpétique, de la douleur musculaire, de la compression nerveuse (centrale et/ou périphérique), des traumatismes de la moelle et/ou

du cerveau, de l'ischémie (de la moelle et/ou du cerveau), de la neurodégénération, des accidents vasculaires hémorragiques (de la moelle et/ou du cerveau), de la douleur post-stroke.

Les composés de l'invention peuvent être utilisés pour la préparation d'un médicament destiné à prévenir et/ou à traiter les désordres urologiques tels que l'hyperactivité de la vessie, l'hyperéflexie vésicale, l'instabilité vésicale, l'incontinence, la miction d'urgence, incontinence urinaire, la cystite, la colique néphrétique, l'hypersensibilité pelvienne et la douleur pelvienne.

Les composés de l'invention peuvent être utilisés pour la préparation d'un médicament destiné à prévenir et/ou à traiter les désordres gynécologiques comme la vulvodynie, les douleurs liées aux salpingites, aux dysménorrhées.

On peut également utiliser ces produits pour la préparation d'un médicament destiné à prévenir et/ou à traiter les désordres gastrointestinaux tels que le désordre du réflexe gastroesophagique, l'ulcère de l'estomac, l'ulcère du duodénum, la dyspepsie fonctionnelle, la colite, l'IBS, la maladie de Crohn, la pancréatite, l'oesophagite, la colique hépatique.

Les composés de l'invention peuvent également être utilisés pour la préparation d'un médicament destiné à traiter le diabète.

De même, les produits de la présente invention peuvent être utiles dans la prévention et/ou le traitement des désordres respiratoires tels que l'asthme, la toux, la COPD, la bronchoconstriction et les désordres inflammatoires. Ces produits peuvent également être utilisés pour prévenir et/ou traiter le psoriasis, le pruritis, les irritations dermiques, des yeux ou des muqueuses, l'herpès, le zona.

Les composés de l'invention peuvent également être utilisés pour la préparation d'un médicament destiné à traiter la dépression.

Selon un autre de ses aspects, la présente invention concerne des compositions pharmaceutiques comprenant, en tant que principe actif, un composé selon l'invention. Ces compositions pharmaceutiques contiennent une dose efficace d'au moins un composé selon l'invention, ou un sel pharmaceutiquement acceptable, un hydrate ou solvat dudit composé, ainsi qu'au moins un excipient pharmaceutiquement acceptable.

Lesdits excipients sont choisis selon la forme pharmaceutique et le mode d'administration souhaité, parmi les excipients habituels qui sont connus de l'homme du métier.

Dans les compositions pharmaceutiques de la présente invention pour l'administration orale, sublinguale, sous-cutanée, intramusculaire, intra-veineuse, topique, locale, intratrachéale, intranasale, transdermique ou rectale, le principe actif de formule (I) ci-dessus, ou son sel, solvat ou hydrate éventuel, peut être administré sous forme unitaire d'administration, en mélange avec des excipients pharmaceutiques classiques, aux animaux et aux êtres humains pour la prophylaxie ou le traitement des troubles ou des maladies citées ci-dessus.

Les formes unitaires d'administration appropriées comprennent les formes par voie orale telles que les comprimés, les gélules molles ou dures, les poudres, les granules et les solutions ou suspensions orales, les formes d'administration sublinguale, buccale, intratrachéale, intraoculaire, intranasale, par inhalation, les formes d'administration topique, transdermique, sous-cutanée, intramusculaire ou intraveineuse, les formes d'administration rectale et les implants. Pour l'application topique, on peut utiliser les composés selon l'invention dans des crèmes, gels, pommades ou lotions.

A titre d'exemple, une forme unitaire d'administration d'un composé selon l'invention sous forme de comprimé peut comprendre les composants suivants :

| | |
|---|---|
| Composé selon l'invention | 50,0 mg |
| Mannitol | 223,75 mg |
| Croscaramellose sodique | 6,0 mg |
| Amidon de maïs | 15,0 mg |
| Hydroxypropyl-méthylcellulose | 2,25 mg |
| Stéarate de magnésium | 3,0 mg |

Lesdites formes unitaires sont dosées pour permettre une administration journalière de 0,001 à 30 mg de principe actif par kg de poids corporel, selon la forme galénique.

Il peut y avoir des cas particuliers où des dosages plus élevés ou plus faibles sont appropriés ; de tels dosages ne sortent pas du cadre de l'invention. Selon la pratique habituelle, le dosage approprié à chaque patient est déterminé par le médecin selon le mode d'administration, le poids et la réponse dudit patient.

## Revendications

**1.** Composé répondant à la formule (I)

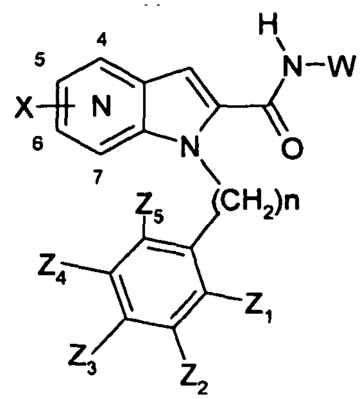

(I)

dans laquelle

n est égal à 0, 1, 2 ou 3 ;

le noyau pyrrolopyridine est un groupe pyrrolo[3,2-*b*]pyridine, un groupe pyrrolo[3,2-*c*]pyridine, un groupe pyrrolo [2,3-*c*]pyridine ou un groupe pyrrolo[2,3-*b*]pyridine ;

le noyau pyrrolopyridine étant éventuellement substitué en position carbonée 4, 5, 6 et /ou 7 par un ou plusieurs substituents X, identiques ou différents l'un de l'autre, choisis parmi un atome d'halogène ou un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, $C_1$-$C_6$-alcoxyle, $C_1$-$C_6$-fluoroalcoxyle, cyano, $C(O)NR_1R_2$, nitro, $NR_1R_2$, $C_1$-$C_6$-thioalkyle, -S(O)-$C_1$-$C_6$-alkyle, -$S(O)_2$-$C_1$-$C_6$-alkyle, $SO_2NR_1R_2$, $NR_3COR_4$, $NR_3SO_2R_5$ ou aryle, l'aryle étant éventuellement substitué par un ou plusieurs substitutants choisi parmi un halogène, un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, $C_1$-$C_6$-alcoxyle, $C_1$-$C_6$-fluoroalcoxyle, nitro ou cyano ;

$Z_1$, $Z_2$, $Z_3$, $Z_4$ et $Z_5$ représentent, indépendamment l'un de l'autre, un atome d'hydrogène ou d'halogène ou un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, $C_1$-$C_6$-alcoxyle, $C_1$-$C_6$-fluoroalcoxyle, cyano, $C(O)NR_1R_2$, nitro, $NR_1R_2$, $C_1$-$C_6$-thioalkyle, -S(O)-$C_1$-$C_6$-alkyle, -$S(O)_2$-$C_1$-$C_6$-alkyle, $SO_2NR_1R_2$, $NR_3COR_4$, $NR_3SO_2R_5$, aryle-$C_1$-$C_6$-alkylène ou aryle, l'aryle et l'aryle-$C_1$-$C_6$-alkylène étant éventuellement substitué par un ou plusieurs substitutants choisis parmi un halogène, un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, $C_1$-$C_6$-alcoxyle, $C_1$-$C_6$-fluoroalcoxyle, nitro ou cyano ;

$R_1$ et $R_2$, représentent, indépendamment l'un de l'autre, un atome d'hydrogène ou un groupe $C_1$-$C_6$-alkyle, $C_3$-$C_7$-cycloalkyle, $C_3$-$C_7$-cycloalkyle-$C_1$-$C_3$-alkylène, aryle-$C_1$-$C_6$-alkylène ou aryle ; ou $R_1$ et $R_2$ formant ensemble, avec l'atome d'azote qui les porte, un groupe azétidine, pyrrolidine, pipéridine, azépine, morpholine, thiomorpholine, pipérazine, homopipérazine, ce groupe étant éventuellement substitué par un groupe $C_1$-$C_6$-alkyle, $C_3$-$C_7$-cycloalkyle, $C_3$-$C_7$-cydoalkyle-$C_1$-$C_3$-alkylène, aryle-$C_1$-$C_6$-alkylène ou aryle ;

$R_3$ et $R_4$ représentent, indépendamment l'un de l'autre, un atome d'hydrogène ou un groupe $C_1$-$C_6$-alkyle, aryle-$C_1$-$C_6$-alkylène ou aryle ;

$R_5$ représente un groupe $C_1$-$C_6$-alkyle ou aryle ;

W représente un groupe bicyclique fusionné de formule :

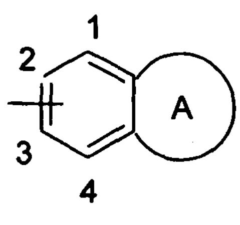

lié à l'atome d'azote par les positions 1, 2, 3 ou 4 ;

A représente un hétérocycle de 5 à 7 chaînons comprenant de un à trois hétéroatomes choisi parmi O, S ou N ;

le ou les atomes de carbones de A étant éventuellement substitués par un ou plusieurs groupes choisis parmi un atome d'hydrogène ou un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, aryle, aryle-$C_1$-$C_6$-alkylène, oxo ou thio;

le ou les atomes d'azote de A étant éventuellement substitués par $R_6$ lorsque l'azote est adjacent à un atome de carbone substitué par un groupe oxo, ou par $R_7$ dans les autres cas ;

$R_6$ représente un atome d'hydrogène ou un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, aryle-$C_1$-$C_6$-alkylène ou aryle ; $R_7$ représente un atome d'hydrogène ou un groupe $C_1$-$C_6$-alkyle, $C_3$-$C_7$-cycloalkyle, $C_3$-$C_7$-cycloalkyle-$C_1$-$C_3$-alkylène, $C_1$-$C_6$-fluoroalkyle, aryle-$C_1$-$C_6$-alkylène, $C_1$-$C_6$-alkyle-C(O)-, $C_3$-$C_7$-cycloalkyle-$C_1$-$C_3$-alkylène-(CO)-, $C_1$-$C_6$-fluoroalkyle-C(O)-, $C_3$-$C_7$-cycloalkyle-C(O)-, aryle-C(O)-, aryle-$C_1$-$C_6$-alkylène-C(O)-, $C_1$-$C_6$-alkyle-$S(O)_2$-, $C_1$-$C_6$-fluoroalkyle-$S(O)_2$-, $C_3$-$C_7$-cycloalkyle-$S(O)_2$-, $C_3$-$C_7$-cycloalkyle-$C_1$-$C_3$-alkylène-$S(O)_2$-, aryle-$S(O)_2$- ou aryle-$C_1$-$C_6$-alkylène-$S(O)_2$- ou aryle ;
le ou les atomes de soufre de l'hétérocycle A pouvant être sous forme oxydée ;
le ou les atomes d'azote de l'hétérocycle A pouvant être sous forme oxydée ;
l'atome d'azote en position 4, 5, 6 ou 7 de la pyrrolopyridine peut être sous forme oxydée ;
à l'état de base ou de sel d'addition à un acide, ainsi qu'à l'état d'hydrate ou de solvat.

**2.** Composé de formule (I) selon la revendication 1, **caractérisé en ce que** n est égal à 1 ou 2 ; à l'état de base ou de sel d'addition à un acide, ainsi qu'à l'état d'hydrate ou de solvat.

**3.** Composé de formule (I) selon la revendication 1 ou 2, **caractérisé en ce que** le noyau pyrrolopyridine est un groupe pyrrolo[3,2-*b*]pyridine, un groupe pyrrolo[3,2-*c*]pyridine, un groupe pyrrolo[2,3-*c*]pyridine ou un groupe pyrrolo[2,3-*b*] pyridine ;
le noyau pyrrolopyridine étant éventuellement substitué en position carbonée 4, 5, 6 et /ou 7 par un ou plusieurs substituants X, identiques ou différents l'un de l'autre, choisis parmi un atome d'hydrogène ou d'halogène, ou un groupe $C_1$-$C_6$-alkyle, $C_3$-$C_7$-cycloalkyle, $C_1$-$C_6$-fluoroalkyle, $C_1$-$C_6$-alcoxyle, $C_1$-$C_6$-fluoroalcoxyle, nitro, $NR_1R_2$, $C_1$-$C_6$-thioalkyle, -S(O)-$C_1$-$C_6$-alkyle, -$S(O)_2$-$C_1$-$C_6$-alkyle, ou aryle ; $R_1$ et $R_2$ représentent, indépendamment l'un de l'autre, un atome d'hydrogène ; à l'état de base ou de sel d'addition à un acide, ainsi qu'à l'état d'hydrate ou de solvat.

**4.** Composé de formule (I) selon l'une quelconque des revendications 1 ou 2, **caractérisé en ce que** le noyau pyrrolopyridine est un groupe pyrrolo[3,2-*b*]pyridine, un groupe pyrrolo[3,2-*c*]pyridine, un groupe pyrrolo[2,3-*c*]pyridine ou un groupe pyrrolo[2,3-*b*]pyridine ;
le noyau pyrrolopyridine étant éventuellement substitué en position carbonée 4, 5, 6 et /ou 7 par un ou plusieurs substituants X, identiques ou différents l'un de l'autre, choisis parmi un atome d'halogène, ou un groupe $C_1$-$C_6$-fluoroalkyle ou aryle ; à l'état de base ou de sel d'addition à un acide, ainsi qu'à l'état d'hydrate ou de solvat.

**5.** Composé de formule (I) selon l'une quelconque des revendications 1 à 4, **caractérisé en ce que** $Z_1$, $Z_2$, $Z_3$, $Z_4$ et $Z_5$ représentent, indépendamment l'un de l'autre, un atome d'hydrogène ou d'halogène ; à l'état de base ou de sel d'addition à un acide, ainsi qu'à l'état d'hydrate ou de solvat.

**6.** Composé de formule (I) selon l'une quelconque des revendications 1 à 5, **caractérisé en ce que** W est choisi parmi les groupes indolinyle, indolyle, isoindolyle, isoindolinyle, benzofuranyle, dihydrobenzofuranyle, benzothiophényle, dihydrobenzothiophényle, benzoxazolyle, dihydrobenzoxazolinyle, isobenzofuranyle, dihydroisobenzofuranyle, benzimidazolyle, dihydrobenzimidazolyle, indazolyle, benzothiazolyle, isobenzothiazolyle, dihydroisobenzothiazolyle, benzotriazolyle, quinoléinyle, dihydroquinoléinyle, tétrahydroquinoléinyle, isoquinoléinyle, dihydroisoquinoléinyle, tétrahydroisoquinoléinyle, benzoxazinyle, dihydrobenzoxazinyle, benzothiazinyle, dihydrobenzothiazinyle, cinnolinyle, quinazolinyle, dihydroquinazolinyle, tétrahydroquinazolinyle, quinoxalinyle, dihydroquinoxalinyle, tétrahydroquinoxalinyle, phtalazinyle, dihydrophtalazinyle, tétrahydrophtalazinyle, tétrahydrobenz[*b*]azépinyle, tétrahydrobenz[*c*]azépinyle, tétrahydrobenz[*d*]azépinyle, tétrahydrobenzo[*b*][1,4]diazépinyle, tétrahydrobenzo[*e*][1,4]diazépinyle, tétrahydrobenzo[*b*][1,4]oxazépinyle ou tétrahydrobenzo[*b*][1,4]thiazépinyle ;
le ou les atomes de carbone et/ou d'azote dudit groupe W étant éventuellement substitués comme défini dans la formule générale (I) selon la revendication 1 ; à l'état de base ou de sel d'addition à un acide, ainsi qu'à l'état d'hydrate ou de solvat.

**7.** Composé de formule (I) selon l'une quelconque des revendications 1 à 5, **caractérisé en ce que** W représente un groupe bicyclique fusionné de formule :

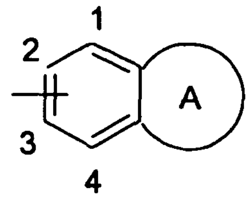

lié à l'atome d'azote par les positions 1, 2, 3 ou 4 ;
A représente un hétérocycle de 5 à 7 chaînons comprenant de un à trois hétéroatomes choisi parmi O, S ou N ;
et W est choisi parmi les groupes indolyle, benzimidazolyle, tétrahydroquinoléinyle, quinoléinyle, benzothiazolyle ; et/ou
le ou les atomes de carbones de A étant éventuellement substitués par un ou plusieurs groupes choisis parmi un atome d'hydrogène ou un groupe $C_1$-$C_6$-alkyle ou un groupe oxo ; et/ou
le ou les atomes d'azote de A étant éventuellement substitués par $R_6$ lorsque l'azote est adjacent à un atome de carbone substitué par un groupe oxo, ou par $R_7$ dans les autres cas ;
$R_6$ représente un atome d'hydrogène ;
$R_7$ représente un atome d'hydrogène ou un groupe $C_1$-$C_6$-alkyle ; à l'état de base ou de sel d'addition à un acide, ainsi qu'à l'état d'hydrate ou de solvat.

**8.** Composé de formule (I) selon l'une quelconque des revendications 1 à 7 choisi parmi:

- *N*-(1-méthyl-1*H*-indol-5-yl)-1-[(3-fluorophényl)méthyl]-1*H*-pyrrolo[2,3-*c*]pyridine-2-carboxamide ;
- *N*-(1-méthyl-1*H*-benzimidazol-5-yl)-5-fluoro-1-[(3-fluorophényl)méthyl]-1*H*-pyrrolo[2,3-*c*]pyridine-2-carboxamide ;
- *N*-(1,2-diméthyl-1*H*-benzimidazol-5-yl)-5-chloro-1-[(3-fluorophényl)méthyl]-1*H*-pyrrolo[2,3-*c*]pyridine-2-carboxamide ;
- *N*-(1,2-diméthyl-1*H*-benzimidazol-5-yl)-1-[(3-fluorophényl)méthyl]-1*H*-pyrrolo[2,3-*c*]pyridine-2-carboxamide ;
- *N*-(2-méthyl-1*H*-benzothiazol-5-yl)-5-fluoro-1-[(3-fluorophényl)méthyl]-1*H*-pyrrolo[2,3-*c*]pyridine-2-carboxamide ;
- *N*-(1,2-diméthyl-1*H*-benzimidazol-5-yl)-5-fluoro-1-[(3-fluorophényl)méthyl]-1*H*-pyrrolo[2,3-*c*]pyridine-2-carboxamide ;
- *N*-(1,2-diméthyl-1*H*-benzimidazol-5-yl)-5-phényl-1-[(3-fluorophényl)méthyl]-1*H*-pyrrolo[2,3-*c*]pyridine-2-carboxamide ;
- *N*-(1-méthyl-1*H*-indol-5-yl)-1-[(3-fluorophényl)méthyl]-1*H*-pyrrolo[2,3-*b*]pyridine-2-carboxamide ;
- *N*-(1-méthyl-1*H*-benzimidazol-5-yl)-1-[(3-fluorophényl)méthyl]-1*H*-pyrrolo[2,3-*b*]pyridine-2-carboxamide ;
- *N*-(2-oxo-1,2,3,4-tétrahydroquinoléin-7-yl)-1-[(3-fluorophényl)méthyl]-1*H*-pyrrolo[2,3-*b*]pyridine-2-carboxamide ;
- *N*-(quinoléin-7-yl)-1-[(3-fluorophényl)méthyl]-1*H*-pyrrolo[2,3-*b*]pyridine-2-carboxamide;
- *N*-(1-méthyl-1*H*-indol-5-yl)-1-(phénylméthyl)-1*H*-pyrrolo[2,3-*b*]pyridine-2-carboxamide ;
- *N*-(1-méthyl-1*H*-indol-5-yl)-1-(phényléthyl)-1*H*-pyrrolo[2,3-*b*]pyridine-2-carboxamide ;
- *N*-(2-méthyl-benzothiazol-5-yl)-1-[(3-fluorophényl)méthyl]-1*H*-pyrrolo[2,3-*b*]pyridine-2-carboxamide ;
- *N*-(1-méthyl-1*H*-benzimidazol-5-yl)-5-fluoro-1-[(3-fluorophényl)méthyl]-1*H*-pyrrolo[2,3-*b*]pyridine-2-carboxamide ;
- *N*-(1,2-diméthyl-1*H*-benzimidazol-5-yl)-5-trifluorométhyl-1-[(3-fluorophényl)méthyl]-1*H*-pyrrolo[2,3-*b*]pyridine-2-carboxamide ;
- *N*-(1-méthyl-1*H*-indol-5-yl)-5-trifluorométhyl-1-[(3-fluorophényl)méthyl]-1*H*-pyrrolo[2,3-*b*]pyridine-2-carboxamide ;
- *N*-(2-méthyl-benzothiazol-5-yl)-5-trifluorométhyl-1-[(3-fluorophényl)méthyl]-1*H*-pyrrolo[2,3-*b*]pyridine-2-carboxamide ;
- *N*-(1,2-diméthyl-1*H*-benzimidazol-5-yl)-1-[(3-fluorophényl)méthyl]-1*H*-pyrrolo[2,3-*b*]pyridine-2-carboxamide ;
- *N*-(1,2-diméthyl-1*H*-benzimidazol-5-yl)-5-fluoro-1-[(3-fluorophényl)méthyl]-1*H*-pyrrolo[2,3-*b*]pyridine-2-carboxamide ;
- *N*-(1,2-diméthyl-1*H*-benzimidazol-5-yl)-1-[(3-fluorophényl)méthyl]-1*H*-pyrrolo[3,2-*c*]pyridine-2-carboxamide ;
- *N*-(1-méthyl-1*H*-benzimidazol-5-yl)-1-[(3-fluorophényl)méthyl]-1*H*-pyrrolo[3,2-*b*]pyridine-2-carboxamide ;
- *N*-(1,2-diméthyl-1*H*-benzimidazol-5-yl)-5-trifluorométhyl-1-[(3-fluorophényl)méthyl]-1*H*-pyrrolo[3,2-*b*]pyridine-2-carboxamide ;
- *N*-(2-méthyl-benzothiazol-5-yl)-1-[(3-fluorophényl)méthyl]-1*H*-pyrrolo[3,2-*b*]pyridine-2-carboxamide ;

- *N*-(1,2-diméthyl-1*H*-benzimidazol-5-yl)-1-[(3-fluorophényl)méthyl]-1*H*-pyrrolo[3,2-*b*]pyridine-2-carboxamide ;
- *N*-(1,2-diméthyl-1*H*-benzimidazol-5-yl)-1-(phényl)-1*H*-pyrrolo[2,3-*b*]pyridine-2-carboxamide.

**9.** Procédé de préparation d'un composé de formule (I) selon l'une quelconque des revendications 1 à 8, **caractérisé en ce que** l'on fait réagir un composé de formule générale (IV)

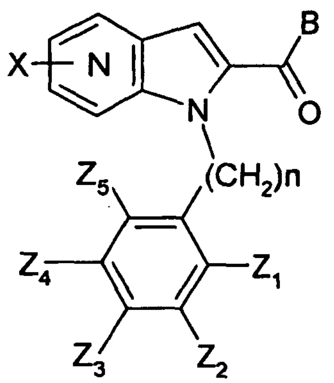

**(IV)**

dans laquelle $X_1$, $X_2$, $X_3$, $X_4$, $Z_1$, $Z_2$, $Z_3$, $Z_4$, $Z_5$ et n sont tels que définis dans la formule générale (I) selon la revendication 1 et B représente un groupe $C_1$-$C_4$-alcoxyle, avec un amidure du composé de formule générale (V)

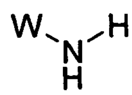

**(V)**

dans laquelle W est tel que défini dans la formule générale (I) selon la revendication 1, au reflux d'un solvant, l'amidure du composé de formule générale (V) étant préparé par action préalable du triméthylaluminium sur les composés de formule générale (V).

**10.** Procédé de préparation d'un composé de formule (I) selon l'une quelconque des revendications 1 à 8, **caractérisé en ce que** l'on transforme un composé de formule générale (IV)

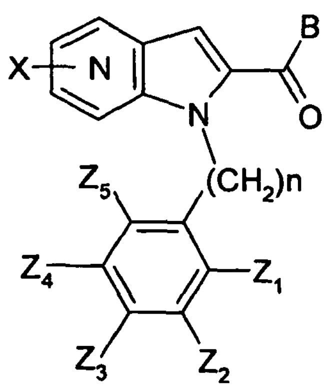

**(IV)**

dans laquelle $X_1$, $X_2$, $X_3$, $X_4$, $Z_1$, $Z_2$, $Z_3$, $Z_4$, $Z_5$ et n sont tels que définis dans la formule générale (I) selon la revendication 1 et B représente un groupe hydroxyle,
en chlorure d'acide par action du chlorure de thionyle au reflux d'un solvant,
puis **en ce que** l'on fait réagir, en présence d'une base, le composé de formule générale (IV) obtenu, dans laquelle $X_1$, $X_2$, $X_3$, $X_4$, $Z_1$, $Z_2$, $2_3$, $Z_4$, $Z_5$ et n sont tels que définis dans la formule générale (I) selon la revendication 1 et B représente un atome de chlore, avec le composé de formule générale (V),

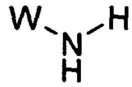

(V)

dans laquelle W est tel que défini dans la formule générale (I) selon la revendication 1,
ou bien **en ce que** l'on effectue une réaction de couplage entre un composé de formule générale (IV) dans laquelle $X_1$, $X_2$, $X_3$, $X_4$, $Z_1$, $Z_2$, $Z_3$, $Z_4$, $Z_5$ et n sont tels que définis dans la formule générale (I) selon la revendication 1 et B représente un groupe hydroxyle, et le composé de formule générale (V), dans laquelle W est tel que défini dans la formule générale (I) selon la revendication 1, en présence d'un agent de couplage et d'une base, dans un solvant.

**11.** Médicament, **caractérisé en ce qu'**il comprend un composé de formule (I), selon l'une quelconque des revendications 1 à 8, ou un sel pharmaceutiquement acceptable, ou encore un hydrate ou un solvat du composé de formule (I).

**12.** Composition pharmaceutique, **caractérisée en ce qu'**elle comprend un composé de formule (I), selon l'une quelconque des revendications 1 à 8, ou un sel pharmaceutiquement acceptable, un hydrate ou un solvat de ce composé, ainsi qu'au moins un excipient pharmaceutiquement acceptable.

**13.** Utilisation d'un composé de formule (I), selon l'une quelconque des revendications 1 à 8, pour la préparation d'un médicament destiné à prévenir ou à traiter les pathologies dans lesquelles les récepteurs de type TRPV1 sont impliqués.

**14.** Utilisation d'un composé de formule (I), selon l'une quelconque des revendications 1 à 8, pour la préparation d'un médicament destiné à prévenir ou à traiter la douleur, l'inflammation, les désordres urologiques, les désordres gynécologiques, les désordres gastrointestinaux, des désordres respiratoires, le psoriasis, le pruritis, les irritations dermiques, des yeux ou des muqueuses, l'herpès, le zona, ou à traiter la dépression ou le diabète.

## Claims

**1.** Compound corresponding to formula (I)

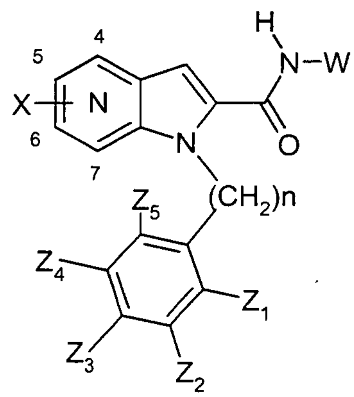

(I)

in which
n is equal to 0, 1, 2 or 3;
the pyrrolopyridine nucleus is a pyrrolo[3,2-*b*]pyridine group, a pyrrolo[3,2-*c*]pyridine group, a pyrrolo[2,3-*c*]pyridine group or a pyrrolo[2,3-*b*]pyridine group;
the pyrrolopyridine nucleus being optionally substituted in the carbon position 4, 5, 6 and/or 7 with one or more substituents X, which may be identical or different, chosen from a halogen atom and a $C_1$-$C_6$-alkyl, $C_3$-$C_7$-cycloalkyl, $C_3$-$C_7$-cycloalkyl-$C_1$-$C_3$-alkylene, $C_1$-$C_6$-fluoroalkyl, $C_1$-$C_6$-alkoxyl, $C_1$-$C_6$-fluoroalkoxyl, cyano, $C(O)NR_1R_2$, nitro, $NR_1R_2$, $C_1$-$C_6$-thioalkyl, -S(O)-$C_1$-$C_6$-alkyl, -$S(O)_2$-$C_1$-$C_6$-alkyl, $SO_2NR_1R_2$, $NR_3COR_4$, $NR_3SO_2R_5$ or aryl group, the aryl being optionally substituted with one or more substituents chosen from a halogen and a $C_1$-$C_6$-alkyl, $C_3$-$C_7$-cycloalkyl, $C_3$-$C_7$-cycloalkyl-$C_1$-$C_3$-alkylene, $C_1$-$C_6$-fluoroalkyl, $C_1$-$C_6$-alkoxyl, $C_1$-$C_6$-fluoroalkoxyl, nitro or cyano group;

$Z_1$, $Z_2$, $Z_3$, $Z_4$ and $Z_5$ represent, independently of each other, a hydrogen or halogen atom or a $C_1$-$C_6$-alkyl, $C_3$-$C_7$-cycloalkyl, $C_3$-$C_7$-cycloalkyl-$C_1$-$C_3$-alkylene, $C_1$-$C_6$-fluoroalkyl, $C_1$-$C_6$-alkoxyl, $C_1$-$C_6$-fluoroalkoxyl, cyano, C(O)$NR_1R_2$, nitro, $NR_1R_2$, $C_1$-$C_6$-thioalkyl, -S(O)-$C_1$-$C_6$-alkyl, -$S(O)_2$-$C_1$-$C_6$-alkyl, $SO_2NR_1R_2$, $NR_3COR_4$, $NR_3SO_2R_5$, aryl-$C_1$-$C_6$-alkylene or aryl group, the aryl and the aryl-$C_1$-$C_6$-alkylene being optionally substituted with one or more substituents chosen from a halogen and a $C_1$-$C_6$-alkyl, $C_3$-$C_7$-cycloalkyl, $C_3$-$C_7$-cycloalkyl-$C_1$-$C_3$-alkylene, $C_1$-$C_6$-fluoroalkyl, $C_1$-$C_6$-alkoxyl, $C_1$-$C_6$-fluoroalkoxyl, nitro or cyano group;

$R_1$ and $R_2$ represent, independently of each other, a hydrogen atom or a $C_1$-$C_6$-alkyl, $C_3$-$C_7$-cycloalkyl, $C_3$-$C_7$-cycloalkyl-$C_1$-$C_3$-alkylene, aryl-$C_1$-$C_6$-alkylene or aryl group; or $R_1$ and $R_2$ together forming, with the nitrogen atom that bears them, an azetidine, pyrrolidine, piperidine, azepine, morpholine, thiomorpholine, piperazine, homopiperazine group, this group being optionally substituted with a $C_1$-$C_6$-alkyl, $C_3$-$C_7$-cycloalkyl, $C_3$-$C_7$-cycloalkyl-$C_1$-$C_3$-alkylene, aryl-$C_1$-$C_6$-alkylene or aryl group;

$R_3$ and $R_4$ represent, independently of each other, a hydrogen atom or a $C_1$-$C_6$-alkyl, aryl-$C_1$-$C_6$-alkylene or aryl group;

$R_5$ represents a $C_1$-$C_6$-alkyl or aryl group;

W represents a fused bicyclic group of formula:

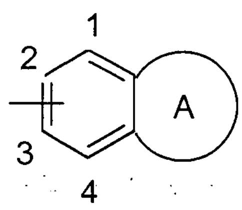

bonded to the nitrogen atom via positions 1, 2, 3 or 4;

A represents a 5- to 7-membered heterocycle comprising from one to three heteroatoms chosen from O, S and N; the carbon atom(s) of A being optionally substituted with one or more groups chosen from a hydrogen atom and a $C_1$-$C_6$-alkyl, $C_3$-$C_7$-cycloalkyl, $C_3$-$C_7$-cycloalkyl-$C_1$-$C_3$-alkylene, $C_1$-$C_6$-fluoroalkyl, aryl, aryl-$C_1$-$C_6$-alkylene, oxo or thio group;

the nitrogen atom(s) of A being optionally substituted with $R_6$ when the nitrogen is adjacent to a carbon atom substituted with an oxo group, or with $R_7$ in the other cases;

$R_6$ represents a hydrogen atom or $C_1$-$C_6$-alkyl, $C_3$-$C_7$-cycloalkyl, $C_3$-$C_7$-cycloalkyl-$C_1$-$C_3$-alkylene, $C_1$-$C_6$-fluoroalkyl, aryl-$C_1$-$C_6$-alkylene or aryl group;

$R_7$ represents a hydrogen atom or a $C_1$-$C_6$-alkyl, $C_3$-$C_7$-cycloalkyl, $C_3$-$C_7$-cycloalkyl-$C_1$-$C_3$-alkylene, $C_1$-$C_6$-fluoroalkyl, aryl-$C_1$-$C_6$-alkylene, $C_1$-$C_6$-alkyl-C(O)-, $C_3$-$C_7$-cycloalkyl-$C_1$-$C_3$-alkylene-(CO)-, $C_1$-$C_6$-fluoroalkyl-C(O)-, $C_3$-$C_7$-cycloalkyl-C(O)-, aryl-C(O)-, aryl-$C_1$-$C_6$-alkylene-C(O)-, $C_1$-$C_6$-alkyl-$S(O)_2$-, $C_1$-$C_6$-fluoroalkyl-$S(O)_2$-, $C_3$-$C_7$-cycloalkyl-$S(O)_2$-, $C_3$-$C_7$-cycloalkyl-$C_1$-$C_3$-alkylene-$S(O)_2$-, aryl-$S(O)_2$- or aryl-$C_1$-$C_6$-alkylene-$S(O)_2$- or aryl group;

the sulfur atom(s) of the heterocycle A possibly being in oxidized form;

the nitrogen atom(s) of the heterocycle A possibly being in oxidized form;

the nitrogen atom in position 4, 5, 6 or 7 of the pyrrolopyridine may be in oxidized form;

in the form of base or of acid-addition salt, and also in the form of hydrate or solvate.

**2.** Compound of formula (I) according to Claim 1,
**characterized in that** n is equal to 1 or 2; in the form of base or of acid-addition salt, and also in the form of hydrate or solvate.

**3.** Compound of formula (I) according to Claim 1 or 2, **characterized in that** the pyrrolopyridine nucleus is a pyrrolo[3,2-*b*]pyridine group, a pyrrolo[3,2-*c*]pyridine group, a pyrrolo[2,3-*c*]pyridine group or a pyrrolo[2,3-*b*]pyridine group; the pyrrolopyridine nucleus being optionally substituted in the carbon position 4, 5, 6 and/or 7 with one or more substituents X, which may be identical or different, chosen from a hydrogen or halogen atom and a $C_1$-$C_6$-alkyl, $C_3$-$C_7$-cycloalkyl, $C_1$-$C_6$-fluoroalkyl, $C_1$-$C_6$-alkoxyl, $C_1$-$C_6$-fluoroalkoxyl, nitro, $NR_1R_2$, $C_1$-$C_6$-thioalkyl, -S(O)-$C_1$-$C_6$-alkyl, -$S(O)_2$-$C_1$-$C_6$-alkyl, or aryl group; $R_1$ and $R_2$ represent, independently of each other, a hydrogen atom; in the form of base or acid-addition salt, and also in the form of hydrate or solvate.

**4.** Compound of formula (I) according to either of Claims 1 and 2, **characterized in that** the pyrrolopyridine nucleus is a pyrrolo[3,2-*b*]pyridine group, a pyrrolo[3,2-*c*]pyridine group, a pyrrolo[2,3-*c*]pyridine group or a pyrrolo[2,3-*b*] pyridine group; the pyrrolopyridine nucleus being optionally substituted in the carbon position 4, 5, 6 and/or 7 with

one or more substituents X, which may be identical or different, chosen from a halogen atom and a $C_1$-$C_6$-fluoroalkyl or aryl group; in the form of base or of acid-addition salt, and also in the form of hydrate or solvate.

5. Compound of formula (I) according to any one of Claims 1 to 4, **characterized in that** $Z_1$, $Z_2$, $Z_3$, $Z_4$ and $Z_5$ represent, independently of each other, a hydrogen or halogen atom; in the form of base or of acid-addition salt, and also in the form of hydrate or solvate.

6. Compound of formula (I) according to any one of Claims 1 to 5, **characterized in that** W is chosen from indolinyl, indolyl, isoindolyl, isoindolinyl, benzofuranyl, dihydrobenzofuran.yl, benzothiophenyl, dihydrobenzothiophenyl, benzoxazolyl, dihydrobenzoxazolinyl, isobenzofuranyl, dihydroisobenzofuranyl, benzimidazolyl, dihydrobenzimidazolyl, indazolyl, benzothiazolyl, isobenzothiazolyl, dihydroisobenzothiazolyl, benzotriazolyl, quinolyl, dihydroquinolyl, tetrahydroquinolyl, isoquinolyl, dihydroisoquinolyl, tetrahydroisoquinolyl, benzoxazinyl, dihydrobenzoxazinyl, benzothiazinyl, dihydrobenzothiazinyl, cinnolinyl, quinazolinyl, dihydroquinazolinyl, tetrahydroquinazolinyl, quinoxalinyl, dihydro-quinoxalinyl, tetrahydroquinoxalinyl, phthalazinyl, dihydrophthalazinyl, tetrahydrophthalazinyl, tetrahydrobenz[*b*]azepinyl, tetrahydrobenz[*c*]azepinyl, tetrahydrobenz[*d*]azepinyl, tetrahydrobenzo[*b*][1,4]-diazepinyl, tetrahydrobenzo[*e*][1,4]diazepinyl, tetrahydrobenzo[*b*][1,4]oxazepinyl or tetrahydrobenzo-[*b*][1,4]thiazepinyl groups; the carbon and/or nitrogen atom(s) of said group W being optionally substituted as defined in the general formula (I) according to Claim 1; in the form of base or of acid-addition salt, and also in the form of hydrate or solvate.

7. Compound of formula (I) according to any one of Claims 1 to 5, **characterized in that** W represents a fused bicyclic group of formula:

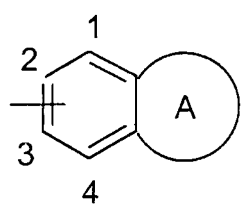

bonded to the nitrogen atom via positions 1, 2, 3 or 4; A represents a 5- to 7-membered heterocycle comprising from one to three heteroatoms chosen from O, S and N; and W is chosen from indolyl, benzimidazolyl, tetrahydroquinolyl, quinolyl and benzothiazolyl groups; and/or
the carbon atom(s) of A being optionally substituted with one or more groups chosen from a hydrogen atom and a $C_1$-$C_6$-alkyl or an oxo group; and/or
the nitrogen atom(s) of A being optionally substituted with $R_6$ when the nitrogen is adjacent to a carbon atom substituted with an oxo group, or with $R_7$ in the other cases;
$R_6$ represents a hydrogen atom;
$R_7$ represents a hydrogen atom or a $C_1$-$C_6$-alkyl group; in the form of base or of acid-addition salt, and also in the form of hydrate or solvate.

8. Compound of formula (I) according to any one of Claims 1 to 7, chosen from:

- *N*-(1-methyl-1*H*-indol-5-yl)-1-[(3-fluorophenyl)methyl]-1*H*-pyrrolo[2,3-*c*]pyridine-2-carboxamide;
- *N*-(1-methyl-1*H*-benzimidazol-5-yl)-5-fluoro-1-[(3-fluorophenyl)methyl]-1*H*-pyrrolo[2,3-*c*]pyridine-2-carboxamide;
- *N*-(1,2-methyl-1*H*-benzimidazol-5-yl)-5-chloro-1-[(3-fluorophenyl)methyl]-1*H*-pyrrolo[2,3-*c*]pyridine-2carboxamide;
- *N*-(1,2-methyl-1*H*-benzimidazol-5-yl)-1-[(3-fluorophenyl)methyl]-1*H*-pyrrolo[2,3-*c*]pyridine-2-carboxamide;
- *N*-(2-methyl-1*H*-benzothiazol-5-yl)-5-fluoro-1-[(3-fluorophenyl)methyl]-1H-pyrrolo[2,3-*c*]pyridine-2-carboxamide;
- *N*-(1,2-dimethyl-1*H*-benzimidazol-5-yl)-5-fluoro-1-[(3-fluorophenyl)methyl]-1*H*-pyrrolo[2,3-*c*]pyridine-2-carboxamide;
- *N*-(1,2-dimethyl-1*H*-benzimidazol-5-yl)-5-phenyl-1-[(3-fluorophenyl)methyl]-1*H*-pyrrolo[2,3-*c*]pyridine-2-carboxamide;
- *N*-(1-methyl-1*H*-indol-5-yl)-1-[(3-fluorophenyl)methyl]-1*H*-pyrrolo[2,3-*b*]pyridine-2-carboxamide;
- *N*-(1-methyl-1*H*-benzimidazol-5-yl)-1-[(3-fluorophenyl)methyl]-1*H*-pyrrolo[2,3-*b*]pyridine-2-carboxamide;
- *N*-(2-oxo-1,2,3,4-tetrahydroquinol-7-yl)-1-[(3-fluorophenyl)methyl]-1*H*-pyrrolo[2,3-*b*]pyridine-2-carboxamide;

- *N*-(quinol-7-yl)-1-[(3-fluorophenyl)methyl]-1*H*-pyrrolo[2,3-*b*]pyridine-2-carboxamide;
- *N*-(1-methyl-1*H*-indol-5-yl)-1-(phenylmethyl)-1*H*-pyrrolo[2,3-*b*]pyridine-2-carboxamide;
- *N*-(1-methyl-1*H*-indol-5-yl)-1-(phenylethyl)-1*H*-pyrrolo[2,3-*b*]pyridine-2.carboxamide;
- *N*-(2-methyl-benzothiazol-5-yl)-1-[(3-fluorophenyl)methyl]-1*H*-pyrrolo[2,3-*b*]pyridine-2-carboxamide;
- *N*-(1-methyl-1*H*-benzimidazol-5-yl)-5-fluoro-1-[(3-fluorophenyl)methyl]-1*H*-pyrrolo[2,3-*b*]pyridine-2-carboxamide;
- *N*-(1,2-dimethyl-1*H*-benzimidazol-5-yl)-5-trifluoromethyl-1-[(3-fluorophenyl)methyl]-1*H*-pyrrolo[2,3-*b*]pyridine-2-carboxamide;
- *N*-(1-methyl-1*H*-indol-5-yl)-5-trifluoromethyl-1-[(3-fluorophenyl)methyl]-1*H*-pyrrolo[2,3-*b*]pyridine-2-carboxamide;
- *N*-(2-methyl-benzothiazol-5-yl)-5-trifluoromethyl-1-[(3-fluorophenyl)methyl]-1*H*-pyrrolo[2,3-*b*]pyridine-2-carboxamide;
- *N*-(1,2-dimethyl-1*H*-benzimidazol-5-yl)-1-[(3-fluorophenyl)methyl]-1*H*-pyrrolo[2,3-*b*]pyridine-2-carboxamide;
- *N*-(1,2-dimethyl-1*H*-benzimidazol-5-yl)-5-fluoro-1-[(3-fluorophenyl)methyl]-1*H*-pyrrolo[2,3-*b*]pyridine-2-carboxamide;
- *N*-(1,2-dimethyl-1*H*-benzimidazol-5-yl)-1-[(3-fluorophenyl)methyl]-1*H*-pyrrolo[3,2-*c*]pyridine-2-carboxamide;
- *N*-(1-methyl-1*H*-benzimidazol-5-yl)-1-[(3-fluorophenyl)methyl]-1*H*-pyrrolo[3,2-*b*]pyridine-2-carboxamide;
- *N*-(1,2-dimethyl-1*H*-benzimidazol-5-yl)-5-trifluoromethyl-1-[(3-fluorophenyl)methyl]-1*H*-pyrrolo[3,2-*b*]pyridine-2-carboxamide;
- *N*-(2-methyl-benzothiazol-5-yl)-1-[(3-fluorophenyl)methyl]-1*H*-pyrrolo[3,2-*b*]pyridine-2-carboxamide;
- *N*-(1,2-dimethyl-1*H*-benzimidazol-5-yl)-1-[(3-fluorophenyl)methyl]-1*H*-pyrrolo[3,2-*b*]pyridine-2-carboxamide;
- *N*-(1,2-dimethyl-1*H*-benzimidazol-5-yl)-1-(phenyl)-1*H*-pyrrolo[2,3-*b*]pyridine-2-carboxamide.

**9.** Process for preparing a compound of formula (I) according to any one of Claims 1 to 8, **characterized in that** a compound of general formula (IV)

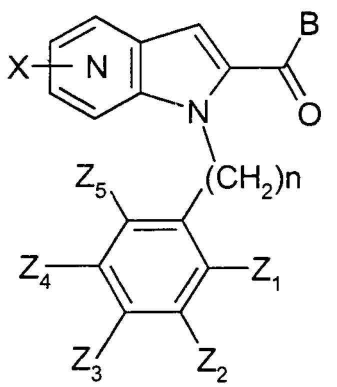

**(IV)**

in which $X_1$, $X_2$, $X_3$, $X_4$, $Z_1$, $Z_2$, $Z_3$, $Z_4$, $Z_5$ and n are as defined in the general formula (I) according to Claim 1 and B represents a $C_1$-$C_4$-alkoxyl group,
is reacted with an amide of the compound of general formula (V)

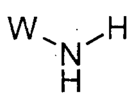

**(V)**

in which W is as defined in general formula (I) according to Claim 1,
at the reflux point of a solvent, the amide of the compound of general formula (V) being prepared by first reacting trimethylaluminium with the compounds of general formula (V).

**10.** Process for preparing a compound of formula (I) according to any one of Claims 1 to 8, **characterized in that** a compound of general formula (IV)

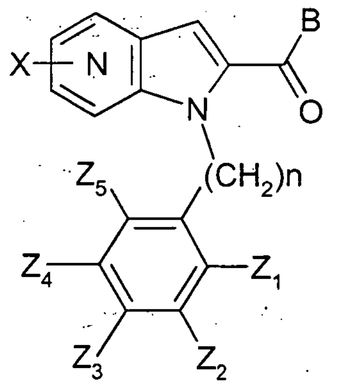

(IV)

in which $X_1$, $X_2$, $X_3$, $X_4$, $Z_1$, $Z_2$, $Z_3$, $Z_4$, $Z_5$ and n are as defined in the general formula (I) according to Claim 1 and B represents a hydroxyl group,
is converted into the acid chloride via the action of thionyl chloride at the reflux point of a solvent,
and the compound of general formula (IV) obtained, in which $X_1$, $X_2$, $X_3$, $X_4$, $Z_1$, $Z_2$, $Z_3$, $Z_4$, $Z_5$ and n are as defined in the general formula (I) according to Claim 1 and B represents a chlorine atom, is then reacted, in the presence of a base, with the compound of general formula (V),

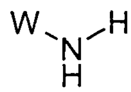

(V)

in which W is as defined in the general formula (I) according to Claim 1,
or a coupling reaction is performed between a compound of general formula (IV), in which $X_1$, $X_2$, $X_3$, $X_4$, $Z_1$, $Z_2$, $Z_3$, $Z_4$, $Z_5$ and n are as defined in the general formula (I) according to Claim 1 and B represents a hydroxyl group, and the compound of general formula (V), in which W is as defined in the general formula (I) according to Claim 1, in the presence of a coupling agent and a base, in a solvent.

**11.** Medicament, **characterized in that** it comprises a compound of formula (I), according to any one of Claims 1 to 8, or a pharmaceutically acceptable salt, or alternatively a hydrate or a solvate of the compound of formula (I).

**12.** Pharmaceutical composition, **characterized in that** it comprises a compound of formula (I), according to any one of Claims 1 to 8, or a pharmaceutically acceptable salt, a hydrate or a solvate of this compound, and also at least one pharmaceutically acceptable excipient.

**13.** Use of a compound of formula (I) according to any one of Claims 1 to 8, for the preparation of a medicament for preventing or treating pathologies in which the TRPV1 receptors are involved.

**14.** Use of a compound of formula (I) according to any one of Claims 1 to 8, for the preparation of a medicament for preventing or treating pain, inflammation, urological disorders, gynaecological disorders, gastrointestinal disorders, respiratory disorders, psoriasis, pruritus, dermal, ocular or mucous irritation, herpes and zona, or for treating depression or diabetes.

**Patentansprüche**

**1.** Verbindung der Formel (I)

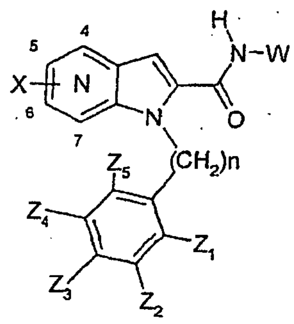

(I)

worin
n gleich 0, 1, 2 oder 3 ist;
der Pyrrolopyridinkern eine Pyrrolo[3,2-*b*]pyridingruppe, eine Pyrrolo[3,2-*c*]pyridingruppe, eine Pyrrolo[2,3-*c*]pyridingruppe oder eine Pyrrolo[2,3-*b*]pyridingruppe ist;
wobei der Pyrrolopyridinkern gegebenenfalls in Kohlenstoffposition 4, 5, 6 und/oder 7 durch einen oder mehrere Substituenten X substituiert ist, die gleich oder voneinander verschieden sind und unter einem Halogenatom oder einer $C_1$-$C_6$-Alkyl-, $C_3$-$C_7$-Cycloalkyl-, $C_3$-$C_7$-Cycloalkyl-$C_1$-$C_3$-alkylen-, $C_1$-$C_6$-Fluoralkyl-, $C_1$-$C_6$-Alkoxy-, $C_1$-$C_6$-Fluoralkoxy-, Cyano-, $C(O)NR_1R_2$-, Nitro-, $NR_1R_2$-, $C_1$-$C_6$-Thioalkyl-, -S(O)-$C_1$-$C_6$-Alkyl-, -$S(O)_2$-$C_1$-$C_6$-Alkyl-, $SO_2NR_1R_2$-, $NR_3COR_4$-, $NR_3SO_2R_5$- oder Arylgruppe ausgewählt sind, wobei das Aryl gegebenenfalls durch einen oder mehrere Substituenten substituiert ist, die unter einem Halogen oder einer $C_1$-$C_6$-Alkyl-, $C_3$-$C_7$-Cycloalkyl-, $C_3$-$C_7$-Cycloalkyl-$C_1$-$C_3$-alkylen-, $C_1$-$C_6$-Fluoroalkyl-, $C_1$-$C_6$-Alkoxy-, $C_1$-$C_6$-Fluoralkoxy-, Nitro- oder Cyanogruppe ausgewählt sind;
$Z_1$, $Z_2$, $Z_3$, $Z_4$ und $Z_5$ unabhängig voneinander für ein Wasserstoff- oder Halogenatom oder eine $C_1$-$C_6$-Alkyl-, $C_3$-$C_7$-Cycloalkyl-, $C_3$-$C_7$-Cycloalkyl-$C_1$-$C_3$-alkylen-, $C_1$-$C_6$-Fluoralkyl-, $C_1$-$C_6$-Alkoxy, $C_1$-$C_6$-Fluoralkoxy-, Cyano-, $C(O)NR_1R_2$-, Nitro-, $NR_1R_2$-, $C_1$-$C_6$-Thioalkyl-, -S(O)-$C_1$-$C_6$-Alkyl-, -$S(O)_2$-$C_1$-$C_6$-Alkyl-, $SO_2NR_1R_2$-, $NR_3COR_4$-, $NR_3SO_2R_5$-, Aryl-$C_1$-$C_6$-alkylen- oder Arylgruppe stehen, wobei das Aryl und das Aryl-$C_1$-$C_6$-alkylen gegebenenfalls durch einen oder mehrere Substituenten substituiert sind, die unter einem Halogen oder einer $C_1$-$C_6$-Alkyl-, $C_3$-$C_7$-Cycloalkyl-, $C_3$-$C_7$-Cycloalkyl-$C_1$-$C_3$-alkylen-, $C_1$-$C_6$-Fluoralkyl-, $C_1$-$C_6$-Alkoxy-, $C_1$-$C_6$-Fluoralkoxy-, Nitro- oder Cyanogruppe ausgewählt sind;
$R_1$ und $R_2$ unabhängig voneinander für ein Wasserstoffatom oder eine $C_1$-$C_6$-Alkyl-, $C_3$-$C_7$-Cycloalkyl-, $C_3$-$C_7$-Cycloalkyl-$C_1$-$C_3$-alkylen-, Aryl-$C_1$-$C_6$-alkylen- oder Arylgruppe stehen oder $R_1$ und $R_2$ gemeinsam mit dem Stickstoffatom, an das sie gebunden sind, eine Azetidin-, Pyrrolidin-, Piperidin-, Azepin-, Morpholin-, Thiomorpholin-, Piperazin- oder Homopiperazingruppe bilden, wobei diese Gruppe gegebenenfalls durch eine $C_1$-$C_6$-Alkyl-, $C_3$-$C_7$-Cycloalkyl-, $C_3$-$C_7$-Cycloalkyl-$C_1$-$C_3$-alkylen-, Aryl-$C_1$-$C_6$-alkylen- oder Arylgruppe substituiert ist;
$R_3$ und $R_4$ unabhängig voneinander für ein Wasserstoffatom oder eine $C_1$-$C_6$-Alkyl-, Aryl-$C_1$-$C_6$-alkylen- oder Arylgruppe stehen;
$R_5$ für eine $C_1$-$C_6$-Alkyl- oder Arylgruppe steht;
W für eine anellierte bicyclische Gruppe der Formel:

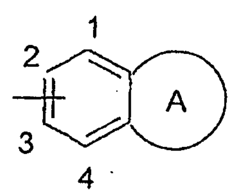

steht, die über die Positionen 1, 2, 3 oder 4 an das Stickstoffatom gebunden ist;
A für einen 5- bis 7-gliedrigen Heterocyclus mit einem bis drei unter O, S und N ausgewählten Heteroatomen steht;
wobei das Kohlenstoffatom bzw. die Kohlenstoffatome von A gegebenenfalls durch eine oder mehrere Gruppen substituiert ist bzw. sind, die unter einem Wasserstoffatom oder einer $C_1$-$C_6$-Alkyl-, $C_3$-$C_7$-Cycloalkyl-, $C_3$-$C_7$-Cycloalkyl-$C_1$-$C_3$-alkylen-, $C_1$-$C_6$-Fluoralkyl-, Aryl-, Aryl-$C_1$-$C_6$-alkylen-, Oxo- oder Thiogruppe ausgewählt sind;
wobei das Stickstoffatom bzw. die Stickstoffatome von A dann, wenn der Stickstoff neben einem durch eine Oxogruppe substituierten Kohlenstoffatom steht, gegebenenfalls durch $R_6$ oder in den anderen Fällen durch $R_7$ substituiert ist bzw. sind;

$R_6$ für ein Wasserstoffatom oder eine $C_1$-$C_6$-Alkyl-, $C_3$-$C_7$-Cycloalkyl-, $C_3$-$C_7$-Cycloalkyl-$C_1$-$C_3$-alkylen-, $C_1$-$C_6$-Fluoralkyl-, Aryl-$C_1$-$C_6$-alkylen- oder Arylgruppe steht;
$R_7$ für ein Wasserstoffatom oder eine $C_1$-$C_6$-Alkyl-, $C_3$-$C_7$-Cycloalkyl-, $C_3$-$C_7$-Cycloalkyl-$C_1$-$C_3$-alkylen-, $C_1$-$C_6$-Fluoralkyl-, Aryl-$C_1$-$C_6$-alkylen-, $C_1$-$C_6$-Alkyl-C(O)-, $C_3$-$C_7$-Cycloalkyl-$C_1$-$C_3$-alkylen-(CO)-, $C_1$-$C_6$-Fluoralkyl-C(O)-, $C_3$-$C_7$-Cycloalkyl-C(O)-, Aryl-C(O)-, Aryl-$C_1$-$C_6$-alkylen-C(O)-, $C_1$-$C_6$-Alkyl-S$(O)_2$-, $C_1$-$C_6$-Fluoralkyl-S$(O)_2$-, $C_3$-$C_7$-Cycloalkyl-S$(O)_2$-, $C_3$-$C_7$-Cycloalkyl-$C_1$-$C_3$-alkylen-S$(O)_2$-, Aryl-S$(O)_2$-oder Aryl-$C_1$-$C_6$-alkylen-S$(O)_2$-Gruppe steht;
wobei das Schwefelatom bzw. die Schwefelatome des Heterocyclus A in oxidierter Form vorliegen kann bzw. können;
wobei das Stickstoffatom bzw. die Stickstoffatome des Heterocyclus A in oxidierter Form vorliegen kann bzw. können;
das Stickstoffatom in Position 4, 5, 6 oder 7 des Pyrrolopyridins in oxidierter Form vorliegen kann;
in Basen- oder Säureadditionssalzform sowie in Hydrat- oder Solvatform.

2. Verbindung der Formel (I) nach Anspruch 1, **dadurch gekennzeichnet, daß** n gleich 1 oder 2 ist; in Basen- oder Säureadditionssalzform sowie in Hydrat- oder Solvatform.

3. Verbindung der Formel (I) nach Anspruch 1 oder 2, **dadurch gekennzeichnet, daß** der Pyrrolopyridinkern eine Pyrrolo[3,2-*b*]pyridingruppe, eine Pyrrolo-[3,2-*c*]pyridingruppe, eine Pyrrolo[2,3-*c*]pyridin-gruppe oder eine Pyrrolo [2,3-*b*]pyridingruppe ist;
wobei der Pyrrolopyridinkern gegebenenfalls in Kohlenstoffposition 4, 5, 6 und/oder 7 durch einen oder mehrere Substituenten X substituiert ist, die gleich oder voneinander verschieden sind und unter einem Wasserstoff- oder Halogenatom oder einer $C_1$-$C_6$-Alkyl-, $C_3$-$C_7$-Cycloalkyl-, $C_1$-$C_6$-Fluoralkyl-, $C_1$-$C_6$-Alkoxy-, $C_1$-$C_6$-Fluoralkoxy-, Nitro-, $NR_1R_2$-, $C_1$-$C_6$-Thioalkyl-, -S(O)-$C_1$-$C_6$-Alkyl-, -S$(O)_2$-$C_1$-$C_6$-Alkyl- oder Arylgruppe ausgewählt sind; und $R_1$ und $R_2$ unabhängig voneinander für ein Wasserstoffatom stehen; in Basen- oder Säureadditionssalzform sowie in Hydrat- oder Solvatform.

4. Verbindung der Formel (I) nach Anspruch 1 oder 2, **dadurch gekennzeichnet, daß** der Pyrrolopyridinkern eine Pyrrolo[3,2-*b*]pyridingruppe, eine Pyrrolo-[3,2-*c*]pyridingruppe, eine Pyrrolo[2,3-*c*]pyridin-gruppe oder eine Pyrrolo [2,3-*b*]pyridingruppe ist; wobei der Pyrrolopyridinkern gegebenenfalls in Kohlenstoffposition 4, 5, 6 und/oder 7 durch einen oder mehrere Substituenten X substituiert ist, die gleich oder voneinander verschieden sind und unter einem Halogenatom oder einer $C_1$-$C_6$-Fluoralkyl- oder Arylgruppe ausgewählt sind; in Basen- oder Säureadditionssalzform sowie in Hydrat- oder Solvatform.

5. Verbindung der Formel (I) nach einem der Ansprüche 1 bis 4, **dadurch gekennzeichnet, daß** $Z_1$, $Z_2$, $Z_3$, $Z_4$ und $Z_5$ unabhängig voneinander für ein Wasserstoff- oder Halogenatom stehen; in Basen- oder Säureadditionssalzform sowie in Hydrat- oder Solvatform.

6. Verbindung der Formel (I) nach einem der Ansprüche 1 bis 5, **dadurch gekennzeichnet, daß** W unter Indolinyl-, Indolyl-, Isoindolyl-, Isoindolinyl-, Benzofuranyl-, Dihydrobenzofuranyl-, Benzothiophenyl-, Dihydrobenzothiophenyl-, Benzoxazolyl-, Dihydrobenzoxazolinyl-, Isobenzofuranyl-, Dihydroisobenzofuranyl-, Benzimidazolyl-, Dihydrobenzimidazolyl-, Indazolyl-, Benzothiazolyl-, Isobenzothiazolyl-, Dihydroisobenzothiazolyl-, Benzotriazolyl-, Chinolinyl-, Dihydrochinolinyl-, Tetrahydrochinolinyl-, Isochinolinyl-, Dihydroisochinolinyl-, Tetrahydroisochinolinyl-, Benzoxazinyl-, Dihydrobenzoxazinyl-, Benzothiazinyl-, Dihydrobenzothiazinyl-, Cinnolinyl-, Chinazolinyl-, Dihydrochinazolinyl-, Tetrahydrochinazolinyl-, Chinoxalinyl-, Dihydrochinoxalinyl-, Tetrahydrochinoxalinyl-, Phthalazinyl-, Dihydrophthalazinyl-, Tetrahydrophthalazinyl-, Tetrahydrobenz[*b*]azepinyl-, Tetrahydrobenz[*c*]azepinyl-, Tetrahydrobenz [*d*]azepinyl-, Tetrahydrobenzo[*b*]-[1,4]diazepinyl-, Tetrahydrobenzo[*e*][1,4]-diazepinyl-, Tetrahydrobenzo[*b*][1,4] oxazepinyl- oder Tetrahydrobenzo[*b*][1,4]thiazepinylgruppen ausgewählt ist;
wobei das Kohlenstoff- und/oder Stickstoffatom bzw. die Kohlenstoff- und/oder Stickstoffatome der Gruppe W gegebenenfalls wie oben in der allgemeinen Formel (I) nach Anspruch 1 definiert ist bzw. sind; in Basen- oder Säureadditionssalzform sowie in Hydrat- oder Solvatform.

7. Verbindung der Formel (I) nach einem der Ansprüche 1 bis 5, **dadurch gekennzeichnet, daß** W für eine anellierte bicyclische Gruppe der Formel:

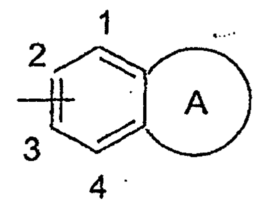

steht, die über die Positionen 1, 2, 3 oder 4 an das Stickstoffatom gebunden ist;
A für einen 5- bis 7-gliedrigen Heterocyclus mit einem bis drei unter O, S und N ausgewählten Heteroatomen steht und W unter Indolyl-, Benzimidazolyl-, Tetrahydrochinolinyl-, Chinolinyl- und Benzothiazolylgruppen ausgewählt ist; und/oder
wobei das Kohlenstoffatom bzw. die Kohlenstoffatome von A gegebenenfalls durch eine oder mehrere Gruppen substituiert ist bzw. sind, die unter einem Wasserstoffatom oder einer $C_1$-$C_6$-Alkyl- oder Oxogruppe ausgewählt sind; und/oder
wobei das Stickstoffatom bzw. die Stickstoffatome von A dann, wenn der Stickstoff neben einem durch eine Oxogruppe substituierten Kohlenstoffatom steht, gegebenenfalls durch $R_6$ oder in den anderen Fällen durch $R_7$ substituiert ist bzw. sind; $R_6$ für ein Wasserstoffatom steht;
$R_7$ für ein Wasserstoffatom oder eine $C_1$-$C_6$-Alkylgruppe steht; in Basen- oder Säureadditionssalzform sowie in Hydrat- oder Solvatform.

**8.** Verbindung der Formel (I) nach einem der Ansprüche 1 bis 7, ausgewählt unter:

- *N*-(1-Methyl-1*H*-indol-5-yl)-1-[(3-fluorphenyl)-methyl]-1*H*-pyrrolo[2,3-*c*]pyridin-2-carboxamid;
- *N*-(1-Methyl-1*H*-benzimidazol-5-yl)-5-fluor-1-[(3-fluorphenyl)methyl]-1*H*-pyrrolo[2,3-*c*]pyridin-2-carboxamid;
- *N*-(1,2-Dimethyl-1*H*-benzimidazol-5-yl)-5-chlor-1-[(3-fluorphenyl)methyl]-1*H*-pyrrolo[2,3-*c*]pyridin-2-carboxamid;
- *N*-(1,2-Dimethyl-1*H*-benzimidazol-5-yl)-1-[(3-fluorphenyl)methyl]-1*H*-pyrrolo[2,3-*c*]pyridin-2-carboxamid;
- *N*-(2-Methyl-1*H*-benzothiazol-5-yl)-5-fluor-1-[(3-fluorphenyl)methyl]-1*H*-pyrrolo[2,3-*c*]pyridin-2-carboxamid;
- *N*-(1,2-Dimethyl-1*H*-benzimidazol-5-yl)-5-fluor-1-[(3-fluorphenyl)methyl]-1*H*-pyrrolo[2,3-c]pyridin-2-carboxamid;
- *N*-(1,2-Dimethyl-1*H*-benzimidazol-5-yl)-5-phenyl-1-[(3-fluorphenyl)methyl]-1*H*-pyrrolo[2,3-*c*]-pyridin-2-carboxamid;
- *N*-(1-Methyl-1*H*-indol-5-yl)-1-[(3-fluorphenyl)-methyl]-1*H*-pyrrolo[2,3-*b*]pyridin-2-carboxamid;
- *N*-(1-Methyl-1*H*-benzimidazol-5-yl)-1-[(3-fluorphenyl)methyl]-1*H*-pyrrolo[2,3-*b*]pyridin-2-carboxamid;
- *N*-(2-Oxo-1,2,3,4-tetrahydrochinolin-7-yl)-1-[(3-fluorphenyl)methyl]-1*H*-pyrrolo[2,3-*b*]pyridin-2-carboxamid;
- *N*-(Chinolin-7-yl)-1-[(3-fluorphenyl)methyl]-1*H*-pyrrolo[2,3-*b*]pyridin-2-carboxamid;
- *N*-(1-Methyl-1*H*-indol-5-yl)-1-(phenylmethyl)-1*H*-pyrrolo[2,3-*b*]pyridin-2-carboxamid;
- *N*-(1-Methyl-1*H*-indol-5-yl)-1-(phenylethyl)-1*H*-pyrrolo[2,3-*b*]pyridin-2-carboxamid;
- *N*-(2-Methylbenzothiazol-5-yl)-1-[(3-fluorphenyl)methyl]-1*H*-pyrrolo[2,3-*b*]pyridin-2-carboxamid;
- *N*-(1-Methyl-1*H*-benzimidazol-5-yl)-5-fluor-1-[(3-fluorphenyl)methyl]-1*H*-pyrrolo[2,3-*b*]pyridin-2-carboxamid;
- *N*-(1,2-Dimethyl-1*H*-benzimidazol-5-yl)-5-trifluormethyl-1-[(3-fluorphenyl)methyl]-1*H*-pyrrolo[2,3-*b*]pyridin-2-carboxamid;
- *N*-(1-Methyl-1*H*-indol-5-yl)-5-trifluormethyl-1-[(3-fluorphenyl)methyl]-1*H*-pyrrolo[2,3-*b*]pyridin-2-carboxamid;
- *N*-(2-Methyl-benzothiazol-5-yl)-5-trifluormethyl-1-[(3-fluorphenyl)methyl]-1*H*-pyrrolo[2,3-*b*]-pyridin-2-carboxamid;
- *N*-(1,2-Dimethyl-1*H*-benzimidazol-5-yl)-1-[(3-fluorphenyl)methyl]-1*H*-pyrrolo[2,3-*b*]pyridin-2-carboxamid;
- *N*-(1,2-Dimethyl-1*H*-benzimidazol-5-yl)-5-fluor-1-[(3-fluorphenyl)methyl]-1*H*-pyrrolo[2,3-*b*]pyridin-2-carboxamid;
- *N*-(1,2-Dimethyl-1*H*-benzimidazol-5-yl)-1-[(3-fluorphenyl)methyl]-1*H*-pyrrolo[3,2-*c*]pyridin-2-carboxamid;
- *N*-(1-Methyl-1*H*-benzimidazol-5-yl)-1-[(3-fluorphenyl)methyl]-1*H*-pyrrolo[3,2-*b*]pyridin-2-carboxamid;
- *N*-(1,2-Dimethyl-1*H*-benzimidazol-5-yl)-5-trifluormethyl-1-[(3-fluorphenyl)methyl]-1*H*-pyrrolo[3,2-*b*]pyridin-2-carboxamid;
- *N*-(2-Methylbenzothiazol-5-yl)-1-[(3-fluorphenyl)methyl]-1*H*-pyrrolo[3,2-*b*]pyridin-2-carboxamid;
- *N*-(1,2-Dimethyl-1*H*-benzimidazol-5-yl)-1-[(3-fluorphenyl)methyl]-1*H*-pyrrolo[3,2-*b*]pyridin-2-carboxamid;
- *N*-(1,2-Dimethyl-1*H*-benzimidazol-5-yl)-1-(phenyl)-1*H*-pyrrolo[2,3-*b*]pyridin-2-carboxamid.

**9.** Verfahren zur Herstellung einer Verbindung der Formel (I) nach einem der Ansprüche 1 bis 8, **dadurch gekennzeichnet, daß** man eine Verbindung der allgemeinen Formel (IV)

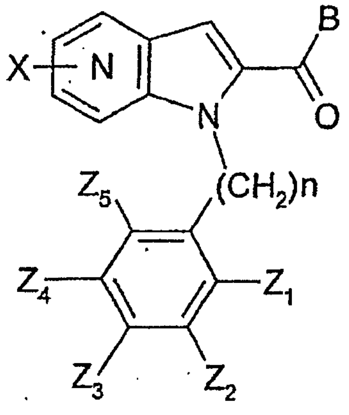

(IV).

worin $X_1$, $X_2$, $X_3$, $X_4$, $Z_1$, $Z_2$, $Z_3$, $Z_4$, $Z_5$ und n die in der allgemeinen Formel (I) nach Anspruch 1 angegebene Bedeutung besitzen und B für eine $C_1$-$C_4$-Alkoxygruppe steht,
am Rückflußpunkt eines Lösungsmittels mit einem Amid der allgemeinen Formel (V)

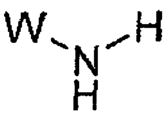

(V)

worin W die in der allgemeinen Formel (I) nach Anspruch 1 angegebene Bedeutung besitzt, umsetzt,
wobei das Amid der Verbindung der allgemeinen Formel (V) durch vorherige Einwirkung von Trimethylaluminium auf Verbindungen der allgemeinen Formel (V) hergestellt wird.

**10.** Verfahren zur Herstellung einer Verbindung der Formel (I) nach einem der Ansprüche 1 bis 8, **dadurch gekennzeichnet, daß** man eine Verbindung der allgemeinen Formel (IV)

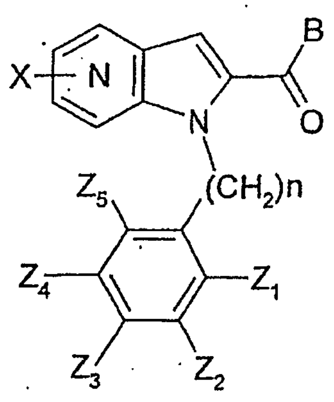

(IV).

worin $X_1$, $X_2$, $X_3$, $X_4$, $Z_1$, $Z_2$, $Z_3$, $Z_4$, $Z_5$ und n die in der allgemeinen Formel (I) nach Anspruch 1 angegebene Bedeutung besitzen und B für eine Hydroxylgruppe steht,
durch Einwirkung von Thionylchlorid am Rückflußpunkt eines Lösungsmittels in das Säurechlorid umwandelt und dann die erhaltene Verbindung der allgemeinen Formel (IV), worin $X_1$, $X_2$, $X_3$, $X_4$, $Z_1$, $Z_2$, $Z_3$, $Z_4$, $Z_5$ und n die in der allgemeinen Formel (I) nach Anspruch 1 angegebene Bedeutung besitzen und B für ein Chloratom steht, in

Gegenwart einer Base mit der Verbindung der allgemeinen Formel (V)

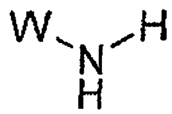 (V)

worin W die in der allgemeinen Formel (I) nach Anspruch 1 angegebene Bedeutung besitzt, umsetzt oder eine Kupplungsreaktion zwischen einer Verbindung der allgemeinen Formel (IV), worin $X_1$, $X_2$, $X_3$, $X_4$, $Z_1$, $Z_2$, $Z_3$, $Z_4$, $Z_5$ und n die in der allgemeinen Formel (I) nach Anspruch 1 angegebene Bedeutung besitzen und B für eine Hydroxylgruppe steht,
und der Verbindung der allgemeinen Formel (V), worin W die in der allgemeinen Formel (I) nach Anspruch 1 angegebene Bedeutung besitzt, in Gegenwart eines Kupplungsmittels und einer Base in einem Lösungsmittel durchführt.

**11.** Arzneimittel, **dadurch gekennzeichnet, daß** es eine Verbindung der Formel (I) nach einem der Ansprüche 1 bis 8 oder ein pharmazeutisch annehmbares Salz oder auch ein Hydrat oder ein Solvat der Verbindung der Formel (I) enthält.

**12.** Pharmazeutische Zusammensetzung, **dadurch gekennzeichnet, daß** sie eine Verbindung der Formel (I) nach einem der Ansprüche 1 bis 8 oder ein pharmazeutisch annehmbares Salz oder auch ein Hydrat oder ein Solvat dieser Verbindung sowie mindestens einen pharmazeutisch annehmbaren Hilfsstoff enthält.

**13.** Verwendung einer Verbindung der Formel (I) nach einem der Ansprüche 1 bis 8 zur Herstellung eines Arzneimittels zur Prävention oder Behandlung von Pathologien, an denen die Rezeptoren vom TRPV1-Typ beteiligt sind.

**14.** Verwendung einer Zusammensetzung der Formel (I) nach einem der Ansprüche 1 bis 8 zur Herstellung eines Arzneimittels zur Prävention oder Behandlung von Schmerzen, Entzündungen, urologischen Störungen, gynäkologischen Störungen, gastrointestinalen Störungen, respiratorischen Störungen, Psoriasis, Pruritis, Haut-, Augen- oder Schleimhautreizungen, Herpes oder Zona oder zur Behandlung von Depression oder Diabetes.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description


- WO 2004104001 A
- WO 03049702 A
- US 0149367 A
- WO 03068749 A
- US 20050131012 A


### Littérature non-brevet citée dans la description


- **J. March.** Advances in Organic Chemistry. Wiley Interscience, 2001
- **Kolasa T.** *Bioorg.Med.Chem.,* 1997, vol. 5 (3), 507
- **Abramovitch R.** *Synth. Commun.,* 1995, vol. 25 (1), 1
- **O. Mitsonobu.** *Synthesis,* 1981, 1-28
- **Murakami Y.** *Chem.Pharm.Bull.,* 1995, vol. 43 (8), 1281
- **S.L. Buchwald.** *J.Am.Chem.Soc,* 2002, vol. 124, 11684
- **M. Nazare et al.** *Angew Chem Int Ed,* 2004, vol. 43 (34), 4526-4528
- **P.M. Fresneda et al.** *Tetrahedron Lett,* 2000, vol. 41 (24), 4777-4780
- **M.H. Fisher et al.** *J Heterocyclic Chem,* 1969, vol. 6, 775
- **B. Frydman et al.** *J Am Chem Soc,* 1965, vol. 87, 3530
- **L.N. Yakhontov.** *Tetrahedron Lett,* 1969, 1909
- **G.P. Fagan et al.** *J Med Chem,* 1988, vol. 31 (5), 944
- **A. Furstner et al.** *J Am Chem Soc,* 2002, vol. 124 (46), 13856
- **G. Quéguiner et al.** *J Org Chem,* 1998, vol. 63 (9), 2892
- *Pharmazie,* 1990, vol. 45, 346